# EUROPEAN PATENT SPECIFICATION

(11) **EP 1 521 594 B1**
(45) Date of publication and mention of the grant of the patent: **02.10.2013**
(21) Application number: 02786981.7
(22) Date of filing: 09.12.2002
(51) Int. Cl.: C07K 14/005, C12Q 1/68, C12N 7/00, G01N 33/574

(54) **ENDOGENOUS RETROVIRUS POLYPEPTIDES LINKED TO ONCOGENIC TRANSFORMATION**
MIT ONKOGENER TRANSFORMATION IN VERBINDUNG GEBRACHTE ENDOGENE RETROVIRUSPOLYPEPTIDE
POLYPEPTIDES DE RETROVIRUS ENDOGENES LIES A LA TRANSFORMATION ONCOGENIQUE

(30) Priority: 07.12.2001 WO PCT/US01/47824; 07.12.2001 US 16604; 07.12.2001 US 340064 P; 12.06.2002 US 388046 P
(43) Date of publication of application: 13.04.2005
(73) Proprietor: Novartis Vaccines and Diagnostics, Inc., Emeryville, CA 94608 (US)
(72) Inventor: GARCIA, Pablo, San Francisco, CA 94131 (US); HARDY, Stephen F., San Francisco, CA 94121 (US); WILLIAMS, Lewis T., Mill Valley, Ca 94902 (US); ESCOBEDO, Jaime, Alamo, CA 94507 (US)
(74) Representative: Marshall, Cameron John
(86) International application number: PCT/US2002/039344
(87) International publication number: WO 2003/050258

(56) References cited:
- SMITH R D ET AL: "HUMAN ENDOGENOUS RETROVIRUS HERV-K EXPRESSION IN PROSTATE CANCER" JOURNAL OF UROLOGY, BALTIMORE, MD, US, vol. 165, no. 5, SUPPL, 2 June 2001 (2001-06-02), pages 136-137, XP009009589 ISSN: 0022-5347
- DATABASE EMBL 6 November 2001 (2001-11-06), XP002418255 Database accession no. AAM75812 & WO 01/57276 A (MOLECULAR DYNAMICS INC) 9 August 2001 (2001-08-09)
- DATABASE EMBL 12 October 2001 (2001-10-12), XP002418256 Database accession no. BI858348
- DATABASE EMBL 30 March 1995 (1995-03-30), XP002418257 retrieved from EMBL Database accession no. x82271 & LOEWER R ET AL: "IDENFIFICATION OF A REV-RELATED PROTEIN BY ANALYSIS OF SPLICED TRANSCRIPTS OF THE HUMAN ENDOGENOUS RETROVIRUSES HTDV/HERV-K" JOURNAL OF VIROLOGY, THE AMERICAN SOCIETY FOR MICROBIOLOGY, US, vol. 69, no. 1, January 1995 (1995-01), pages 141-149, XP002058199 ISSN: 0022-538X
- MAYER J ET AL: "Chromosomal assignment of human endogenous retrovirus K (HERV-K) env open reading frames." CYTOGENETICS AND CELL GENETICS, vol. 79, no. 1-2, 1997, pages 157-161, XP009009741 ISSN: 0301-0171
- WANG-JOHANNING F ET AL: "Expression of human endogenous retrovirus k envelope transcipts in human breast cancer" CLINICAL CANCER RESEARCH, THE AMERICAN ASSOCIATION FOR CANCER RESEARCH, US, vol. 7, no. 6, 2001, pages 1553-1560, XP002983118 ISSN: 1078-0432
- ARMBRUESTER VIVIENNE ET AL: "A novel gene from the human endogenous retrovirus K expressed in transformed cells." CLINICAL CANCER RESEARCH : AN OFFICIAL JOURNAL OF THE AMERICAN ASSOCIATION FOR CANCER RESEARCH JUN 2002, vol. 8, no. 6, June 2002 (2002-06), pages 1800-1807, XP002418243 ISSN: 1078-0432
- WANG-JOHANNING ET AL.: 'Detection of human endogenous retrovirus envelope, HERV-E4-1, mRNA transcriptional activity in prostrate adenocarcimoma by RT-PCR and in situ hybridization' PROCEEDINGS OF THE AMERICAN ASSOCIATION FOR CANCER RESEARCH vol. 40, 1999, page 424, XP008041801
- WANG-JOHANNING ET AL.: 'Expression of human endogenous retrovirus k envelope transcipts in human breast cancer' CLINICAL CANCER RESEARCH vol. 7, no. 6, 2001, pages 1553 - 1560, XP002983118
- BARBULESCU ET AL.: 'Many human endogenous retrovirus K (HERV-K) proviruses are unique to humans' CURRENT BIOLOGY vol. 9, no. 26, August 1999, pages 861 - 868, XP000953273
- TONJES ET AL.: 'Genome-wide screening, cloning, chromosomal assignment, and expression of full-length human endogenous retrovirus type K.' JOURNAL OF VIROLOGY vol. 73, no. 11, November 1999, pages 9187 - 9195, XP002238923
- SAUTER ET AL.: 'Human endogenous retrovirus K10: expression of Gag protein and detection of antibodies in patients with seminomas' JOURNAL OF VIROLOGY vol. 69, no. 1, January 1995, pages 414 - 421, XP002031129
- GOEDERT ET AL.: 'High prevalence of antibodies against HERV-K10 in patients with testicular cancer but not with AIDS' CANCER EPIDEMIOLOGY BIOMARKERS AND PREVENTION vol. 8, no. 4, April 1999, pages 293 - 296, XP002983119

## Description

### TECHNICAL FIELD

The present invention relates to the diagnosis of cancer e.g. prostate cancer. In particular, it relates to a subgroup of human endogenous retroviruses (HERVs) which show up-regulated expression in prostate tumors, and to the polypeptides encoded by spliced mRNAs expressed by these viruses.

### BACKGROUND ART

References 1 and 2 disclose that human endogenous retroviruses (HERVs) of the HML-2 subgroup of the HERV-K family show up-regulated expression in prostate tumors.

It is an object of the invention to provide further materials that can be used in the prevention, treatment and diagnosis of cancer, *e.g.*, prostate cancer. It is a further object to provide improvements in the prevention, treatment and diagnosis of cancer e.g. prostate cancer and breast cancer.

### DISCLOSURE OF THE INVENTION

HERVs have been known for many years, and genomic sequence for the HERV-K family has been known since 1986 {ref. 187}. The usual gag, prt, pol and env retroviral proteins have been identified for HERV-K, as has an analogue of HIV Rev or HTLV Rex, known as cORF or Rec {3}, but analogues of other regulatory proteins (*e.g.* HIV Tat or HTLV Tax proteins) have not been identified.

The Rev/Rex analog 'cORF' is encoded by an ORF which shares the same 5' region and start codon as env, but in which a splicing event removes env-coding sequences and shifts to a reading frame +1 relative to that of env {4, 5}. Within the final exon in the env region of PCAV, therefore, reading frames 1 and 2 encode env and cORF, respectively, but no protein encoded by the third reading frame has previously been reported, and this +2 reading frame has no known function in HERV-K.

The inventors have now found a series of proteins generated by splicing in the env region of HERV-K genomes, including several which utilize the +2 reading frame. The proteins show activity typical of transcriptional regulators, and they also have oncogenic potential. These proteins can be used in cancer diagnosis and therapy, and are also drug targets *e.g.* for adjuvant therapy.

The identification of these new polypeptide products is remarkable because full sequence information has been available for HERV-K viruses for over 15 years.

The invention provides a method for diagnosing prostate cancer, the method comprising the step of detecting the presence or absence in a patient sample of a HML-2 expression product produced by a splicing event in which the 5' region of a HML-2 env coding region and the start codon of the env coding region are joined to a downstream coding region in the reading frame +2 relative to that of env in the genome. Higher levels of expression product relative to normal tissue indicate that the patient from whom the sample was taken has prostate cancer. The expression product may or may not be functional in a viral life cycle.

The expression product which is detected is either a mRNA transcript or a polypeptide translated from such a transcript. These expression products may be detected directly or indirectly. A direct test uses an assay which detects HML-2 RNA or polypeptide in a patient sample. An indirect test uses an assay which detects biomolecules which are not directly expressed *in vivo* from HML-2 *e.g.* an assay to detect cDNA which has been reverse-transcribed from a HML-2 mRNA, or an assay to detect an antibody which has been raised in response to a HML-2 polypeptide.

### A - THE PATIENT SAMPLE

Where the diagnostic method of the invention is based on mRNA for diagnosis of prostate cancer, the patient sample will generally comprise cells from the tissue of interest *e.g.* prostate cells for prostate cancer. These cells may be present in a sample of tissue taken from the relevant organ, or may be cells which have escaped into circulation (*e.g.* during metastasis). Instead of or as well as comprising cells, the sample may comprise virions which contain mRNA from HML-2, or bodily fluids.

Where the diagnostic method of the invention is based on polypeptide, the patient sample may comprise cells and/or virions (as described above for mRNA), or may comprise antibodies which recognize the polypeptide. Such antibodies will typically be present in circulation.

In general, therefore, the patient sample for males is a prostate sample (*e.g.* a biopsy) or a blood sample.

The patient is generally a human, and preferably an adult human.

Expression products may be detected in the patient sample itself, or may be detected in material derived from the sample (*e.g.* the supernatant of a cell lysate, or a RNA extract, or cDNA generated from a RNA extract, or polypeptides translated from a RNA extract, or cells derived from culture of cells extracted from a patient, *etc.*). These are still considered to be "patient samples" within the meaning of the invention.

Methods of the invention can be conducted *in vitro* or *in vivo.*

Other possible sources of patient samples include isolated cells, whole tissues, or bodily fluids (*e.g.* blood, plasma, serum, urine, pleural effusions, cerebro-spinal fluid, breast milk, colostrum, other fluids secreted by the breast, semen, seminal fluid, *etc.)*

### B - THE mRNA EXPRESSION PRODUCT

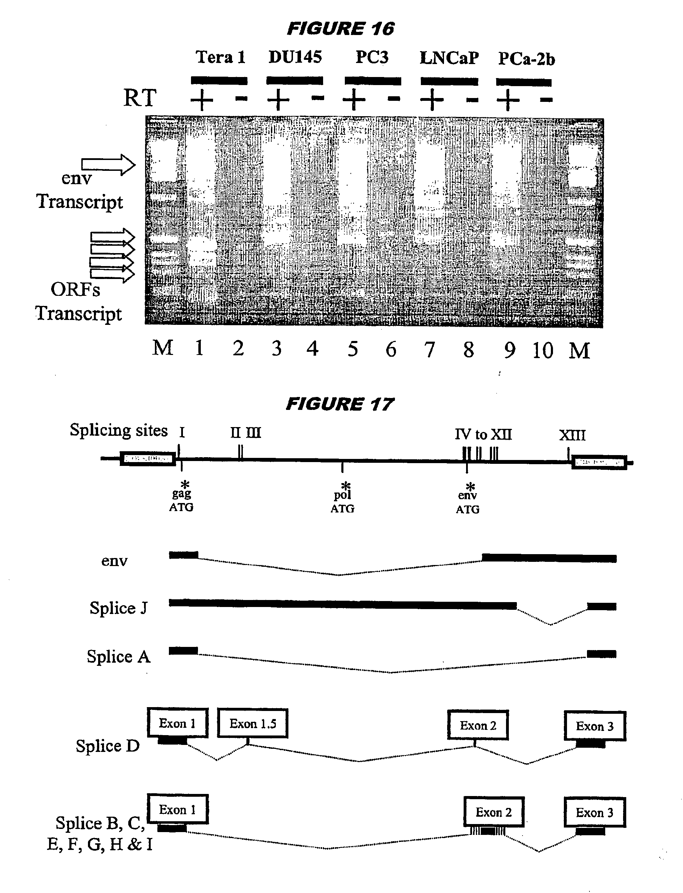

Where the diagnostic method of the invention is based on mRNA detection, it typically involves detecting a RNA which encodes a polypeptide of the invention. The RNA will comprise the ATG codon of the Env ORF which, through splicing as shown in Figure 17, is in the same reading frame as sequences from the 3' end of the Env ORF, but which are (relative to the ATG in the genomic DNA copy of HML-2) in the +2 reading frame (*i.e.* the third reading frame). The invention may thus involve a step of detecting a RNA produced by a splicing event in which the 5' region and start codon of the env coding region are joined to a downstream coding region in the reading frame +2 relative to that of env.

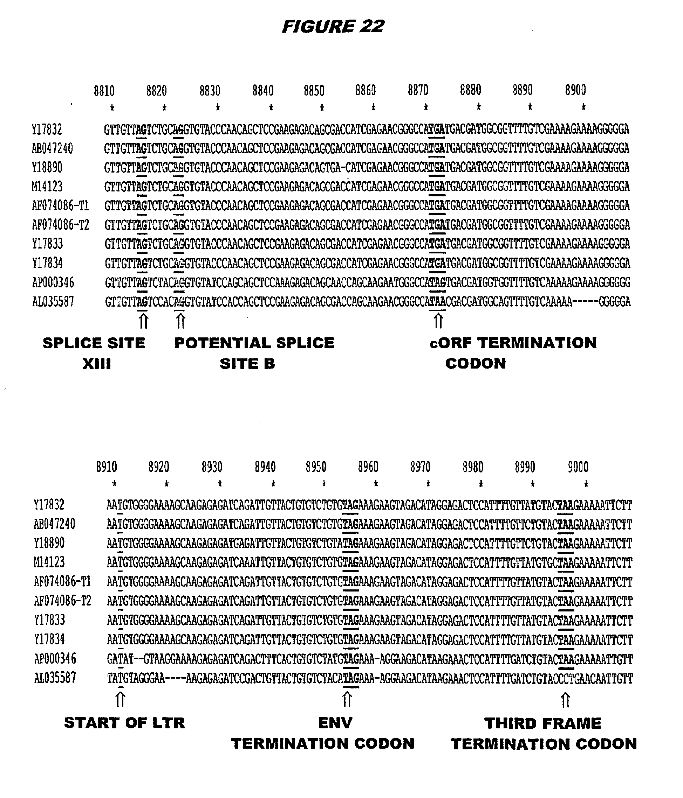

Preferred RNAs comprise a sequence which has at least *s%* sequence identity to SEQ ID 52. SEQ ID 52 is the 50 nucleotides of the HERV-K(C7) virus {ref. 6} immediately downstream of 'Potential splice site B' in Figure 22.

Other preferred RNAs comprise a sequence which has at least *s%* sequence identity to one or more of SEQ IDs 19, 20, 21, 24, 25, 26, 38, 40 and/or 42. Particularly preferred RNAs comprise a sequence which has at least *s%* sequence identity to one or more of SEQ IDs 38, 40 and/or 42.

Preferred RNAs comprise a sequence which encodes a polypeptide having at least *s%* sequence identity to one or more of SEQ IDs 7, 8, 9, 10, 11, 21, 28, 29, 30, 31, 34, 35, 36, 39, 41, 43, 67, 68 and 69. Particularly preferred RNAs comprise a sequence which encodes a polypeptide having at least *s%* sequence identity to one or more of SEQ IDs 7, 8 and/or 9.

The value of *s* is preferably at least 50 *(e.g.* at least 55, 60, 65, 70, 75, 80, 85, 90, 91, 92, 93, 94, 95, 96, 97, 98, 99, 99.5, 99.9, *etc.).*

Preferred RNAs encode a polypeptide which may bind to RNA comprising SEQ ID 49.

The RNA will usually also comprise one, two, three, four or five of the following:
1. An upstream sequence which has at least 75% identity to SEQ ID 49 (*e.g*. 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.5%, 99.9%, 100% identity); or a sequence which has at least 50% identity to SEQ ID 49 (*e.g*. 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.5%, 99.9%, 100% identity) and is expressed at least 1.5 fold (e.g. 2, 2.5, 5, 10, 20, 50, *etc.,* fold) higher level relative to expression in a normal (*i.e.,* non cancerous) cell with at least a 95% confidence level; or a sequence which has at least 80% identity (*e.g.* 81%, 82%, 83%, 84%, 85%, 86%, 87%, 88%, 89%, 90%, 91%, 92%, 93%, 94%, 95%, 96%, 97%, 98%, 99%, 99.5%, 99.9%, 100% identity) to at least a 20 contiguous nucleotide fragment (*e.g.* 25, 30, 35, 40, 45, 50, 55, 60, 65, 70, 75, 80, 85, 90, 95, 100, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, *etc.*, contiguous nucleotides) of SEQ ID 49; or a sequence which has at least 80% identity (*e.g*. 81%, 82%, 83%, 84%, 85%, 86%, 87%, 88%, 89%, 90%, 91%, 92%, 93%, 94%, 95%, 96%, 97%, 98%, 99%, 99.5%, 99.9%, 100% identity) to at least a 20 contiguous nucleotide fragment (*e.g*. 25, 30, 35, 40, 45, 50, 55, 60, 65, 70, 75, 80, 85, 90, 95, 100, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, *etc.*, contiguous nucleotides) of SEQ ID 49 and is expressed at least 1.5 fold (*e.g.* 2, 2.5, 5, 10, 20, 50, *etc*., fold) higher level relative to expression in a normal (*i.e.*, non cancerous) cell with at least a 95% confidence level. This sequence will typically be at the 5' end of the RNA. SEQ ID 49 is the nucleotide sequence of the start of R region in the LTR of the 'ERVK6' HML-2 virus {ref. 7}. This portion of the R region may be found in HML-2 transcripts, and transcription of mRNA molecules including this portion of the R region is up-regulated in prostate cancer.
2. An upstream region comprising a sequence which has at least 75% sequence identity to SEQ ID 50 (*e.g*. 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.5%, 99.9%, 100% identity); or a sequence which has at least 50% identity to SEQ ID 50 (*e.g*. 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.5%, 99.9%, 100% identity) and is expressed at least 1.5 fold (*e.g.* 2, 2.5, 5, 10, 20, 50, *etc.,* fold) higher level relative to expression in a normal (*i.e*., non cancerous) cell with at least a 95% confidence level; or a sequence which has at least 80% identity (*e.g.* 81%, 82%, 83%, 84%, 85%, 86%, 87%, 88%, 89%, 90%, 91%, 92%, 93%, 94%, 95%, 96%, 97%, 98%, 99%, 99.5%, 99.9%, 100% identity) to at least a 20 contiguous nucleotide fragment (*e.g.* 25, 30, 35, 40, 45, 50, 55, 60, 65, 70, 75, 80, 85, 90, 95, 100, 110, 115, 120, 125, 130, 135, 140, 145, *etc.*, contiguous nucleotides) of SEQ ID 50; or a sequence which has at least 80% identity (*e.g.* 81%, 82%, 83%, 84%, 85%, 86%, 87%, 88%, 89%, 90%, 91%, 92%, 93%, 94%, 95%, 96%, 97%, 98%, 99%, 99.5%, 99.9%, 100% identity) to at least a 20 contiguous nucleotide fragment (*e.g.* 25, 30, 35, 40, 45, 50, 55, 60, 65, 70, 75, 80, 85, 90, 95, 100, 110, 115, 120, 125, 130, 135, 140, 145, *etc.,* contiguous nucleotides) of SEQ ID 50 and is expressed at least 1.5 fold (*e.g.* 2, 2.5, 5, 10, 20, 50, *etc.,* fold) higher level relative to expression in a normal (*i.e.*, non cancerous) cell with at least a 95% confidence level. SEQ ID 50 is the nucleotide sequence of the RU₅ region downstream of SEQ ID 49 in the ERVK6 LTR This region is found in full-length HML-2 transcripts, but may not be present in all mRNAs transcribed from a HML-2 LTR promoter (*e.g.* if transcription is attenuated).
3. An upstream region comprising a sequence which has at least 75% sequence identity to SEQ ID 6 (*e.g.* 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.5%, 99.9%, 100% identity); or a sequence which has at least 50% identity to SEQ ID 6 (*e.g.* 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.5%, 99.9%, 100% identity) and is expressed at least 1.5 fold (*e.g.* 2, 2.5, 5, 10, 20, 50, *etc.,* fold) higher level relative to expression in a normal (*i.e.*, non cancerous) cell with at least a 95% confidence level; or a sequence which has at least 80% identity (*e.g.* 81%, 82%, 83%, 84%, 85%, 86%, 87%, 88%, 89%, 90%, 91%, 92%, 93%, 94%, 95%, 96%, 97%, 98%, 99%, 99.5%, 99.9%, 100% identity) to at least a 20 contiguous nucleotide fragment (*e.g.* 25, 30, 35, 40, 45, 50, 55, 60, 65, 70, 75, 80, 85, 90, 95, 100, *etc.*, contiguous nucleotides) of SEQ ID 6; or a sequence which has at least 80% identity (*e.g.* 81%, 82%, 83%, 84%, 85%, 86%, 87%, 88%, 89%, 90%, 91%, 92%, 93%, 94%, 95%, 96%, 97%, 98%, 99%, 99.5%, 99.9%, 100% identity) to at least a 20 contiguous nucleotide fragment (*e.g.* 25, 30, 35, 40, 45, 50, 55, 60, 65, 70, 75, 80, 85, 90, 95, 100, *etc.*, contiguous nucleotides) of SEQ ID 6 and is expressed at least 1.5 fold (*e.g.* 2, 2.5, 5, 10, 20, 50, *etc.,* fold) higher level relative to expression in a normal (*i.e.*, non cancerous) cell with at least a 95% confidence level. SEQ ID 6 is the nucleotide sequence of the region of the ERVK6 virus between the U₅ region and the first 5' splice site. This region is found in full-length HML-2 transcripts, but has been lost by some variants and, like region 2 above, may not be present in all mRNAs transcribed from a HML-2 LTR promoter.
4. A downstream region comprising a sequence which has at least 75% sequence identity to SEQ ID 5 (*e.g.* 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.5%, 99.9%, 100% identity); or a sequence which has at least 50% identity to SEQ ID 5 (*e.g.* 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.5%, 99.9%, 100% identity) and is expressed at least 1.5 fold (*e.g.* 2, 2.5, 5, 10, 20, 50, *etc.,* fold) higher level relative to expression in a normal (*i.e.*, non cancerous) cell with at least a 95% confidence level; or a sequence which has at least 80% identity (*e.g.* 81%, 82%, 83%, 84%, 85%, 86%, 87%, 88%, 89%, 90%, 91%, 92%, 93%, 94%, 95%, 96%, 97%, 98%, 99%, 99.5%, 99.9%, 100% identity) to at least a 20 contiguous nucleotide fragment (*e.g.* 25, 30, 35, 40, 45, 50, 55, 60, 65, 70, 75, 80, 85, 90, 95, 100, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 350, 400, 450, 500, 550, 600, 650, 700, 750, 800, *etc.*, contiguous nucleotides) of SEQ ID 5; or a sequence which has at least 80% identity (*e.g.* 81%, 82%, 83%, 84%, 85%, 86%, 87%, 88%, 89%, 90%, 91%, 92%, 93%, 94%, 95%, 96%, 97%, 98%, 99%, 99.5%, 99.9%, 100% identity) to at least a 20 contiguous nucleotide fragment (*e.g.*. 25, 30, 35, 40, 45, 50, 55, 60, 65, 70, 75, 80, 85, 90, 95, 100, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 295, 300, 350, 400, 450, 500, 550, 600, 650, 700, 750, 800, *etc.*, contiguous nucleotides) of SEQ ID 5 and is expressed at least 1.5 fold (*e.g.* 2, 2.5, 5, 10, 20, 50, *etc*., fold) higher level relative to expression in a normal (*i.e.*, non cancerous) cell with at least a 95% confidence level. SEQ ID 5 is the nucleotide sequence of the U₃R region in the 3' end of ERVK6. This sequence will typically be between the stop codon of the Tat-coding region and immediately precedes any polyA tail.
5. A downstream 3' polyA tail.

The percent identity of the sequences described above are determined by the Smith-Waterman algorithm using the default parameters: open gap penalty = -20 and extension penalty = -5.

These mRNA molecules are referred to below as "PCA-mRNA" molecules ("prostate cancer associated mRNA"), and endogenous viruses which express these PCA-mRNAs are referred to as PCAVs ("prostate cancer associated viruses"). Nevertheless, said PCAVs may also be associated with other types of cancer and, in particular, breast cancer.

In general, therefore, the mRNA to be detected has formula N₁-N₂-N₃-N₄-N₅-polyA, wherein:
- N₁ has at least 75% sequence identity to SEQ ID 49; or has at least 50% identity to SEQ ID 49 and is expressed at least 1.5 fold higher relative to expression in a normal (*i.e.*, non cancerous) cell with at least a 95% confidence level; or has at least 80% identity to at least a 20 contiguous nucleotide fragment of SEQ ID 49; or has at least 80% identity to at least a 20 contiguous nucleotide fragment of SEQ ID 49 and is expressed at least 1.5 fold higher relative to expression in a normal (*i.e.*, non cancerous) cell with at least a 95% confidence level;
- N₂ has at least 75% sequence identity to SEQ ID 50; or has at least 50% identity to SEQ ID 50 and is expressed at least 1.5 fold higher relative to expression in a normal (*i.e.*, non cancerous) cell with at least a 95% confidence level; or has at least 80% identity to at least a 20 contiguous nucleotide fragment of SEQ ID 50; or has at least 80% identity to at least a 20 contiguous nucleotide fragment of SEQ ID 50 and is expressed at least 1.5 fold higher relative to expression in a normal (*i.e.*, non cancerous) cell with at least a 95% confidence level;
- N₃ has at least 75% sequence identity to SEQ ID 6; or has at least 50% identity to SEQ ID 6 and is expressed at least 1.5 fold higher relative to expression in a normal (*i.e.*, non cancerous) cell with at least a 95% confidence level; or has at least 80% identity to at least a 20 contiguous nucleotide fragment of SEQ ID 6; or has at least 80% identity to at least a 20 contiguous nucleotide fragment of SEQ ID 6 and is expressed at least 1.5 fold higher relative to expression in a normal (*i.e.*, non cancerous) cell with at least a 95% confidence level
- N₄ comprises a RNA sequence which includes the start codon of the env coding region spliced to a downstream coding region in the reading frame +2 relative to that of env;
- N₅ comprises a sequence which has at least 75% sequence identity to SEQ ID 5; or has at least 50% identity to SEQ ID 5 and is expressed at least 1.5 fold higher relative to expression in a normal (*i.e.*, non cancerous) cell with at least a 95% confidence level; or has at least 80% identity to at least a 20 contiguous nucleotide fragment of SEQ ID 5; or has at least 80% identity to at least a 20 contiguous nucleotide fragment of SEQ ID 5 and is expressed at least 1.5 fold higher relative to expression in a normal (*i.e.*, non cancerous) cell with at least a 95% confidence level; and
- N₁ and N₄ are present, but N₂, N₃, N₅ and polyA are optional.

N₁ is present in the mRNA to be detected and, more preferably, N₁-N₂ is present.

N₁ is preferably at the 5' end of the mRNA (*i.e.* 5'-N₁-...). Although N₁ is defined above by reference to SEQ ID 49, up to 100 nucleotides (*e.g.* 10, 20, 30, 40, 50, 60, 70, 71, 72, 73, 74, 75, 76, 77, 78, 79, 80, 81, 82, 83, 84, 85, 86, 87, 88, 89, 90 or 100) from the 5' end of SEQ ID 49 may be omitted, depending on the start site of transcription *e.g.* N₁ may at least 75% sequence identity to SEQ ID 478.

Where N₅ is present, it is preferably immediately before a 3' polyA tail (*i.e*. ... -N₅-polyA-3').

The RNA will generally have a 5' cap.

### B.1 - Enriching RNA in a sample

Where diagnosis is based on mRNA detection, the method of the invention preferably comprises an initial step of: (a) extracting RNA (*e.g.* mRNA) from a patient sample; (b) removing DNA from a patient sample without removing mRNA; and/or (c) removing or disrupting DNA comprising SEQ ID 4, but not RNA comprising SEQ ID 4, from a patient sample. This is necessary because the genomes of both normal and cancerous cells contain multiple PCAV DNA templates, whereas increased PCA-mRNA levels are only found in cancerous cells. As an alternative, a RNA-specific assay can be used which is not affected by the presence of homologous DNA.

Methods for extracting RNA from biological samples are well known {*e.g.* refs. 8 & 17} and include methods based on guanidinium buffers, lithium chloride, SDS/potassium acetate *etc.* After total cellular RNA has been extracted, mRNA may be enriched *e.g.* using oligo-dT techniques.

Methods for removing DNA from biological samples without removing mRNA are well known {*e.g.* appendix C of ref. 8} and include DNase digestion.

Methods for removing DNA, but not RNA, comprising PCA-mRNA sequences will use a reagent which is specific to a sequence within a PCA-mRNA *e.g.* a restriction enzyme which recognizes a DNA sequence within SEQ ID 4, but which does not cleave the corresponding RNA sequence.

Methods for specifically purifying PCA-mRNAs from a sample may also be used. One such method uses an affinity support which binds to PCA-mRNAs. The affinity support may include a polypeptide sequence which binds to the LTR of PCAV *e.g.* the tat polypeptide described below.

### B.2 - Direct detection of RNA

Various techniques are available for detecting the presence or absence of a particular RNA sequence in a sample {*e.g.* refs. 8 & 17}. If a sample contains genomic PCAV DNA, the detection technique will generally be RNA-specific; if the sample contains no PCAV DNA, the detection technique may or may not be RNA-specific.

Hybridization-based detection techniques may be used, in which a polynucleotide probe complementary to a region of PCA-mRNA is contacted with a RNA-containing sample under hybridizing conditions. Detection of hybridization indicates that nucleic acid complementary to the probe is present. Hybridization techniques for use with RNA include Northern blots, *in situ* hybridization and arrays.

Sequencing may also be used, in which the sequence(s) of RNA molecules in a sample are obtained. These techniques reveal directly whether a sequence of interest is present in a sample. Sequence determination of the 5' end of a RNA corresponding to N₁ will generally be adequate.

Amplification based techniques may also be used. These include PCR, SDA, SSSR, LCR, TMA, NASBA, T7 amplification *etc.* The technique preferably gives exponential amplification. A preferred technique for use with RNA is RT-PCR {*e.g.* see chapter 15 of ref. 8}. RT-PCR of mRNA from prostate cells is reported in references 9, 10, 11, 12, *etc.,* and RT-PCT of mRNA from breast cells is reported in references 13, 14, 15, 16, *etc..*

### B.3 - Indirect detection of RNA

Rather than detect RNA directly, it may be preferred to detect molecules which are derived from RNA (*i.e.* indirect detection of RNA). A typical indirect method of detecting mRNA is to prepare cDNA by reverse transcription and then to directly detect the cDNA. Direct detection of cDNA will generally use the same techniques as described above for direct detection of RNA (but it will be appreciated that methods such as RT-PCR are not suitable for DNA detection and that cDNA is double-stranded, so detection techniques can be based on a sequence, on its complement, or on the double-stranded molecule).

### B.4 - Polynucleotide materials

The invention provides polynucleotide materials *e.g.* for use in the detection of PCAV nucleic acids.

Hybridization reactions can be performed under conditions of different "stringency". Conditions that increase stringency of a hybridization reaction of widely known and published in the art {*e.g.* page 7.52 of reference 17}. Examples of relevant conditions include (in order of increasing stringency): incubation temperatures of 25°C, 37°C, 50°C, 55°C and 68°C; buffer concentrations of 10 X SSC, 6 X SSC, 1 X SSC, 0.1 X SSC (where SSC is 0.15 M NaCl and 15 mM citrate buffer) and their equivalents using other buffer systems; formamide concentrations of 0%, 25%, 50%, and 75%; incubation times from 5 minutes to 24 hours; 1, 2, or more washing steps; wash incubation times of 1, 2, or 15 minutes; and wash solutions of 6 x SSC, 1 x SSC, 0.1 x SSC, or de-ionized water. Hybridization techniques are well known in the art {*e.g.* see references 8, 17, 18, 19, 20 *etc.*}. Depending upon the particular polynucleotide sequence and the particular domain encoded by that polynucleotide sequence, hybridization conditions upon which to compare a polynucleotide of the invention to a known polynucleotide may differ, as will be understood by the skilled artisan.

In some embodiments, the isolated polynucleotide of the invention selectively hybridizes under low stringency conditions; in other embodiments it selectively hybridizes under intermediate stringency conditions; in other embodiments, it selectively hybridizes under high stringency conditions. An exemplary set of low stringency hybridization conditions is 50°C and 10xSSC. An exemplary set of intermediate stringency hybridization conditions is 55°C and 1xSSC. An exemplary set of high stringent hybridization conditions is 68°C and 0.1 x SSC.

Particularly preferred polynucleotides of the invention encode a polypeptide as defined below. By "encode", it is not necessarily implied that the polynucleotide (*e.g.* RNA) is translated, but it will include a series of codons which encode the amino acids of the polypeptides defined below.

The invention also provides a polynucleotide comprising: (a) a nucleotide sequence selected from the group consisting of SEQ IDs 18, 19, 20, 21, 22, 23, 24, 25, 26, 27, 38, 40 and/or 42; (b) a fragment of at least 60 nucleotides of (a) wherein:
(i) the fragment is RNA, and the 5' region and start codon of the env coding region are joined to a downstream coding region in the reading frame +2 relative to that of env;
(ii) the fragment comprises the splice junction;
(iii) the fragment is in the +2 reading frame relative to the start codon of PCAV env; or
(iv) the fragment comprises a region that utilises the +2 reading frame relative to the start codon of PCAV env; or
; (c) a nucleotide sequence having at least 95% identity to (a); or (d) the complement of (a), (b) or (c).

The polynucleotides of the invention are particularly useful as probes and/or as primers for use in hybridization and/or amplification reactions.

More than one polynucleotide of the invention can hybridize to the same nucleic acid target (*e.g.* more than one can hybridize to a single RNA).

References to a percentage sequence identity between two nucleic acid sequences mean that, when aligned, that percentage of bases are the same in comparing the two sequences. This alignment and the percent homology or sequence identity can be determined using software programs known in the art, for example those described in section 7.7.18 of reference 20. A preferred alignment program is GCG Gap (Genetics Computer Group, Wisconsin, Suite Version 10.1), preferably using default parameters, which are as follows: open gap = 3; extend gap = 1.

Polynucleotides of the invention may take various forms *e.g.* single-stranded, double-stranded, linear, circular, vectors, primers, probes *etc.*

Polynucleotides of the invention can be prepared in many ways *e.g.* by chemical synthesis (at least in part), by digesting longer polynucleotides using restriction enzymes, from genomic or cDNA libraries, from the organism itself *etc.*

Polynucleotides of the invention may be attached to a solid support (*e.g.* a bead, plate, filter, film, slide, resin, *etc.*)

Polynucleotides of the invention may include a detectable label (*e.g.* a radioactive or fluorescent label, or a biotin label). This is particularly useful where the polynucleotide is to be used in nucleic acid detection techniques *e.g.* where the nucleic acid is a primer or as a probe for use in techniques such as PCR, LCR, TMA, NASBA, bDNA, *etc.*

The term "polynucleotide" in general means a polymeric form of nucleotides of any length, which contain deoxyribonucleotides, ribonucleotides, and/or their analogs. It includes DNA, RNA, DNA/RNA hybrids, and DNA or RNA analogs, such as those containing modified backbones or bases, and also peptide nucleic acids (PNA) *etc..* The term "polynucleotide" is not intended to be limiting as to the length or structure of a nucleic acid unless specifically indicated, and the following are non-limiting examples of polynucleotides: a gene or gene fragment, exons, introns, mRNA, tRNA, rRNA, ribozymes, cDNA, recombinant polynucleotides, branched polynucleotides, plasmids, vectors, any isolated DNA from any source, any isolated RNA from any sequence, nucleic acid probes, and primers. Polynucleotides may have any three-dimensional structure, and may perform any function, known or unknown. Unless otherwise specified or required, any embodiment of the invention that includes a polynucleotide encompasses both the double-stranded form and each of two complementary single-stranded forms known or predicted to make up the double stranded form.

Polynucleotides of the invention may be isolated and obtained in substantial purity, generally as other than an intact chromosome. Usually, the polynucleotides will be obtained substantially free of other naturally-occurring nucleic acid sequences, generally being at least about 50% (by weight) pure, usually at least about 90% pure.

Polynucleotides of the invention (particularly DNA) are typically "recombinant" *e.g.* flanked by one or more nucleotides with which it is not normally associated on a naturally-occurring chromosome.

The polynucleotides can be used, for example: to produce polypeptides; as probes for the detection of nucleic acid in biological samples; to generate additional copies of the polynucleotides; to generate ribozymes or antisense oligonucleotides; and as single-stranded DNA probes or as triple-strand forming oligonucleotides. The polynucleotides are preferably uses to detect PCA-mRNAs.

A "vector" is a polynucleotide construct designed for transduction/transfection of one or more cell types. Vectors may be, for example, "cloning vectors" which are designed for isolation, propagation and replication of inserted nucleotides, "expression vectors" which are designed for expression of a nucleotide sequence in a host cell, "viral vectors" which is designed to result in the production of a recombinant virus or virus-like particle, or "shuttle vectors", which comprise the attributes of more than one type of vector.

A "host cell" includes an individual cell or cell culture which can be or has been a recipient of exogenous polynucleotides. Host cells include progeny of a single host cell, and the progeny may not necessarily be completely identical (in morphology or in total DNA complement) to the original parent cell due to natural, accidental, or deliberate mutation and/or change. A host cell includes cells transfected or infected *in vivo* or *in vitro* with a polynucleotide of this invention.

### C-POLYPEPTIDE EXPRESSIONPRODUCTS

Where the method is based on polypeptide detection, it will involve detecting expression of a HML-2 polypeptide which is encoded by a transcript produced by a splicing event in which the 5' region of a HERV-K env coding region and the start codon of the env coding region are joined to a downstream coding region in the reading frame +2 relative to that of env in the genome. The polypeptide may or may not be functional in a viral life cycle.

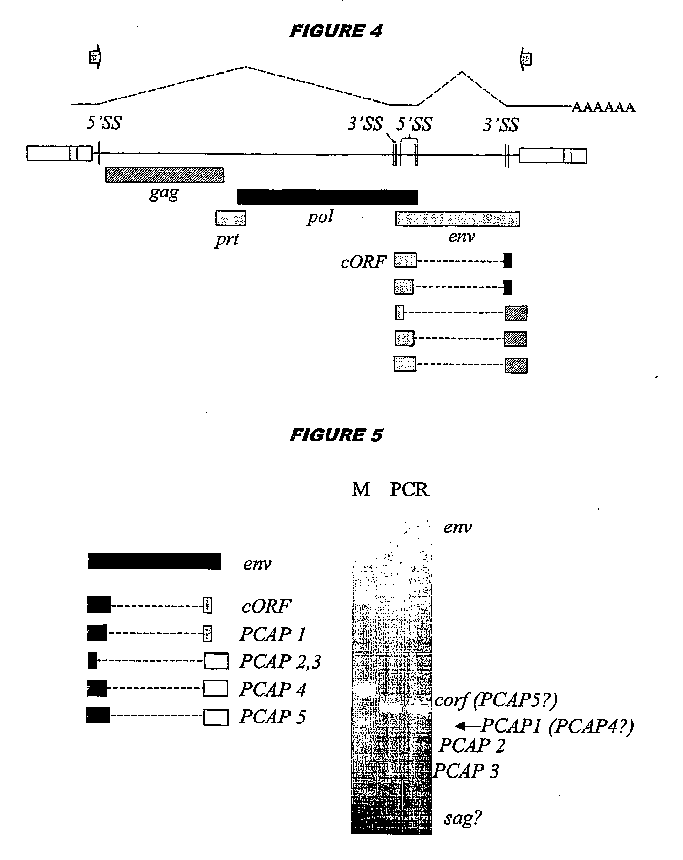

Transcripts which encode HML-2 polypeptides are generated by alternative splicing of the full-length mRNA copy of the endogenous genome {e.g. Figure 4 of ref. 195; Figure 17 herein}.

The polypeptides of the invention are encoded by ORFs which share the same 5' region (and start codon) as env. A splicing event removes env-coding sequences, but the coding sequence continues in the reading frame +2 relative to that of env. Examples of spliced nucleotide sequences are: SEQ IDs 18-27, 38, 40 & 42. Examples of encoded polypeptide sequences are: SEQ IDs 7-12 and SEQ IDs 28, 29, 30, 31, 34, 35, 36, 39, 41, 43, 67, 68 and 69. Some of these (*e.g.* SEQ IDs 10-12) inhibit the function of PCAP4 in a transdominant fashion.

### C.1 - Direct detection of HML-2 polypeptides

Various techniques are available for detecting the presence or absence of a particular polypeptides in a sample. These are generally immunoassay techniques which are based on the specific interaction between an antibody and an antigenic amino acid sequence in the polypeptide. Suitable techniques include standard immunohistological methods, immunoprecipitation, ELISA, RIA, FIA, immunofluorescence *etc.*

In general, therefore, the invention provides a method for detecting the presence of and/or measuring a level of Tat polypeptide of the invention in a biological sample, wherein the method uses an antibody specific for the polypeptide. The method generally comprises the steps of: a) contacting the sample with an antibody specific for the polypeptide; and b) detecting binding between the antibody and polypeptides in the sample.

Polypeptides of the invention can also be detected by functional assays *e.g.* assays to detect binding activity or enzymatic activity. For instance, transcriptionally-active polypeptides of the invention can be assayed by detecting expression of a reporter gene driven by the PCAV LTR, as described in the examples herein.

Another way for detecting polypeptides of the invention is to use standard proteomics techniques *e.g.* purify or separate polypeptides and then use peptide sequencing. For example, polypeptides can be separated using 2D-PAGE and polypeptide spots can be sequenced (*e.g.* by mass spectroscopy) in order to identify if a sequence is present in a target polypeptide.

Detection methods may be adapted for use *in vivo (e.g.* to locate or identify sites where cancer cells are present). In these embodiments, an antibody specific for a target polypeptide is administered to an individual (*e.g.* by injection) and the antibody is located using standard imaging techniques *(e.g.* magnetic resonance imaging, computed tomography scanning, *etc.).* Appropriate labels (*e.g.* spin labels *etc.*) will be used. Using these techniques, cancer cells are differentially labeled.

An immunofluorescence assay can be easily performed on cells without the need for purification of the target polypeptide. The cells are first fixed onto a solid support, such as a microscope slide or microtiter well. The membranes of the cells are then penneablized in order to permit entry of polypeptide-specific antibody (NB: fixing and permeabilization can be achieved together). Next, the fixed cells are exposed to an antibody which is specific for the encoded polypeptideand which is fluorescently labeled. The presence of this label (*e.g.* visualized under a microscope) identifies cells which express the target PCAV polypeptide. To increase the sensitivity of the assay, it is possible to use a second antibody to bind to the anti-PCAV antibody, with the label being carried by the second antibody. {23}

### C.2 - Indirect detection of HML-2 polypeptides

Rather than detect polypeptides directly, it may be preferred to detect molecules which are produced by the body in response to them (*i.e.* indirect detection of a polypeptide). This will typically involve the detection of antibodies, so the patient sample will generally be a blood sample. Antibodies can be detected by conventional immunoassay techniques *e.g.* using PCAV polypeptides of the invention, which will typically be immobilized.

Antibodies against HERV-K polypeptides have been detected in humans {195}.

### C.3 - Polypeptide materials

The invention provides an isolated polypeptide comprising: (a) an amino acid sequence selected from the group consisting of SEQ IDs 7, 8, 9, 10, 11, 12, 28, 29, 30, 31, 34, 35, 36, 39, 41, 43, 66, 67, 68, 69 and 78 to 277; (b) a fragment of at least *x* amino acids of (i) (a) (ii) SEQ IDs 39, 41, 43, wherein the fragment comprises a region which is encoded by a transcript produced by a splicing event in which the 5' region and start codon of the HERV-K env coding region are joined to a downstream coding region in the reading frame +2 relative to that of env in the HERV-K genome; or
(iii) SEQ IDs 39, 41, 43, wherein the fragment comprises a region which is encoded by a transcript produced by a splicing event in which the 5' region and start codon of the HERV-K env coding region are joined to a downstream coding region in the reading frame +2 relative to that of env in the HERV-K genome, and wherein the fragment comprises an epitope; or (c) a polypeptide sequence having at least *s*% identity to (a). These polypeptides include variants (*e.g.* allelic variants, homologs, orthologs, functional and non-functional mutants *etc.).*

The value of x is at least 45 (*e.g.* at least 45, 50, 60, 70, 75, 80, 90, 100 *etc.*). The value ofx may be less than 2000 (*e.g.* less than 1000, 500, 100, or 50).

The value of s is preferably at least 80 (*e.g.* at least 80, 85, 90, 91, 92, 93, 94, 95, 96, 97, 98, 99, 99.5, 99.9 *etc.).*

The fragment of (b) or -B- may comprise a T-cell or, preferably, a B-cell epitope of SEQ IDs 7, 8, 9, 10, 11, 12, 28, 29, 30, 31, 34, 35, 36, 39, 41 43, 66, 67, 68, 69 and 78 to 277. T- and B-cell epitopes can be identified empirically (*e.g.* using the PEPSCAN method {24, 25} or similar methods), or they can be predicted (*e.g.* using the Jameson-Wolf antigenic index {26}, matrix-based approaches {27}, TEPITOPE {28}, neural networks {29}, OptiMer & EpiMer {30, 31}, ADEPT {32}, Tsites {33}, hydrophilicity {34}, antigenic index {35} or the methods disclosed in reference 36 *etc.).* These methods have proved successful in identifying B-cell and T-cell epitopes for HIV tat and HTLV tax{*e.g.* 31, 37, 38, 39, 40, 41, 42, 43, 44, *etc.*}.

Preferred fragments of (b) or -B- are located downstream of the splice site *i.e.* within exon 3. Examples of such fragments are 61 to 68 (or sub-fragments thereof). A polypeptide may include one or more of these sequences. For instance, it may include two or more (*e.g.* 2, 3, 4) of SEQ IDs 62 to 65, preferably in that order (*e.g.* NH₂-O¹-62-O²-63-O³-64-O⁴-65-O⁵-COOH, where O¹ to O⁵ are optional sequences of one or more amino acids), and optionally SEQ ID 61 as well (preferably upstream of SEQ ID 62). Other polypeptides may include SEQ ID 66 and/or SEQ ID 67.

A preferred subset is SEQ IDs 7, 8, 11 and 12 (PCAP2, PCAP3, PCAP4 and PCAP4a).

Preferred polypeptides may bind to RNA comprising SEQ ID 49.

References to a percentage sequence identity between two amino acid sequences means that, when aligned, that percentage of amino acids are the same in comparing the two sequences. This alignment and the percent homology or sequence identity can be determined using software programs known in the art, for example those described in section 7.7.18 of reference 20. A preferred alignment is determined by the Smith-Waterman homology search algorithm using an affine gap search with a gap open penalty of 12 and a gap extension penalty of 2, BLOSUM matrix of 62. The Smith-Waterman homology search algorithm is taught in reference 45.

Polypeptides of the invention can be prepared in many ways *e.g.* by chemical synthesis (at least in part), by digesting longer polypeptides using proteases, by translation from RNA, by purification from cell culture (*e.g.* from recombinant expression), from the organism itself (*e.g.* isolation from prostate or breast tissue), from a cell line source *etc.*

Polypeptides of the invention can be prepared in various forms (*e.g.* native, fusions, glycosylated, non-glycosylated *etc.*).

Polypeptides of the invention may be attached to a solid support.

Polypeptides of the invention may comprise a detectable label (*e.g.* a radioactive or fluorescent label, or a biotin label).

In general, the polypeptides of the subject invention are provided in a non-naturally occurring environment *e.g.* they are separated from their naturally-occurring environment. In certain embodiments, the subject polypeptide is present in a composition that is enriched for the polypeptide as compared to a control. As such, purified polypeptide is provided, whereby purified is meant that the polypeptide is present in a composition that is substantially free of other expressed polypeptides, where by substantially free is meant that less than 90%, usually less than 60% and more usually less than 50% of the composition is made up of other expressed polypeptides.

The term "polypeptide" refers to amino acid polymers of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, *etc.),* as well as other modifications known in the art. Polypeptides can occur as single chains or associated chains. Polypeptides of the invention can be naturally or non-naturally glycosylated (*i.e.* the polypeptide has a glycosylation pattern that differs from the glycosylation pattern found in the corresponding naturally occurring polypeptide).

Mutants can include amino acid substitutions, additions or deletions. The amino acid substitutions can be conservative amino acid substitutions or substitutions to eliminate non-essential amino acids, such as to alter a glycosylation site, a phosphorylation site or an acetylation site, or to minimize misfolding by substitution or deletion of one or more cysteine residues that are not necessary for function. Conservative amino acid substitutions are those that preserve the general charge, hydrophobicity/hydrophilicity, and/or steric bulk of the amino acid substituted. Variants can be designed so as to retain or have enhanced biological activity of a particular region of the polypeptide (*e.g.* a functional domain and/or, where the polypeptide is a member of a polypeptide family, a region associated with a consensus sequence). Selection of amino acid alterations for production of variants can be based upon the accessibility (interior *vs*. exterior) of the amino acid (*e.g.* ref. 46), the thermostability of the variant polypeptide (*e.g.* ref. 47), desired glycosylation sites *(e.g.* ref. 48), desired disulfide bridges *(e.g.* refs. 49 & 50), desired metal binding sites (*e.g.* refs.51 & 52), and desired substitutions with in proline loops (*e.g.* ref. 53). Cysteine-depleted muteins can be produced as disclosed in reference 54.

### C.4 - Antibody materials

The disclosure also includes isolated antibodies, or antigen-binding fragments thereof, that bind to a polypeptide of the invention. The invention also provides isolated antibodies or antigen binding fragments thereof, that bind to a polypeptide encoded by a polynucleotide of the invention.

Antibodies of the invention may be polyclonal or monoclonal and may be produced by any suitable means (*e.g.* by recombinant expression).

Antibodies of the invention may include a label. The label may be detectable directly, such as a radioactive or fluorescent label. Alternatively, the label may be detectable indirectly, such as an enzyme whose products are detectable (*e.g.* luciferase, β-galactosidase, peroxidase *etc.*).

Antibodies of the invention may be attached to a solid support.

Antibodies may be prepared by administering (*e.g.* injecting) a polypeptide of the invention to an appropriate animal (*e.g.* a rabbit, hamster, mouse or other rodent).

Antigen-binding fragments of antibodies include Fv, scFv, Fc, Fab, F(ab')₂ *etc*.

To increase compatibility with the human immune system, the antibodies may be chimeric or humanized {*e.g.* refs. 55 & 56}, or fully human antibodies may be used. Because humanized antibodies are far less immunogenic in humans than the original non-human monoclonal antibodies, they can be used for the treatment of humans with far less risk of anaphylaxis. Thus, these antibodies may be preferred in therapeutic applications that involve *in vivo* administration to a human such as, use as radiation sensitizers for the treatment of neoplastic disease or use in methods to reduce the side effects of cancer therapy.

Humanized antibodies may be achieved by a variety of methods including, for example: (1) grafting non-human complementarity determining regions (CDRs) onto a human framework and constant region ("humanizing"), with the optional transfer of one or more framework residues from the non-human antibody; (2) transplanting entire non-human variable domains, but "cloaking" them with a human-like surface by replacement of surface residues ("veneering"). In the present invention, humanized antibodies will include both "humanized" and "veneered" antibodies. {57, 58, 59, 60, 61, 62, 63}.

CDRs are amino acid sequences which together define the binding affinity and specificity of a Fv region of a native immunoglobulin binding site {*e.g.* refs. 64 & 65}.

The phrase "constant region" refers to the portion of the antibody molecule that confers effector functions. In chimeric antibodies, mouse constant regions are substituted by human constant regions. The constant regions of humanized antibodies are derived from human immunoglobulins. The heavy chain constant region can be selected from any of the 5 isotypes: alpha, delta, epsilon, gamma or mu.

One method of humanizing antibodies comprises aligning the heavy and light chain sequences of a non-human antibody to human heavy and light chain sequences, replacing the non-human framework residues with human framework residues based on such alignment, molecular modeling of the conformation of the humanized sequence in comparison to the conformation of the non-human parent antibody, and repeated back mutation of residues in the framework region which disturb the structure of the non-human CDRs until the predicted conformation of the CDRs in the humanized sequence model closely approximates the conformation of the non-human CDRs of the parent non-human antibody. Such humanized antibodies may be further derivatized to facilitate uptake and clearance *e.g*, via Ashwell receptors. {refs. 66 & 67}

Humanized or fully-human antibodies can also be produced using transgenic animals that are engineered to contain human immunoglobulin loci. For example, ref. 68 discloses transgenic animals having a human Ig locus wherein the animals do not produce functional endogenous immunoglobulins due to the inactivation of endogenous heavy and light chain loci. Ref. 69 also discloses transgenic non-primate mammalian hosts capable of mounting an immune response to an immunogen, wherein the antibodies have primate constant and/or variable regions, and wherein the endogenous immunoglobulin-encoding loci are substituted or inactivated. Ref. 70 discloses the use of the Cre/Lox system to modify the immunoglobulin locus in a mammal, such as to replace all or a portion of the constant or variable region to form a modified antibody molecule. Ref. 71 discloses non-human mammalian hosts having inactivated endogenous Ig loci and functional human Ig loci. Ref. 72 discloses methods of making transgenic mice in which the mice lack endogenous heavy claims, and express an exogenous immunoglobulin locus comprising one or more xenogeneic constant regions.

Using a transgenic animal described above, an immune response can be produced to a PCAV polypeptide, and antibody-producing cells can be removed from the animal and used to produce hybridomas that secrete human monoclonal antibodies. Immunization protocols, adjuvants, and the like are known in the art, and are used in immunization of, for example, a transgenic mouse as described in ref. 73. The monoclonal antibodies can be tested for the ability to inhibit or neutralize the biological activity or physiological effect of the corresponding polypeptide.

### D - COMPARISON WITH CONTROL SAMPLES

### D.1- The control

HML-2 transcripts are up-regulated in tumors. To detect such up-regulation, a reference point is needed *i.e.* a control. Analysis of the control sample gives a standard level of RNA and/or protein expression against which a patient sample can be compared.

A negative control gives a background or basal level of expression against which a patient sample can be compared. Higher levels of expression product relative to a negative control, such as a lifetime baseline or pooled normal samples, indicate that the patient from whom the sample was taken has a tumor. Conversely, equivalent levels of expression product indicate that the patient does not have a HML-2-related tumor.

A positive control gives a level of expression against which a patient sample can be compared. Equivalent or higher levels of expression product relative to a positive control indicate that the patient from whom the sample was taken has a tumor. Conversely, lower levels of expression product indicate that the patient does not have a HML-2 related tumor.

For direct or indirect RNA measurement, or for direct polypeptide measurement, a negative control will generally comprise cells which are not from a prostate tumor cell. For indirect polypeptide measurement, a negative control will generally be a blood sample from a patient who does not have a tumor. The negative control could be a sample from the same patient as the patient sample, but from a tissue in which HML-2 expression is not up-regulated *e.g.* a non-tumor non-prostate cell for a male. The negative control could be a prostate cell from the same patient as the patient sample, but taken at an earlier stage in the patient's life. The negative control could be a cell from a patient without a tumor. This cell may or may not be a prostate cell. The negative control cell could be a prostate cell from a patient with BPH. The negative control could be normal semen, seminal fluid, *etc.*

For direct or indirect RNA measurement, or for direct polypeptide measurement, a positive control will generally comprise cells from the type of tumor in question. For indirect polypeptide measurement, a negative control will generally be a blood sample from a patient who has a prostate tumor. The negative control could be a prostate or breast tumor cell from the same patient as the patient sample, but taken at an earlier stage in the patient's life (*e.g.* to monitor remission). The positive control could be a cell from another patient with a prostate or breast tumor. The positive control could be a prostate cell line.

Other suitable positive and negative controls will be apparent to the skilled person.

HML-2 expression in the control can be assessed at the same time as expression in the patient sample. Alternatively, HML-2 expression in the control can be assessed separately (earlier or later).

Rather than actually compare two samples, however, the control may be an absolute control *i.e.* a level of expression which has been empirically determined from samples taken from tumor patients (*e.g.* under standard conditions).

### D.2 -Degree of up-regulation

The up-regulation relative to the control (100%) will usually be at least 150% (*e.g.* 200%, 250%, 300%, 400%, 500%, 600% or more).

### D.3 - Diagnosis

The invention provides a method for diagnosing prostate cancer. It will be appreciated that "diagnosis" according to the invention can range from a definite clinical diagnosis of disease to an indication that the patient should undergo further testing which may lead to a definite diagnosis. For example, the method of the invention can be used as part of a screening process, with positive samples being subjected to further analysis.

Furthermore, diagnosis includes monitoring the progress of cancer in a patient already known to have the cancer. Cancer can also be staged by the methods of the invention.

The efficacy of a treatment regimen (therametrics) of a cancer can also monitored by the method of the invention *e.g.* to determine its efficacy.

Susceptibility to cancer can also be detected *e.g.* where up-regulation of expression has occurred, but before cancer has developed. Prognostic methods are also encompassed.

The invention is particularly suited to prostate cancer (including prostatic intraepithelial neoplasia).

All of these techniques fall within the general meaning of "diagnosis" in the present invention.

### E - THE PUTATIVE TAR

HIV Tat acts as a transcription factor and its RNA target is the TAR. SEQ IDs 14 and 49 are examples of 150 nucleotide RNAs comprising a putative HML-2 TAR. As for HIV, the minimal tat-binding motif in the TAR may be shorter than these two molecules.

The invention provides an isolated polynucleotide of comprising: (a) the nucleotide sequence of SEQ ID 14 or 49; (b) a fragment of at least *x* nucleotides of (a); (c) a nucleotide sequence having at least *s*% identity to (a); or (d) the complement of (a), (b) or (c).

The isolated polynucleotide is preferably shorter than 250 nucleotides (*e.g.* shorter than 240, 230, 220, 210, 200, 190, 180, 170, 160, or 150 nucleotides).

The value of *x* is at least 7 (*e.g.* at least 8, 9, 10, 11, 12, 13, 14, 15, 16, 17, 18, 19, 20, 21, 22, 23, 24, 25, 30, 35, 40, 45, 50, 60, 70, 75, 80, 90, 100 *etc.).* The value of *x* may be less than 2000 (*e.g.* less than 1000, 500, 100, or 50).

The value of *s* is preferably at least 50 (*e.g.* at least 55, 60, 65, 70, 75, 80, 85, 90, 91, 92, 93, 94, 95, 96, 97, 98, 99, 99.5, 99.9 *etc.).*

The isolated polynucleotide can preferably bind to a protein comprising the amino acid sequence SEQ ID 7, 8 and/or 9 (putative tat analogs).

### F - INHIBITING PCAP FUNCTION

Inhibiting the Tat/TAR interaction has been used for HIV therapy, and inhibition of Tax function has been used for HTLV therapy. By analogy, inhibiting the equivalent functions in PCAV offers ways of treating cancer, and also for treating other diseases linked to HERV-K viruses (*e.g.* testicular cancer {194}, multiple sclerosis {74}, insulin-dependent diabetes mellitus (IDDM) {75} *etc.).*

Various methods have been proposed for inhibiting the Tat/TAR interaction *e.g.*:
- The use of RNA decoys comprising multiple TARs to sequester Tat {76}
- Antisense Tat {77}
- Dominant negative Tat mutants {78}
- Tat Ribozymes {79}
- Anti-TAR hammerhead ribozymes {80}
- The use of small molecule inhibitors of the Tat/TAR interaction {81, 82, 83}
- Use of aptamers {84}
- Use of inhibitory RNAs (siRNAs) for RNA interference {85}

Similar approaches have been used for inhibiting Tax function {86, 87, 88}, although a significant difference between Tax and Tat is that Tat binds nucleic acid directly. All of these methods can be applied to the putative PCAV tat/TAR interaction or Tax function.

The invention therefore provides the following, together with their use as pharmaceuticals and their use in the manufacture of a medicament for treating prostate cancer, testicular cancer, multiple sclerosis and/or insulin-dependent diabetes mellitus:
- A polynucleotide encoding or comprising two or more copies of the putative HML-2 TAR;
- A polynucleotide complementary to a putative tat-coding sequence;
- A polypeptide which can bind to a functional putative tat and act in a transdominant way;
- A ribozyme which can attack tat and/or tar sequences;
- Small molecule inhibitors of the putative Tat/TAR interaction;
- Antibodies or oligobodies {89,90} which specifically bind to putative tat; and
- Aptamer inhibitors of the putative Tat/TAR interaction.
- Small inhibitory RNAs {*e.g.* refs. 91 to 96} complementary to the putative TAR sequence.

In relation to transdominant inhibitors of putative tat function, the invention provides a protein as defined in section C.3 above, comprising: (a) an amino acid sequence selected from the group consisting of SEQ IDs 10, 11, 12 and 13; (b) a fragment of at least *x* amino acids of (a); or (c) a polypeptide sequence having at least *s*% identity to (a). Proteins having amino acid sequences SEQ IDs 10, 11, 12 and 13 have all been found to suppress the activity of putative tat, with SEQ ID 13 (cORF) being the strongest dominant negative.

### G - VACCINES

Tat protein has been used as a vaccine antigen for HIV therapy, and Tax protein has been used as a vaccine antigen for HTLV therapy. Polypeptide vaccines {111,112,113,114,115} and DNA vaccines {116,117} have both been proposed. By analogy, the polypeptides of the invention can be used for immunizing against prostate or breast cancer, and also for treating other diseases linked to HERV-K viruses (*e.g.* testicular cancer, multiple sclerosis, IDDM *etc.*).

The invention therefore provides a composition comprising (a) a polypeptide as defined in section C.3 above and (b) a pharmaceutically acceptable carrier. The invention also provides a composition comprising (a) a polynucleotide encoding a polypeptide as defined above and (b) a pharmaceutically acceptable carrier.

The composition may additionally comprise an adjuvant. For example, the composition may comprise one or more of the following adjuvants: (1) oil-in-water emulsion formulations (with or without other specific immunostimulating agents such as muramyl peptides (see below) or bacterial cell wall components), such as for example (a) MF59™ {118; Chapter 10 in ref. 119}, containing 5% Squalene, 0.5% Tween 80, and 0.5% Span 85 (optionally containing MTP-PE) formulated into submicron particles using a microfluidizer, (b) SAF, containing 10% Squalane, 0.4% Tween 80, 5% pluronic-blocked polymer L121, and thr-MDP either microfluidized into a submicron emulsion or vortexed to generate a larger particle size emulsion, and (c) Ribi™ adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (DetoxTM); (2) saponin adjuvants, such as QS21 or StimulonTM (Cambridge Bioscience, Worcester, MA) may be used or particles generated therefrom such as ISCOMs (immunostimulating complexes), which ISCOMS may be devoid of additional detergent {120}; (3) Complete Freund's Adjuvant (CFA) and Incomplete Freund's Adjuvant (IFA); (4) cytokines, such as interleukins (e.g. IL-1, IL-2, IL-4, IL-5, IL-6, IL-7, IL-12 *etc.*), interferons (e.g. gamma interferon), macrophage colony stimulating factor (M-CSF), tumor necrosis factor (TNF), *etc.*; (5) monophosphoryl lipid A (MPL) or 3-O-deacylated MPL (3dMPL) {*e.g.* 121, 122}; (6) combinations of 3dMPL with, for example, QS21 and/or oil-in-water emulsions {*e.g.* 123, 124, 125}; (7) oligonucleotides comprising CpG motifs *i.e.* containing at least one CG dinucleotide, with 5-methylcytosine optionally being used in place of cytosine; (8) a polyoxyethylene ether or a polyoxyethylene ester {126}; (9) a polyoxyethylene sorbitan ester surfactant in combination with an octoxynol {127} or a polyoxyethylene alkyl ether or ester surfactant in combination with at least one additional non-ionic surfactant such as an octoxynol {128}; (10) an immunostimulatory oligonucleotide (e.g. a CpG oligonucleotide) and a saponin {129}; (11) an immunostimulant and a particle of metal salt {130}; (12) a saponin and an oil-in-water emulsion {131}; (13) a saponin (*e.g.* QS21) + 3dMPL + IL-12 (optionally + a sterol) {132}; (14) aluminium salts, preferably hydroxide or phosphate, but any other suitable salt may also be used (*e.g.* hydroxyphosphate, oxyhydroxide, orthophosphate, sulphate *etc.* {chapters 8 & 9 of ref. 119}). Mixtures of different aluminium salts may also be used. The salt may take any suitable form (*e.g.* gel, crystalline, amorphous *etc.*); (15) chitosan; (16) cholera toxin or *E.coli* heat labile toxin, or detoxified mutants thereof {133}; (17) microparticles of poly(a-hydroxy)acids, such as PLG; (18) other substances that act as immunostimulating agents to enhance the efficacy of the composition. Aluminium salts and/or MF59™ are preferred.

The composition is preferably sterile and/or pyrogen-free. It will typically be buffered around pH 7.

The composition is preferably an immunogenic composition and is more preferably a vaccine composition. The composition can be used to raise antibodies in a mammal (*e.g.* a human).

Vaccines of the invention may be prophylactic (*i.e.* to prevent disease) or therapeutic (*i.e.* to reduce or eliminate the symptoms of a disease).

Efficacy can be tested by monitoring expression of polynucleotides and/or polypeptides of the invention after administration of the composition of the invention. All of the methods previously used in tat-based HIV immunization can be used.

### H - PHARMACEUTICAL COMPOSITIONS

The invention provides a pharmaceutical composition comprising polynucleotide, polypepyide, or antibody as defined above. The invention also provides their use as medicaments, and their use in the manufacture of medicaments for treating cancer.

Pharmaceutical compositions encompassed by the present invention include as active agent, the polynucleotides, polypeptides, or antibodies of the invention disclosed herein in a therapeutically effective amount. An "effective amount" is an amount sufficient to effect beneficial or desired results, including clinical results. An effective amount can be administered in one or more administrations. For purposes of this invention, an effective amount is an amount that is sufficient to palliate, ameliorate, stabilize, reverse, slow or delay the symptoms and/or progression of cancer.

The compositions can be used to treat cancer as well as metastases of primary cancer. In addition, the pharmaceutical compositions can be used in conjunction with conventional methods of cancer treatment, *e.g.* to sensitize tumors to radiation or conventional chemotherapy. The terms "treatment", "treating", "treat" and the like are used herein to generally refer to obtaining a desired pharmacologic and/or physiologic effect. The effect may be prophylactic in terms of completely or partially preventing a disease or symptom thereof and/or may be therapeutic in terms of a partial or complete stabilization or cure for a disease and/or adverse effect attributable to the disease. "Treatment" as used herein covers any treatment of a disease in a mammal, particularly a human, and includes: (a) preventing the disease or symptom from occurring in a subject which may be predisposed to the disease or symptom but has not yet been diagnosed as having it; (b) inhibiting the disease symptom, *i.e.* arresting its development; or (c) relieving the disease symptom, *i.e.* causing regression of the disease or symptom.

Where the pharmaceutical composition comprises an antibody that specifically binds to a gene product encoded by a differentially expressed polynucleotide, the antibody can be coupled to a drug for delivery to a treatment site or coupled to a detectable label to facilitate imaging of a site comprising cancer cells, such as prostate cancer cells. Methods for coupling antibodies to drugs and detectable labels are well known in the art, as are methods for imaging using detectable labels.

The term "therapeutically effective amount" as used herein refers to an amount of a therapeutic agent to treat, ameliorate, or prevent a desired disease or condition, or to exhibit a detectable therapeutic or preventative effect. The effect can be detected by, for example, chemical markers or antigen levels. Therapeutic effects also include reduction in physical symptoms. The precise effective amount for a subject will depend upon the subject's size and health, the nature and extent of the condition, and the therapeutics or combination of therapeutics selected for administration. The effective amount for a given situation is determined by routine experimentation and is within the judgment of the clinician. For purposes of the present invention, an effective dose will generally be from about 0.01mg/kg to about 5 mg/kg, or about 0.01 mg/ kg to about 50 mg/kg or about 0.05 mg/kg to about 10 mg/kg of the compositions of the present invention in the individual to which it is administered.

A pharmaceutical composition can also contain a pharmaceutically acceptable carrier. The term "pharmaceutically acceptable carrier" refers to a carrier for administration of a therapeutic agent, such as antibodies or a polypeptide, genes, and other therapeutic agents. The term refers to any pharmaceutical carrier that does not itself induce the production of antibodies harmful to the individual receiving the composition, and which can be administered without undue toxicity. Suitable carriers can be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art. Pharmaceutically acceptable carriers in therapeutic compositions can include liquids such as water, saline, glycerol and ethanol. Auxiliary substances, such as wetting or emulsifying agents, pH buffering substances, and the like, can also be present in such vehicles. Typically, the therapeutic compositions are prepared as injectables, either as liquid solutions or suspensions; solid forms suitable for solution in, or suspension in, liquid vehicles prior to injection can also be prepared. Liposomes are included within the definition of a pharmaceutically acceptable carrier. Pharmaceutically acceptable salts can also be present in the pharmaceutical composition, e.g. mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington: The Science and Practice of Pharmacy (1995) Alfonso Gennaro, Lippincott, Williams, & Wilkins, or reference 134.

Once formulated, the compositions contemplated by the invention can be (1) administered directly to the subject (*e.g.* as polynucleotide, polypeptides, small molecule agonists or antagonists, and the like); or (2) delivered *ex vivo,* to cells derived from the subject *(e.g.* as in *ex vivo* gene therapy). Direct delivery of the compositions will generally be accomplished by parenteral injection, e.g. subcutaneously, intraperitoneally, intravenously or intramuscularly, intratumoral or to the interstitial space of a tissue. Other modes of administration include oral and pulmonary administration, suppositories, and transdermal applications, needles, and gene guns or hyposprays. Dosage treatment can be a single dose schedule or a multiple dose schedule.

Methods for the *ex vivo* delivery and reimplantation of transformed cells into a subject are known in the art {*e.g.* ref. 135}. Examples of cells useful in *ex vivo* applications include, for example, stem cells, particularly hematopoetic, lymph cells, macrophages, dendritic cells, or tumor cells. Generally, delivery of nucleic acids for both ex vivo and in vitro applications can be accomplished by, for example, dextran-mediated transfection, calcium phosphate precipitation, polybrene mediated transfection, protoplast fusion, electroporation, encapsulation of the polynucleotide(s) in liposomes, and direct microinjection of the DNA into nuclei, all well known in the art.

Differential expression PCAV polynucleotides has been found to correlate with tumors. The tumor can be amenable to treatment by administration of a therapeutic agent based on the provided polynucleotide, corresponding polypeptide or other corresponding molecule (*e.g.* antisense, ribozyme, *etc.).* In other embodiments, the disorder can be amenable to treatment by administration of a small molecule drug that, for example, serves as an inhibitor (antagonist) of the function of the encoded gene product of a gene having increased expression in cancerous cells relative to normal cells or as an agonist for gene products that are decreased in expression in cancerous cells (*e.g.* to promote the activity of gene products that act as tumor suppressors).

The dose and the means of administration of the inventive pharmaceutical compositions are determined based on the specific qualities of the therapeutic composition, the condition, age, and weight of the patient, the progression of the disease, and other relevant factors. For example, administration of polynucleotide therapeutic compositions agents includes local or systemic administration, including injection, oral administration, particle gun or catheterized administration, and topical administration. Preferably, the therapeutic polynucleotide composition contains an expression construct comprising a promoter operably linked to a polynucleotide of the invention. Various methods can be used to administer the therapeutic composition directly to a specific site in the body. For example, a small metastatic lesion is located and the therapeutic composition injected several times in several different locations within the body of tumor. Alternatively, arteries which serve a tumor are identified, and the therapeutic composition injected into such an artery, in order to deliver the composition directly into the tumor. A tumor that has a necrotic center is aspirated and the composition injected directly into the now empty center of the tumor. An antisense composition is directly administered to the surface of the tumor, for example, by topical application of the composition. X-ray imaging is used to assist in certain of the above delivery methods.

Targeted delivery of therapeutic compositions containing an antisense polynucleotide, subgenomic polynucleotides, or antibodies to specific tissues can also be used. Receptor-mediated DNA delivery techniques are described in, for example, references 136 to 141. Therapeutic compositions containing a polynucleotide are administered in a range of about 100ng to about 200mg of DNA for local administration in a gene therapy protocol. Concentration ranges of about 500ng to about 50mg, about 1µg to about 2mg, about 5µg to about 500µg, and about 20µg to about 100µg of DNA can also be used during a gene therapy protocol. Factors such as method of action (*e.g.* for enhancing or inhibiting levels of the encoded gene product) and efficacy of transformation and expression are considerations which will affect the dosage required for ultimate efficacy of the antisense subgenomic polynucleotides. Where greater expression is desired over a larger area of tissue, larger amounts of antisense subgenomic polynucleotides or the same amounts re-administered in a successive protocol of administrations, or several administrations to different adjacent or close tissue portions of, *e.g.*, a tumor site, may be required to effect a positive therapeutic outcome. In all cases, routine experimentation in clinical trials will determine specific ranges for optimal therapeutic effect.

The therapeutic polynucleotides and polypeptides of the present invention can be delivered using gene delivery vehicles. The gene delivery vehicle can be of viral or non-viral origin (see generally references 142, 143, 144 and 145). Expression of such coding sequences can be induced using endogenous mammalian or heterologous promoters. Expression of the coding sequence can be either constitutive or regulated.

Viral-based vectors for delivery of a desired polynucleotide and expression in a desired cell are well known in the art. Exemplary viral-based vehicles include, but are not limited to, recombinant retroviruses (*e.g.* references 146 to 156), alphavirus-based vectors (e.g. Sindbis virus vectors, Semliki forest virus (ATCC VR-67; ATCC VR-1247), Ross River virus (ATCC VR-373; ATCC VR-1246) and Venezuelan equine encephalitis virus (ATCC VR-923; ATCC VR-1250; ATCC VR 1249; ATCC VR-532)), adenovirus vecotrs and adeno-associated virus (AAV) vectors (*e.g.* see refs. 157 to 162). Administration of DNA linked to killed adenovirus {163} can also be employed.

Non-viral delivery vehicles and methods can also be employed, including, but not limited to, polycationic condensed DNA linked or unlinked to killed adenovirus alone {*e.g.* 163}, ligand-linked, DNA {164}, eukaryotic cell delivery vehicles cells {*e.g.* refs. 165 to 169} and nucleic charge neutralization or fusion with cell membranes. Naked DNA can also be employed. Exemplary naked DNA introduction methods are described in refs. 170 and 171. Liposomes that can act as gene delivery vehicles are described in refs. 172 to 176. Additional approaches are described in refs. 177 & 178.

Further non-viral delivery suitable for use includes mechanical delivery systems such as the approach described in ref. 178. Moreover, the coding sequence and the product of expression of such can be delivered through deposition of photopolymerized hydrogel materials or use of ionizing radiation {*e.g.* refs. 179 & 180}. Other conventional methods for gene delivery that can be used for delivery of the coding sequence include, for example, use of hand-held gene transfer particle gun {181} or use of ionizing radiation for activating transferred gene {179 & 182}.

### I- THE HML-2 FAMILY OF HUMAN ENDOGENOUS RETROVIRUSES

Genomes of all eukaryotes contain multiple copies of sequences related to infectious retroviruses. These endogenous retroviruses have been well studied in mice where both true infectious forms and thousands of defective retrovirus-like elements (*e.g.* the IAP and Etn sequence families) exist. Some members of the IAP and Etn families are "active" retrotransposons since insertions of these elements have been documented which cause germ line mutations or oncogenic transformation.

Endogenous retroviruses were identified in human genomic DNA by their homology to retroviruses of other vertebrates {183, 184}. It is believed that the human genome probably contains numerous copies of endogenous proviral DNAs, but little is known about their function. Most HERV families have relatively few members (1-50) but one family (HERV-H) consists of ~1000 copies per haploid genome distributed on all chromosomes. The large numbers and general transcriptional activity of HERVs in embryonic and tumor cell lines suggest that they could act as disease-causing insertional mutagens or affect adjacent gene expression in a neutral or beneficial way.

The K family of human endogenous retroviruses (HERV-K) is well known {185}. It is related to the mouse mammary tumor virus (MMTV) and is present in the genomes of humans, apes and old world monkeys, but several human HERV-K proviruses are unique to humans {186}. The HERV-K family is present at 30-50 full-length copies per haploid human genome and possesses long open reading frames that potentially are translated into viral proteins {187, 188}. Two types of proviral genomes are known, which differ by the presence (type 2) or absence (type 1) of a stretch of 292 nucleotides in the overlapping boundary of the pol and env genes {189}. Some members of the HERV-K family are known to code for the gag protein and retroviral particles, which are both detectable in germ cell tumors and derived cell lines {190}. Analysis of the RNA expression pattern of full-length HERV-K has also identified a doubly-spliced RNA that encodes a 105 amino acid protein termed central ORF ('cORF') which is a sequence-specific nuclear RNA export factor that is functionally equivalent to the Rev protein of HIV {191}. HERV-K10 has been shown to encode a full-length gag homologous 73 kDa protein and a functional protease {192}.

Patients suffering from germ cell tumors show high antibody titers against HERV-K gag and env proteins at the time of tumor detection {193}. In normal testis and testicular tumors the HERV-K transmembrane envelope protein has been detected both in germ cells and tumor cells, but not in the surrounding tissue. In the case of testicular tumor, correlations between the expression of the env-specific mRNA, the presence of the transmembrane env, cORF and gag proteins and antibodies against HERV-K specific peptides in the serum of the patients, have been reported. Reference 194 reports that HERV-K10 gag and/or env proteins are synthesized in seminoma cells and that patients with those tumors exhibit relatively high antibody titers against gag and/or env.

Gag proteins released in form of particles from HERV-K have been identified in the cell culture supernatant of the teratocarcinoma derived cell line Tera 1. These retrovirus-like particles (termed "human teratocarcinoma derived virus" or HTDV) have been shown to have a 90% sequence homology to the HERV-K10 genome {190, 195}.

While the HERV-K family is present in the genome of every human cell, high level expression of mRNAs, proteins and particles is observed only in human teratocarcinoma cell lines {196}. In other tissues and cell lines, only a basal level of expression of mRNA has been demonstrated even using very sensitive methods {197}. The expression of retroviral proviruses is generally regulated by elements of the 5' long terminal repeat (LTR). The activity of HERV-K LTRs is known to be up-regulated by transcriptional factors. Furthermore, the activation of expression of an endogenous retrovirus may trigger the expression of a downstream gene that triggers a neoplastic effect.

The sequence of HERV-K(II), which locates to chromosome 3, has been disclosed {198}.

HML-2 is a subgroup of the HERV-K family {199}. HERV isolates which are members of the HML-2 subgroup include HERV-K10 {189,194}, the 27 HML-2 viruses shown in Figure 4 of reference 200, HERV-K(C7) {201}, HERV-K(II) {198}, and HERV-K(CH).

Because HML-2 is a well-recognized family, the skilled person will be able to determine without difficulty whether any particular endogenous retroviruses is or is not a HML-2. Preferred members of the HML-2 family for use in accordance with the present invention are those whose proviral genome has an LTR which has at least 75% sequence identity to SEQ ID 44 (the LTR sequence from HML-2.HOM {7}). Example LTRs include SEQ IDs 45-48.

### DISCLAIMERS

In some embodiments, the invention may not encompass polypeptides having one of amino acid sequences SEQ IDs 69 to 76, or polypeptides comprising SEQ IDs 69 to 76 {204}.

In some embodiments, the invention may not encompass: (i) nucleic acid comprising a nucleotide sequence disclosed in reference 1; (ii) nucleic acid comprising a nucleotide sequence within SEQ IDs 1 to 225 in reference 1; (iii) a known nucleic acid; (iv) a polypeptide comprising an amino acid sequence disclosed in reference 1; (v) a polypeptide comprising an amino acid sequence within SEQ IDs 1 to 225 in reference 1; (vi) a known polypeptide;(vii) a nucleic acid or polypeptide known as of 7th December 2001 (*e.g*. whose sequence is available in a public database such as GenBank or GeneSeq before 7th December 2001); or (viii) a polypeptide or nucleic acid known as of 10th June 2002 (*e.g*. whose sequence is available in a public database such as GenBank or GeneSeq before 10th June 2002).

### DEFINITIONS

The term "comprising" means "including" as well as "consisting" *e.g.* a composition "comprising" X may consist exclusively of X or may include something additional *e.g.* X + Y.

The term "about" in relation to a numerical value *x* means, for example, *x*±10%.

The terms "neoplastic cells", "neoplasia", "tumor", "tumor cells", "cancer" and "cancer cells", (used interchangeably) refer to cells which exhibit relatively autonomous growth, so that they exhibit an aberrant growth phenotype characterized by a significant loss of control of cell proliferation (i.e. de-regulated cell division). Neoplastic cells can be malignant or benign and include tissue derived from prostate or breast cancer.

The word "substantially" does not exclude "completely" *e.g.* a composition which is "substantially free" from Y may be completely free from Y. Where necessary, the word "substantially" may be omitted from the definition of the invention.

### BRIEF DESCRIPTION OF DRAWINGS

Figure 1 is a schematic representation of a human endogenous retrovirus with a depiction of the HERV-K(CH) polynucleotides and their position relative to the retrovirus.
Figure 2 is a schematic representation of open reading frames within the HERV-K(HML-2.HOM) (also known as 'ERVK6') genome {7}.
Figure 3 shows splicing events described in the prior art for HERV-K mRNAs.
Figure 4 shows multiple splice sites identified near the 5' and 3' ends of the env ORF. The three reading frames are shaded differently. Five multiple-spliced products are shown beneath env ORF, and these are also shown in Figure 5 together with a gel showing PCR products resulting from the primers shown as arrows at the top of Figure 4.
Figure 6 shows the adenovirus vector used in an expression assay to test for tat activity, and Figure 7 shows the results of GFP expression driven from this vector.
Figure 8 shows the vector used to test the activity of PCAP polypeptides, and Figure 9 shows FACS data obtained using this vector in combination with the Figure 6 vector.
Figure 10 shows deletions made in the LTR of PCA-mRNA, and Figure 11 shows GFP expression driven from these LTRs.
Figure 12 shows data on RNA mapping of the 5' end of PCA-mRNA.
Figure 13 shows a predicted secondary structure for SEQ ID 14.
Figure 14 shows northern blot analysis of PCAV transcripts in cancer cell lines. The top arrow on the left shows the position of the genomic mRNA transcript. The next arrow shows the position of the env transcript. The bottom two arrows show the positions of other ORFs. The lanes contain RNA from the following cell lines: (1) Tera 1; (2) DU145; (3) PC3; (4) MDA Pca-2b; (5) LnCaP. Tera 1 is a teratocarcinoma cell line; the others are prostatic carcinoma cell lines.
Figure 15 illustrates the PCR strategy used to detect splice events between the LTRs, and Figure 16 shows the results of this strategy. In Figure 15, the horizontal line represents the HML-2 genome, the vertical lines above the genome are splice sites, and the vertical lines below the genome are ATG codons for gag, pol and env. The approximate positions of forward (F) and reverse (R) PCR primers are also shown.
Figure 17 shows patterns of splicing in HML-2, and Figure 18 shows the diversity of splice junctions in exon 2. Figures 19 to 22 show alignments of: (19) exon 1 in the splice junction region; (20) exon 1.5; (21) exon 2; and (22) exon 3. The numbers in all alignments refer to the positions in GenBank entry Y17832 of a prototype HERV-K sequence.
Figure 23 shows the results of a RT-PCR scanning assay used to map the 5' end of PCAV mRNAs.
Figure 24 gives details of a RNase protection assay. Two antisense probes were used - a long probe (24B) and a short probe (24C). Both probes protected the region shown in 24A. In 24B, the position of the band expected based on the 'usual' 5' end based on the position of the TATA signal is shown, plus the actual band achieved. The three lanes in 24B are: (1) Teral; (2) no RNA; (3) probe, no RNase. The two lanes in 24C are: (1) Tera1; (2) probe, no RNase.
Figure 25 shows the regions deleted with Figure 13 for testing the 5' region of mRNAs, and
Figure 26 shows FACS analysis of GFP expression driven from the deletion mutants.
Figure 27 shows that PCAP4 activates the HERV-K LTR ('LTR62') but not the murine leukemia virus LTR ('MoLTR'). Similarly, Figure 28 shows that PCAP4 can activate the HIV LTR, Fig. 29A shows that activates the EF1A promoter, and Fig. 29B shows it does not activate the CMV promoter.
Figure 30 shows the subcellular localization of PCAP2.
Figure 31 shows various cells plated in matrigel, and Figure 32 shows cells cultured in soft agar.
Figure 33 shows a RT-PCR analysis of various cells {204}. Lane 1 contains 200, 300, 400 and 500 bp markers. For the other lanes, even numbers lanes were obtained with RT and odd numbers were obtained without RT: (2 & 3) primary human lymphocytes; (4 & 5) transformed B cells; (6 & 7) Tera1 cells; (8 & 9) mammary carcinoma biopsy; (10 & 11) seminoma biopsy; (12 & 13) control.
Figure 34 shows the subcellular localization of PCAP4.
Figure 35 shows cells stained with methylene blue after three weeksof culture.
Figure 36 shows the empty pCEP4 vector, and Figure 37 shows NIH3T3 cells after growth for 4 days following transformation with various vectors. Cell density is given in the graph, and the cells themselves are shown at both 1x and 200x magnification.
Figure 38 shows TUNEL analysis of cells expressing (A) PCAP2, (B) PCAP3 or (C) uninfected. In (A) and (B), the multiplicity of infection is 100, 50 and 25 from left to right.
Figure 39 shows PCAP2-transfected PrECs (39B) as well as control PrECs (39A & C). Asterisks show cells with more than one nucleus.
Figure 40 shows bromo-deoxyuridine labeling of PrECs for detecting cell growth.
Figure 41 shows RT-PCR of (41A) cancerous and (41B) normal breast tissue. The positions of PCAP2 and gusB (ßglucuronidase) transcripts are shown.
Figure 42 shows immunofluorescence experiments using an anti-gag monoclonal antibody 5G2 to stain sections of tissue taken from a prostate cancer patient Figure 42A shows a normal prostate gland, 42B shows atrophied tissue, 42C shows a Gleason grade 3 cancer, and 42D shows a Gleason grade 4 cancer.
Figure 43 is a FACS spectrum showing GFP expression from the MDALTR. The three traces from left to right are: (1) uninfected cells; (2) PCAP3; (3) PCAP4.

### MODES FOR CARRYING OUT THE INVENTION

Certain aspects of the present invention are described in greater detail in the non-limiting examples that follow. The examples are put forth so as to provide those of ordinary skill in the art with a complete disclosure and description of how to make and use the present invention, and are not intended to limit the scope of what the inventors regard as their invention nor are they intended to represent that the experiments below are all and only experiments performed. Efforts have been made to ensure accuracy with respect to numbers used (*e.g*. amounts, temperature, *etc*.) but some experimental errors and deviations should be accounted for. Unless indicated otherwise, parts are parts by weight, molecular weight is weight average molecular weight, temperature is in degrees Celsius, and pressure is at or near atmospheric.

### Prostate-associated expression of HML-2 sequences

Reference 1 describes the association of prostate cancer with the up-regulation of expression of the HML-2 subgroups of the HERV-K endogenous retroviruses.

### Splicing patterns of mRNA

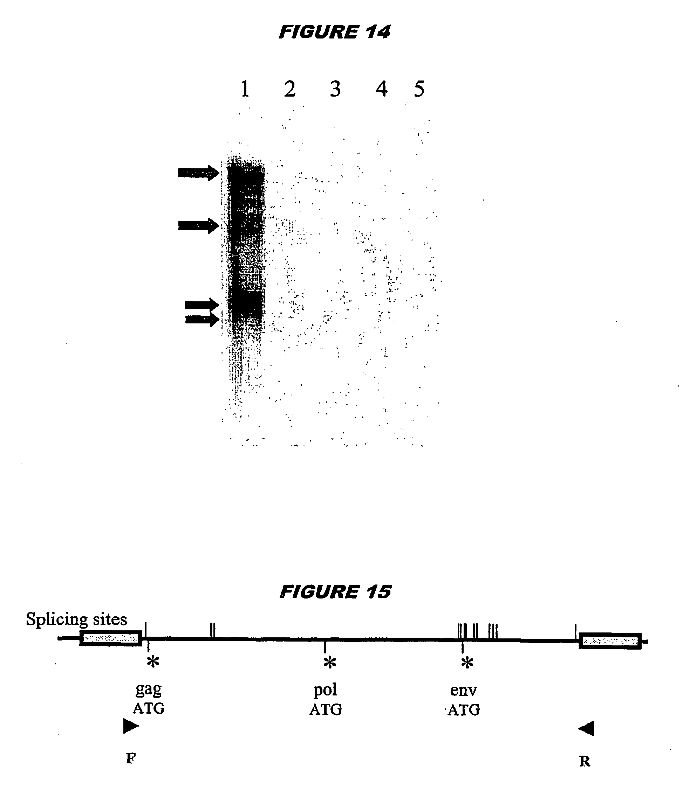

Northern blotting of prostate cancer cell lines indicates that they express PCAV transcripts of several sizes, corresponding to both full-length viral genomic sequences and to sub-genomic spliced transcripts (Figure 14). Expression of such transcripts have also been observed in teratocarcinoma cell lines {4}, as shown in lane 1 of figure 14. To further characterize the splicing patterns of PCAV, a RT-PCR strategy was used that can detect any splice event between the flanking LTRs of the integrated proviral sequences. The approximate position of the forward primer (SEQ ID 15) and reverse primers (SEQ IDs 16 and 17) used in this approach, relative to the general features of the integrated PCAV genome, are indicated by arrows in Figure 15.

DNA fragments corresponding to the transcripts of env and other ORFs could be detected in these experiments only when reverse-transcriptase was included in the RT-PCR reactions (lane 1, 3, 5, 7 and 9 in Figure 16), indicating the they are derived from spliced PCAV mRNAs. These spliced mRNA were detected in the teratocarcinoma cell line Tera 1 (Figure 16, lane 1) and in prostate cancer cell lines DU145, PC3, LNCaP and MDA-PCa-2b (Figure 16). Similar results were also observed in several tumor samples obtained from prostate cancer patients.

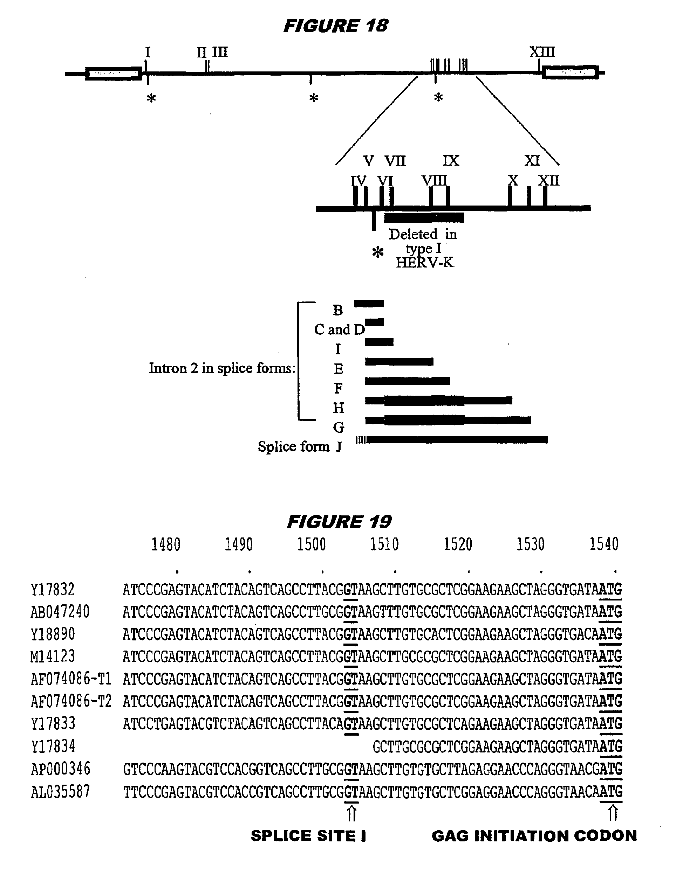

To determine the precise splicing patterns, the RT-PCR products obtained from cell lines and patient tissues were cloned and sequenced. In addition to env spliced mRNA, many other splice variants were seen (named Splice A to J in Figures 17 & 18; see below for consensus sequences of these splice variants). Detailed analysis of these results identify four exons (Exon 1, 1.5, 2 & 3) and 13 splice junctions (named I to XIII in Figures 17 to 22) across HML-2 genomic sequences.

Exon 1 comprises sequences from the transcription start site in the LTR to Splice Site I, as indicated schematically in Figure 17. Splice junction I is conserved among all the integrated copies of HML-2 and is located up-stream of the initiation methionine for gag (Figure 19, between nucleotides 1502 and 1503). All spliced mRNAs examined are precisely spliced at this site, with the exception of the Splice J mRNA, which appears to have removed only the intron between exon 2 and 3 (Figure 17).

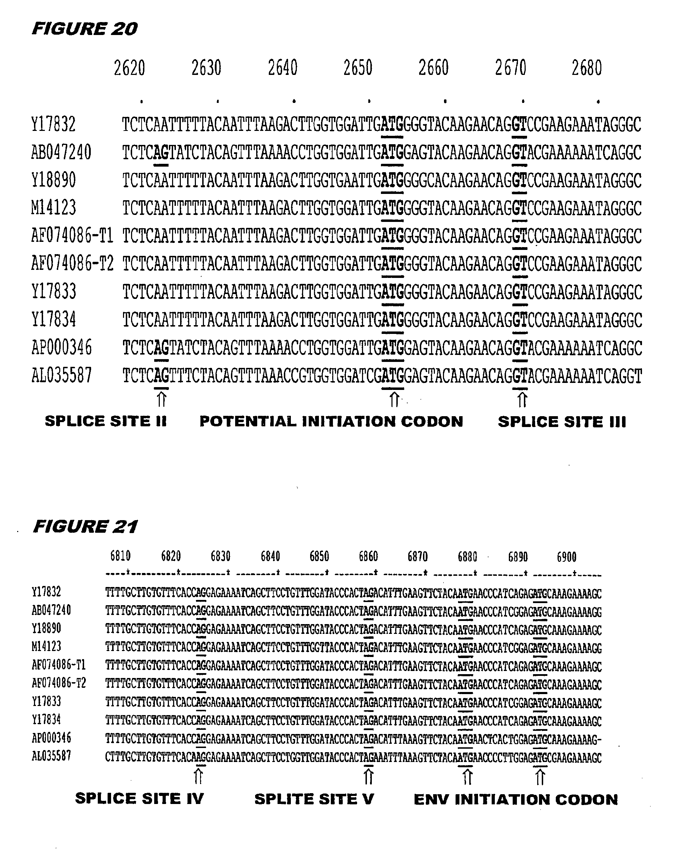

Exon 1.5 is very small and was only detected in the Splice D mRNA (Figure 17). This exon is located in the gag coding sequences (between nucleotides 2624 and 2668 - see Figure 20) and encodes for a potential initiation methionine. Only some of the integrated copies of the PCAV genome contain the AG 3' splice junction consensus sequence found at position 2622-2623 of the prototype Y17832 genome. Probably this represents either a gain or a loss of this intron in some PCAV variants during the evolution as free virus or during primate evolution as integrated viral genomes.

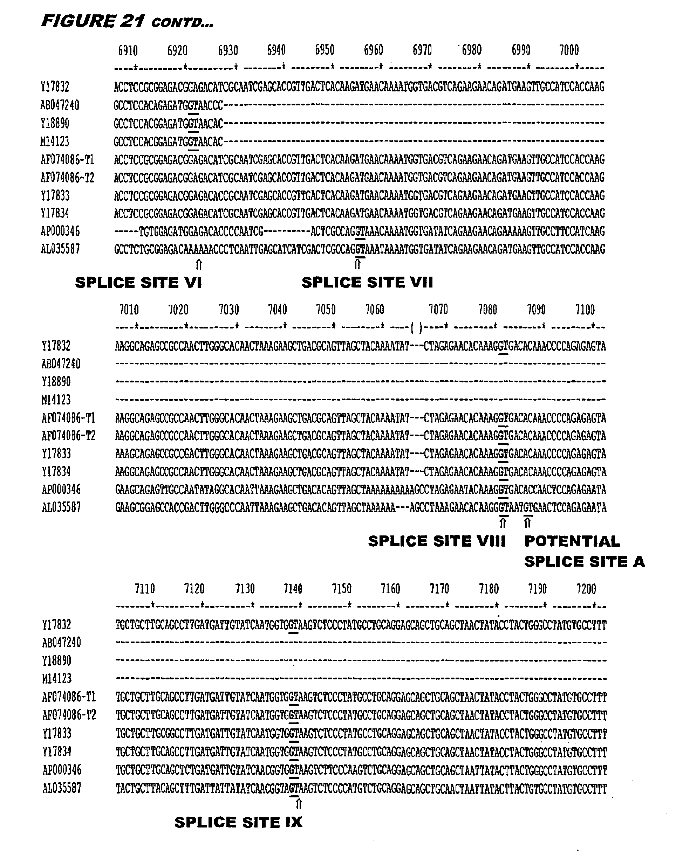

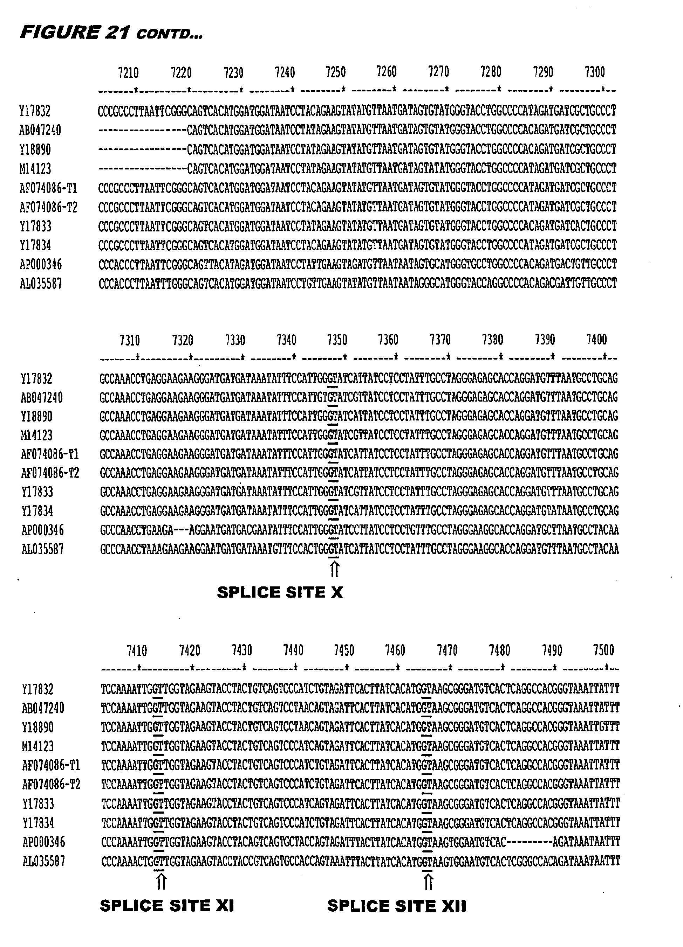

Exon 2 is very heterogeneous, containing two different 3' splice junctions at the 5' end of the exon (Splice Sites IV and V - see Figures 18 and 21) and seven 5' splice junctions at the 3' end of the exon (Splice Sites VI to XII - see Figures 19 and 21). In addition, Exon 2 contains other potential splice sites that were not detected in the experimental analysis. One of these potential sites, indicated in figure 21 as "Potential Splice Site A" may be used to generate a mRNA that encodes for an equivalent of HIV tat or HTLV tax (see below).

The size of Exon 2 in each splice variant depends on which splice sites are used in each independent splice event. Figure 18 summarizes all of the observed splice variants (see also SEQ IDs 18-43), but in principle any other combination is possible with the potential exclusion of exons that are too large for internal exons in mRNA with three or more exons. Adding to the level of complexity, four of these Splice Sites are specific for two PCAV sequence variants found integrated in the human genome. Type I viruses (sequences AB047240, Y18890 and M14123 in Figure 21) contain a deletion of around 300 nucleotides in the N-terminal region of env, about 35-43 nucleotides after the two potential initiation methionine (see Figure 18). Splice Site VI is found only in Type I viruses in close proximity to the site of this deletion (see Figure 21). Splice Sites VII, VIII and IX are only found in the Type II viruses (sequences Y17832, AF074086-T1, AF074086-T2, Y17833, Y17834, AP000346 and AL035587 in Figure 21), as the sequence that contains them is deleted in the Type I virus. Thus, the size of the peptide encoded by exon 2 depends on both the pattern of splicing and on the type of virus from which the mRNA is derived. The initiation methionine for env is present in all detected forms of exon 2 and the open reading frame is open through all the splice sites characterized.

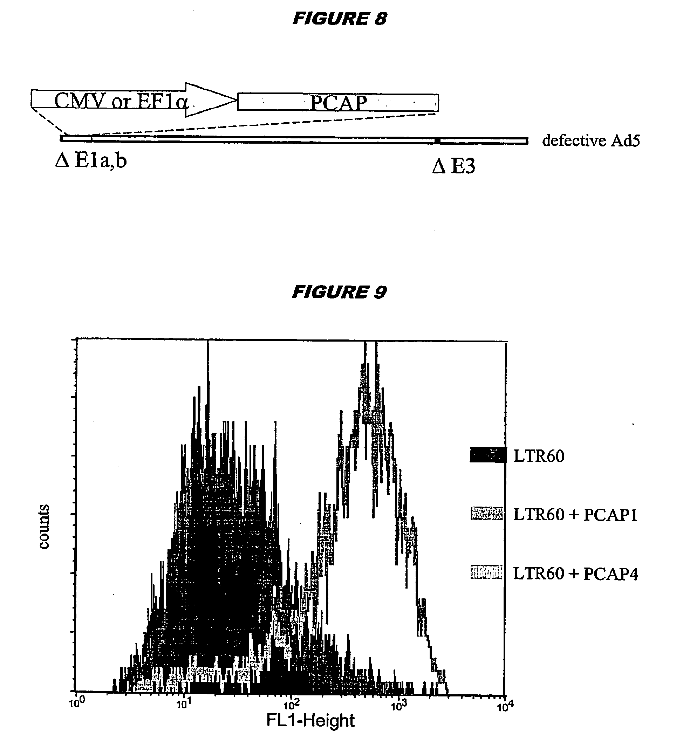

Exon 3 sequences begin about 90 nucleotides before the second LTR (at position 8817 of the prototype sequence Y17832, see Figure 22) and continues into the polyadenylation sites contained within the LTR. All of the splice forms detected use the Splice Site XIII between position 8816 and 8817 (Figure 22). A second potential splice site consensus site is located between position 8824 and 8825 (Figure 22, Potential Splice Site B) but was not observed in any of the cDNA clones analyzed (*i.e.* it may be used at a low frequency). This splice site can also be use to generate a PCAV tat or tax equivalent (see below). In this exon, all three reading frames are open. Frame 1 ends at position 8871 (Figure 22) and adds 18 amino to the encoded polypeptide when in frame with the sequences of exon 2. This reading frame is used in the previously characterized splice form called cORF in which exon 2 at Splice Site IX is joined with exon 3 at Splice site XIII to encode a PCAV *rev* polypeptide. Frame 2 ends at position 8995 (Figure 9) and adds 59 amino acids to the encoded polypeptide when in frame with exon 2. This frame encodes for the PCAV tat/tax equivalent described below. The third frame in exon 3 corresponds to the C-terminus of PCAV *env* and ends at position 8954 in the alignments in Figure 22.

This pattern of splicing and potential to encode multiple products depending of which splice sites are utilized resembles in general the splicing pattern of HIV and HTLV. This suggests that PCAV belongs to the lentivirus type of retroviruses. All possible splice variants are identified as SEQ IDs 18 to 43 (consensus sequences) and are described in Table 1.

### Identification of polypeptide similar to tat or tax

A defining characteristic of lentiviruses is that they encode a polypeptide that can activate transcription from the viral LTR promoter. HIV's tat polypeptide is the best understood example of these activators. The tat gene physically overlaps the rev and env genes in HIV and is made through alternative splicing of HIV mRNA spanning the env region. Tat polypeptide binds to the 5' end of HIV mRNA at a specific site called TAR and provides HIV-specific activation.

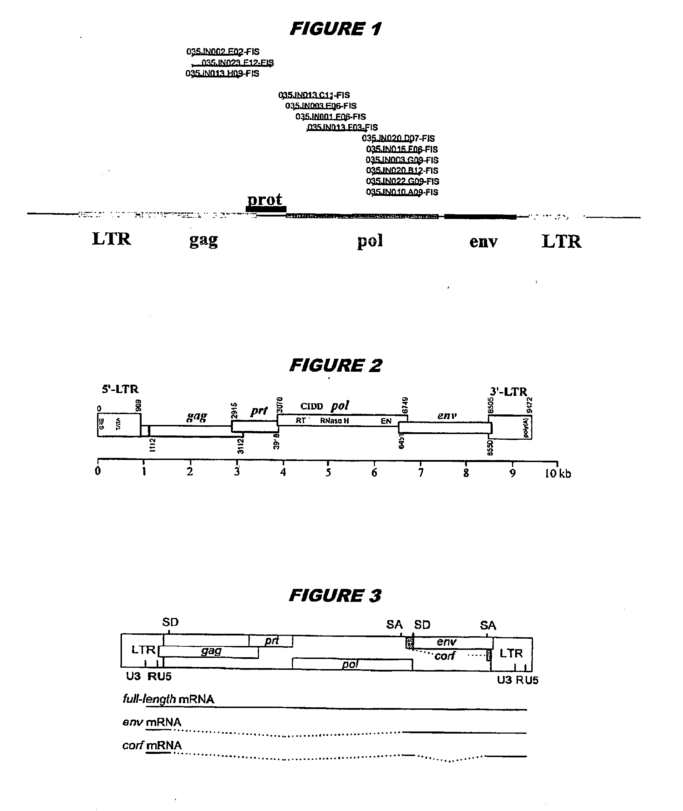

Full-length HERV-K mRNAs can be spliced twice - once to remove gag-prt-pol and once to remove the bulk of the env gene (Figure 3). The polypeptide encoded by the double spliced RNA has been identified and is called 'cORF'. This polypeptide is believed to have activity similar to HIV rev, but a tat polypeptide has not previously been identified for members of the HERV-K family. Spliced PCAV-mRNAs which encode a potential tat homolog have now been identified.

Multiple alternative splice sites in PCAV-mRNAs have been identified (Figures 4 and 5). These indicate that the final exon in the env region can be used in all three reading frames. Frames 1 and 2 encode env and cORF, respectively, but the third frame contains the longest open reading frame of the three. Several alternative mRNAs will connect the first coding exon to this reading frame.

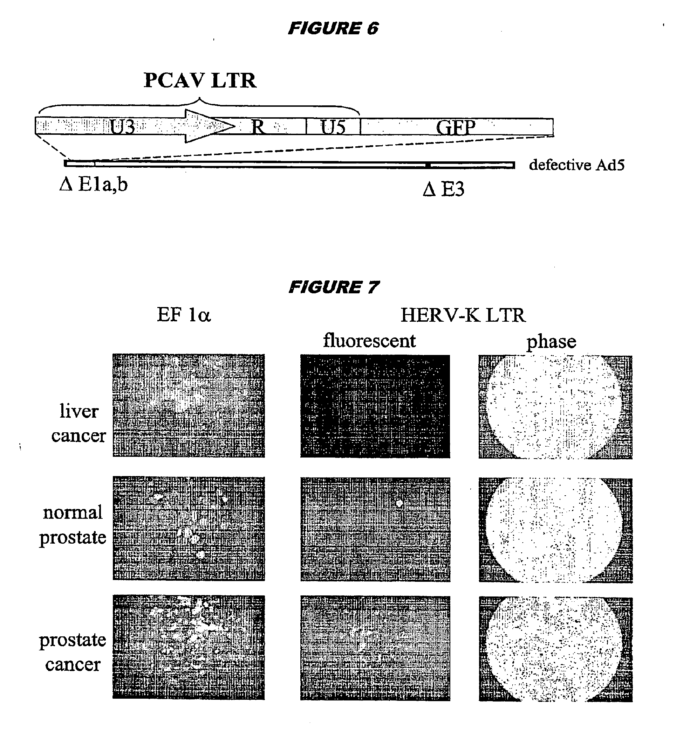

A functional expression assay was designed to determine if the third reading frame in the final env exon encodes a polypeptide with the ability to activate transcription of PCAV-mRNA. The first component of the assay is an adenovirus vector with a PCAV LTR (SEQ ID 45) driving GFP expression (figure 6). A variety of human cell lines were infected with this virus and fluorescence was measured either by fluorescent microscopy or FACS. As a positive control, a vector was used in which GFP expression was driven by the EFα promoter. This should be active in all eukaryotic cells.

GFP expression from this LTR was minimal in ovarian, breast, colon and liver cancer cells. It was also minimal in 293 cells, an immortalized kidney cell line, and also in primary prostate epithelium cells. GFP was easily detected in various prostate cancer cell lines (PC3, LNCaP, MDA2B PCA, DU145). Representative data are shown in figure 7. The GFP expression pattern exactly matches the genomics results from patient samples. These data indicate that expression driven from a PCAV-mRNA LTR is a marker for prostate cancer.

As GFP expression from the LTR appeared to be silent in primary prostate cells and active in prostate cancer, polypeptides from the env region were tested for their ability to activate expression in primary prostate cells. The coding sequences shown in figure 5 were inserted into expression cassettes and these were incorporated into adenovirus vectors. The first coding exon is common to env, rev and the five PCAP products. This exon contains a RNA-binding domain that also functions as a nuclear localization signal (NLS), a polypeptide dimerization region, and a highly hydrophobic sequence. The cORF polypeptide contains all three of these domains fused to a very short region in the terminal exon (figure 4). The PCAP1 transcript encodes a polypeptide using an alternative 5' splice site 57 bases upstream of the normal site and deletes the hydrophobic domain from cORF. PCAP2 is derived from a type I HERV-K deletion that destroys all three domains but connects the env ATG to the third frame in the last exon. PCAP3 is similar to PCAP2, but is based on a different virus where alternative splicing instead of a deletion makes the product. PCAP4 is based on a genomic sequence where a potential 5' splice site 52 bases upstream of the normal cORF site is connected to the 3' splice site used in cORF, and it contains the RNA binding domain and the dimerization region fused to the 3rd coding frame in the last exon. In a separate experiment, it was found that a 3' splice site exists 7 bases downstream of the cORF site. This site was matched to the cORF 5' splice site and the site 57 bases upstream of this site. The product with the upstream site is called PCAP4a and has the same structure as PCAP4 but is missing 4 amino acids. The cORF 5' splice site hooked to the alternative is called PCAP5 and will have the 3 domains hooked to the third coding frame. The label 'sag' in Figure 5 corresponds to the "Splice A" product (see below).

Vectors encoding cORF or the five PCAP products (Figure 8) were co-infected with the GFP vector into primary prostate epithelial cells. Representative FACS data are shown in Figure 9. Three PCAP products were able to activate expression, namely PCAP 4, 4a & 5, whereas PCAP 1, 2 & 3 and cORF all failed to activate expression. PCAP 4a showed the highest activity in this assay.

The interactions of PCAP4 and the non-activating PCAP products were tested by infecting cells with the GFP vector, the PCAP4 vector, and an excess of the vector encoding the non-activating product. PCAP 1, 2 & 3 and cORF could all suppress the activity of PCAP4, with cORF being the strongest dominant negative.

These data suggested that PCAV-mRNAs encode a tat homolog which contains a RNA binding domain (NLS), a polypeptide dimerization region and the third reading frame. The nucleotide sequences that make up this polypeptide product have been known since 1986, but their functional connection via alternative splicing has not previously been reported.

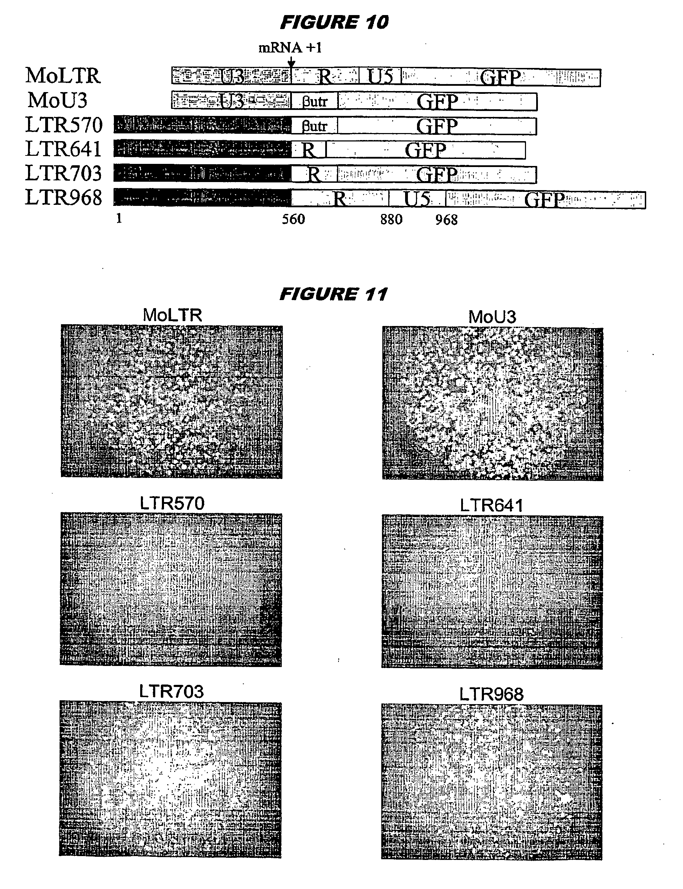

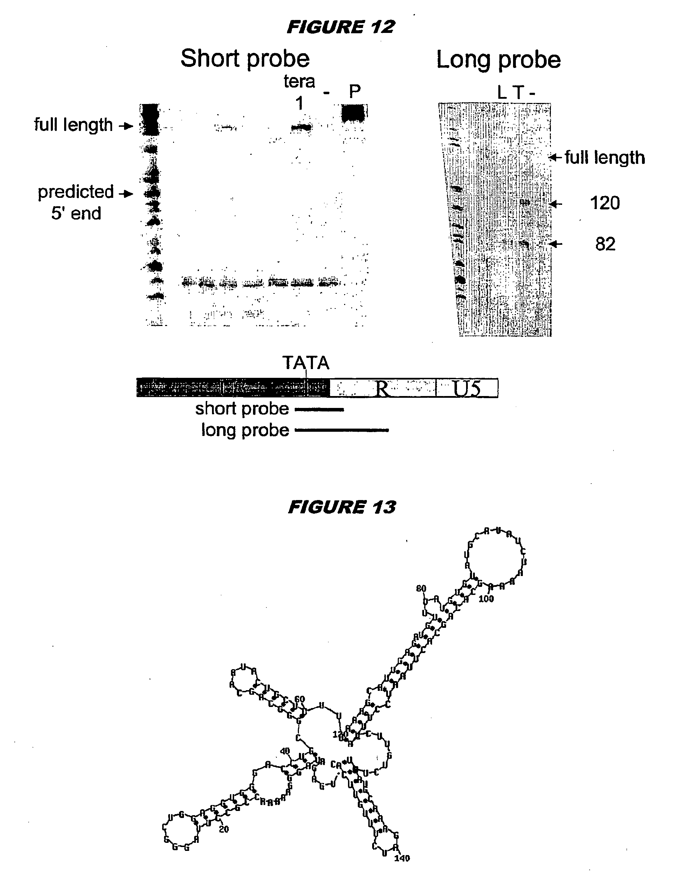

The RNA ligand of tat polypeptide in HIV is the TAR. Potential TAR sites in the LTR of PCAV-mRNAs have been investigated (Figure 10). Deletions in the LTR showed that a region in R has a very strong effect on expression (Figure 11), assuming that the 5' end of PCAV-mRNA falls 30 bases downstream of the canonical TATA sequence (Figure 12). The deletions in mutants LTR570 and LTR641 (Figure 10) would therefore be located in the 5' end of the PCAV mRNA and their effects would be consistent with their being the TAR. Furthermore, the first 150 nucleotides of PCAV mRNA (SEQ ID 14) are capable of forming RNAs with a highly stable secondary structure (Figure 13), like HIV TAR.

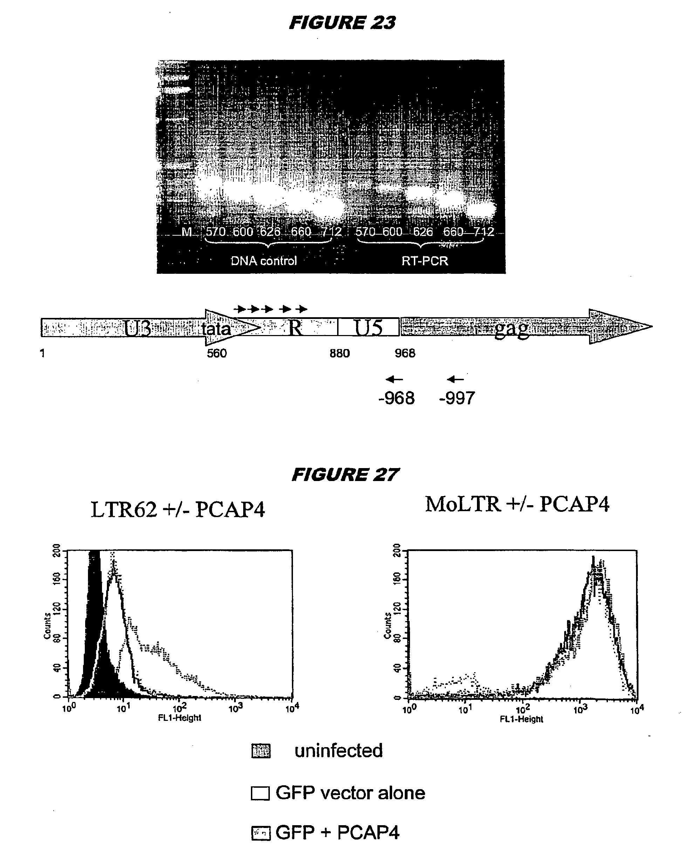

However, other work suggests that the 5' end of PCAV-mRNA is further downstream. Figure 23 shows the results of a RT-PCR scanning assay used to map the 5' end. cDNA of the 5' LTR was prepared by priming total Tera1 RNA with an antisense oligonucleotide spanning 997 to 972 in the proviral genome (SEQ ID 53). This cDNA was then divided and run in PCR analyses with an antisense primer from 968 to 950 (SEQ ID 54) combined with a sense primer from a set of primers designed to cover the likely 5' ends: 1) 571 <SEQ ID 55>, 2) 600 <SEQ ID 56>, 3) 626 <SEQ ID 57>, 4) 660 <SEQ ID 58>, 5) 712 <SEQ ID 59>. Duplicate PCR reactions on 1 µg genomic HeLa DNA were used as a positive control, and these reactions showed all primer pairs were effective. The reactions primed with cDNA showed a marked difference between primers 600 and 626, suggesting that the 5' end lies near position 626 in the proviral genome.

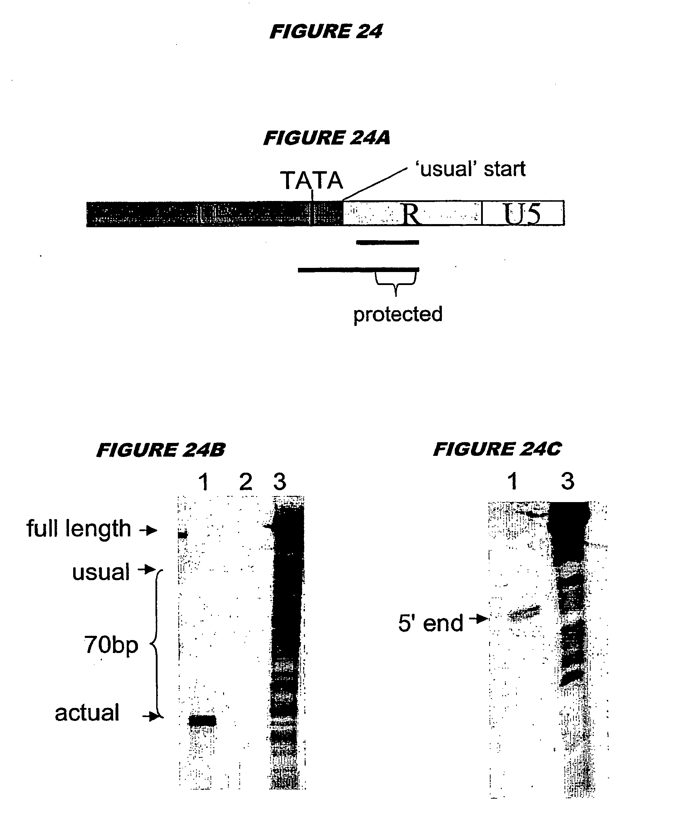

This result was confirmed using RNase protection assays (Figure 24). Labeled antisense RNA probes covering bases (24B) 509-735 and (24C) 600-735 in the proviral genome were hybridized to total RNA from Tera1 cells and digested with RNase under standard conditions. After processing and detection by urea-containing PAGE, both probes gave 100 base products. These two results agree and show that 5' end of HERV-K RNA is around base 635 in the proviral genome i.e. around 100bp downstream of the TATA signal, rather than the 30bp which is usual for TATA-dependent genes.

These two experiments suggest that the deletions used to generate the earlier data may have resulted in deletion of promoter sequences as well as transcribed sequences.

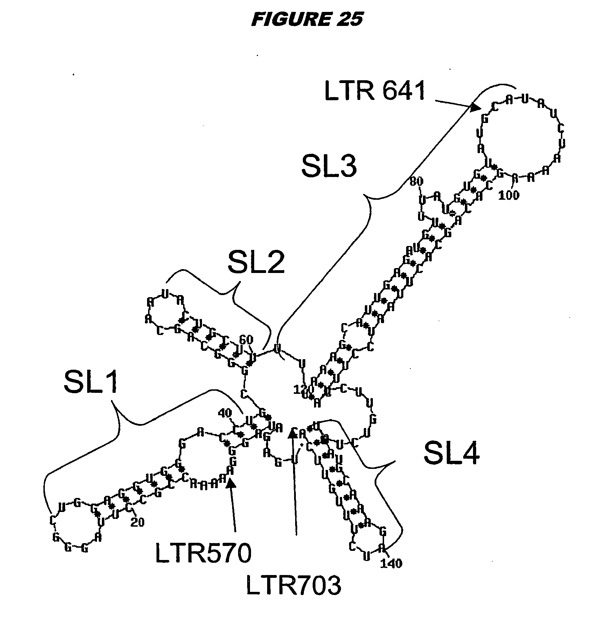

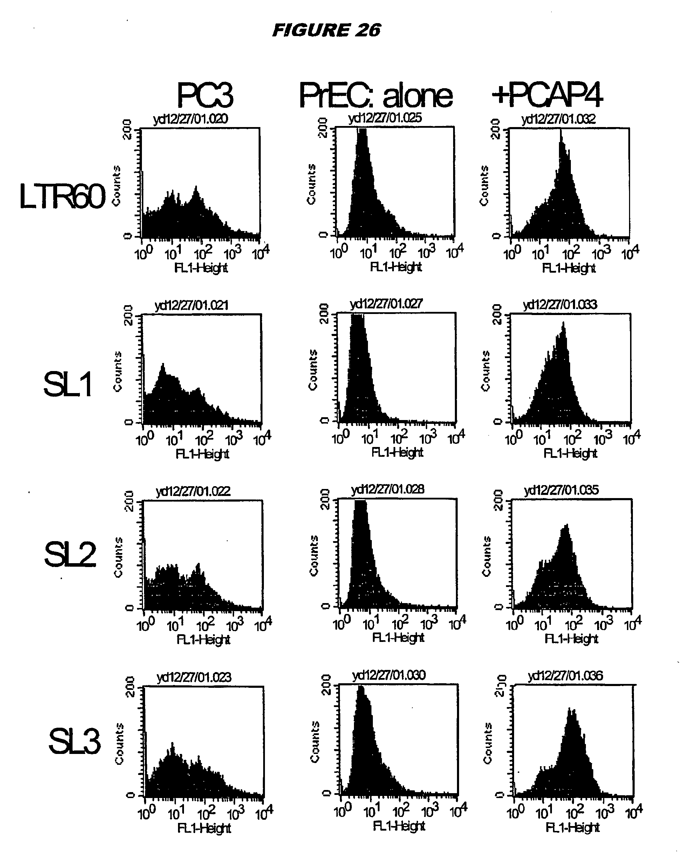

To resolve the discrepancy, stem and loop sequences of the predicted TAR structure (Figure 13) were deleted for LTR60. If PCAV uses a tat/TAR system of transcription then these deletions would greatly diminish transcription. A deletion of each stem and loop (Figure 25) was tested using E1-deleted adenovirus vectors with each LTR deletion mutant driving GFP. PC-3 cells were infected with each vector at a multiplicity of infection (moi) of 50 and fluorescence was measured by FACS after 3 days (Figure 26). The full length and all deletions showed similar GFP expression. The ability of each mutant LTR to be induced by PCAP4 in a co-infection assay in PrEC cells was also tested (Figure 26) and, again, all LTRs were induced to the same extent.

These data therefore indicate that the stem and loop regions are not involved in HERV-K LTR-driven expression, suggesting that PCAV is not controlled using a lentiviral-like tat/TAR system. Another mechanism used by complex retroviruses to activate infected cells for viral expression is the tax type, employed by HTLV I and II. Tax acts at multiple levels in infected T-cells {202}. It up-regulates HTLV transcription by binding to several transcription factors and coactivators, and deregulates the cell cycle by binding to inhibitors of CDK4/6. This combination leads to aberrant differentiation of infected cells in which the virus is activated, and is thought to be instrumental in eventually inducing adult T-cell leukemia in infected individuals. One of the hallmarks of tax-type activation is that multiple promoters respond to tax, as opposed to the high specificity of tat for the HIV TAR.

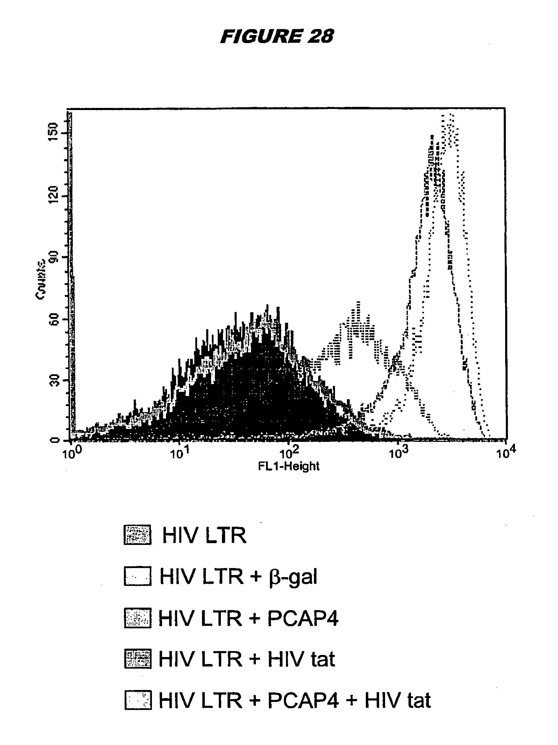

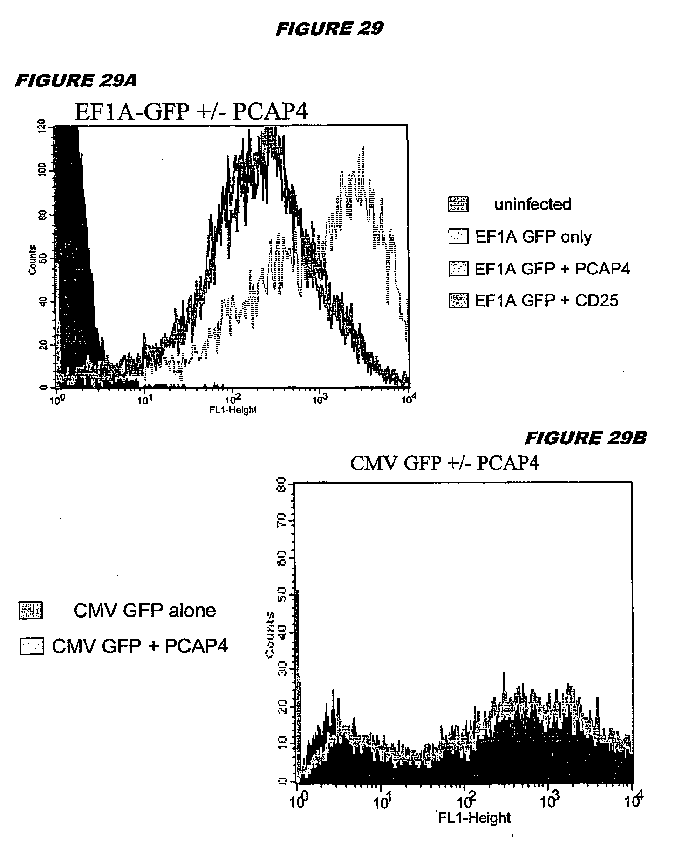

PCAP4 activates HERV-K LTR (LTR60), but not murine leukemia virus (MLV) LTR (Figure 27). Surprisingly, PCAP4 was also found to induce expression from the HIV LTR (Figure 28). In PrEC cells infected with an adenovirus vector carrying the HIV LTR driving GFP, the GFP expression was induced by co-infection with a vector expressing PCAP4 (10 fold), and HIV LTR expression was very strongly activated by co-infection with a tat vector (100 fold), while co-infection with a lacZ vector had no effect. In further experiments on A549 cells the elongation factor 1A promoter (EF1A) was also found to be induced (Figure 29A) whereas the CMV promoter was not (Figure 29B).

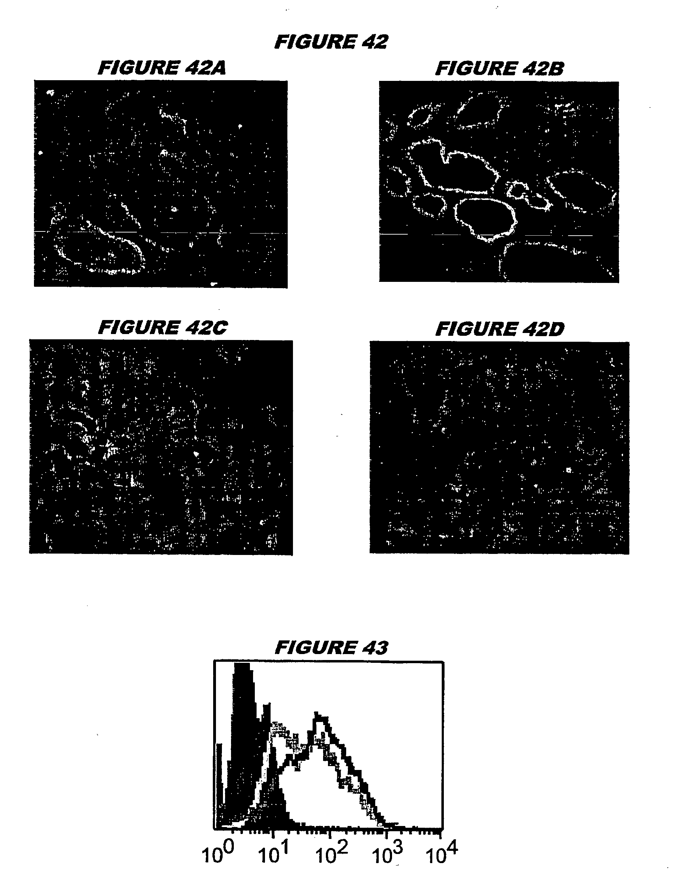

In a separate experiment, high passage PrECs (approaching senescence) were co-infected with an adenovirus vector expressing GFP from an old-type HERV-K LTR ('MDALTR': SEQ ID 77), and a second vector expressing PCAP3 or PCAP4 at moi of about 20. After 3 days, the fluorescent intensity was measured by FACs and activation by PCAP3 and PCAP4 was seen (Figure 43). In a similar experiment with LTR60 and PCAP3, however, there was no activation.

The PCAP proteins of the invention therefore seem more akin to tax than to tat, although the precise mechanism of their action is not important to the basic practice of the invention.

### PCAP2

Within the final exon in the env region of PCAV, reading frames 1 and 2 encode env and cORF, respectively (Figures 4 & 5). SEQ IDs 11, 28,29 and 31 are PCAP2, which shares the same 5' region and start codon as env, but in which the deletion found in type 1 viruses introduces a 5' splice site which joins to a downstream 3' splice site (Figure 4).

The majority of the PCAP2 coding sequence is thus located after the splice, within the exon which contains the 3' LTR. Although the +2 reading frame has no known function in HERV-K, cDNA prepared from prostate tumors included PCAP2-encoding transcripts.

Inspection of various aligned HERV-K genomes suggests that PCAP2 is a mutated form of an original protein. The protein is thus unlikely to be functioning in its original capacity, but oncogenic activity could arise through retention of a functional domain. Retention of activity by fragments is another property which matches tax rather than tat.

### PCAP2 SUB-CELL ULAR LOCALIZATION

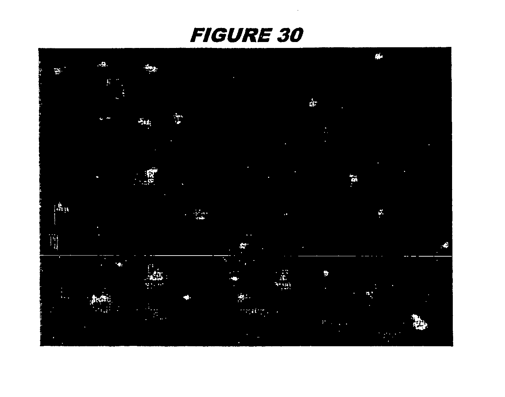

To study the subcellular localization of PCAP2, in order to better understand its role, an adenovirus expressing PCAP2 with a C-terminal V5 tag (SEQ ID 60) was used to infect primary prostate epithelial cells. The protein was not highly expressed, but was visible in the nucleoli using anti-V5 and, more diffusely, throughout the whole cell (Figure 30). The concentration of this small protein in this cellular location shows that it is specifically interacting with something within the nucleus.

These results are consistent with the presence of NLS motifs in PCAP2.

### PCAP2 AND PROSTATE CELL GROWTH

RWPE1 cells were created by immortalizing normal prostate epithelial cells with human papillomavirus 18 {203}. The cells are non-tumorgenic in nude mice and possess markers and growth characteristics of normal prostate epithelial cells.

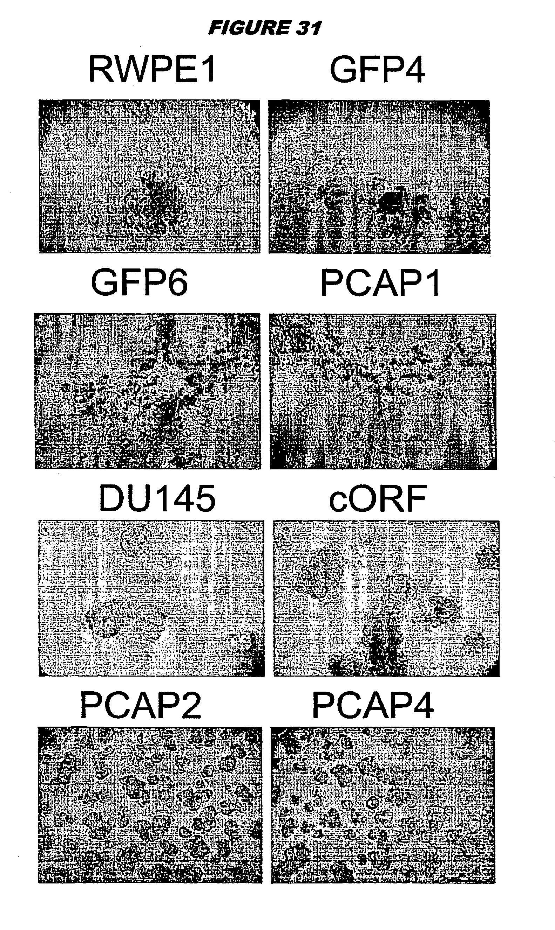

A plasmid expressing PCAP2 from an EF1A cassette was co-transfected into RWPE1 with a puromycin selection marker. Individual resistant colonies were expanded, total RNA was prepared and positive clones were picked based on RT-PCR analysis. To assess growth characteristics, parental cells, DU145 prostate cancer cells, or selected clones were plated into matrigel plus complete keratinocyte serum-free media (complete KSFM is media with bovine pituitary extract and EGF supplements). The plated cells are shown in Figure 31.

Normal prostate epithelial cells and RWPE1 cells migrated toward each other upon plating in matrigel, and over a week these aggregates formed hollow structures reminiscent of a gland. In contrast, DU145 cancer cells seeded solid cored colonies without apparent migration or differentiation. In the cell lines tested, both GFP lines resembled the parent RWPE1, indicating that the introduction of the vector, the selection process and the culture conditions did not change the cells. The cells expressing PCAP1 also behaved similarly to RWPE1. A clone expressing cORF initially aggregated like RWPE1, but then the structure dissolved and the cells took on more of a colony morphology. Three independent PCAP2 colonies failed to aggregate but instead seeded colonies like DU145 cancer cells. These data suggest that PCAP2 interferes with normal prostate cell growth and differentiation.

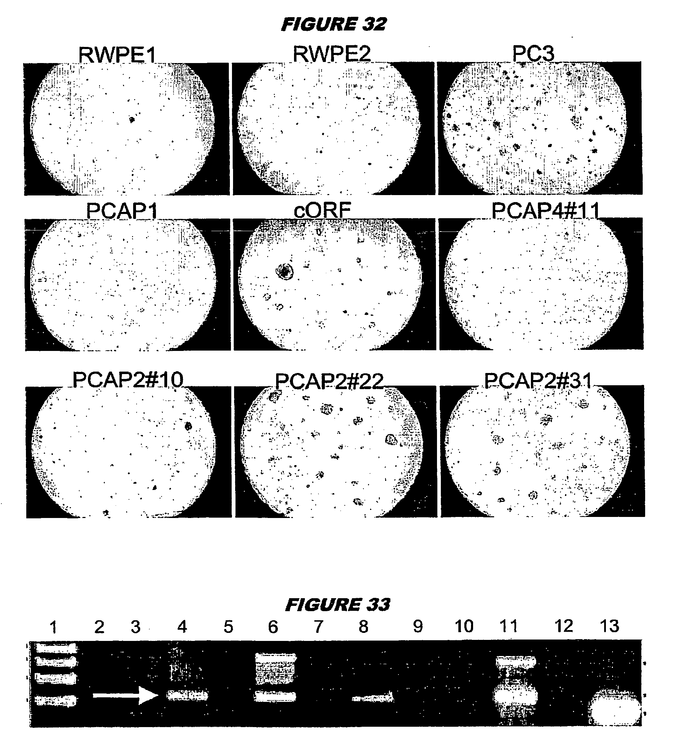

Using the same cell lines, the effect of PCAP2 on anchorage-independent growth of RWPE1 was tested. RWPE1 cells do not grow in 0.35% soft agar, but they do grow at lower agar concentrations (*e.g*. 0.3%). 1,000 cells of each type were plated in complete KSFM plus soft agar (0.35%). As shown in Figure 32, PCAP2-expressing cells grew in soft agar to a similar extent as the positive control PC-3 cells.

### PCAP2 EXPRESSION IN TUMOR TISSUES AND TRANSFORMED CELL LINES

PCAP2 expression has been found to be associated with various tumor tissues and transformed cell lines, but not with normal non-transformed cells {204}. In particular, expression has been seen in mammary carcinoma cell lines and patient tissues.

RNA extracted from tissues or cell lines as described in reference 204 has been analyzed by RT-PCR on a panel of established cell lines, tumor biopsies, lymphocytes from leukemic and normal individuals, and normal non-transformed cells. Figure 33 shows that PCAP2 is expressed in mammary carcinoma and seminoma biopsies, as well as in transformed B cells and Tera-1 teratocarcinoma cells. Expression was seen in >90% of all transformed cell lines (n=15). PCAP2 could be detected in >45% of the samples, but it was not equally distributed among tumor types. It was most frequently seen in mammary carcinomas (52%; n=21), but was less frequently seen in germ-cell tumors (37%; n=8) and leukemia blood lymphocytes (33%; n=6). Two ovarian carcinomas tested negative. In parallel, no healthy tissue (n=14; lymphocytes, fibroblasts, gut, placenta, and stomach) expressed PCAP2. The normal diploid human fibroblasts KH5109 and non-transformed derivatives designed to express the dominant-negative mutant 175H of the p53 tumor suppressor also failed to test positive, as did immortal non-transformed human 041 fibroblasts lacking wild-type p53 and their 175H-transduced derivatives. PCAP2 expression is thus closely correlated with transformation {204}.

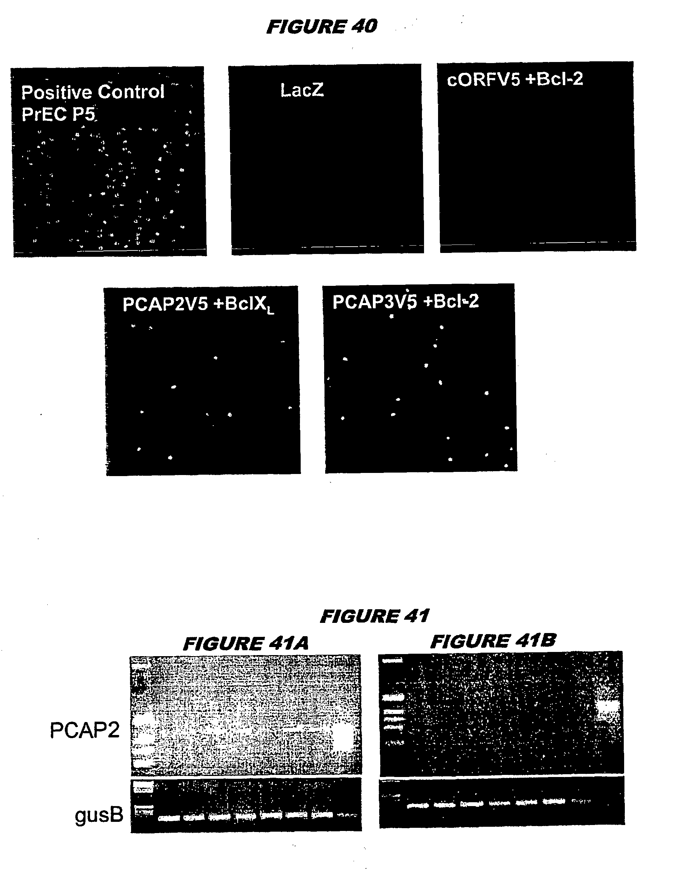

The RT-PCR results in Figure 41 give further evidence of PCAP2 expression breast cancer. RNA was prepared and amplified from seven breast cancer biopsy samples using laser-capture microscospy of tumor tissue and peri-tumor normal tissue. cDNA was prepared with a dT primer and PCAP2 or gusB sequences were amplified using PCR for 30 (gusB) or 35 (PCAP2) cycles. PCAP2 is seen in breast cancer tissue (41A) but not in normal breast tissue (41B).

### PCAP3

SEQ IDs 12 & 36 are PCAP3, which shares the same 5' region and start codon as env, but in which a splicing event removes env-coding sequences and shifts to a reading frame +2 relative to that of env:

ATGAACTCACTGGAGATGCAAAGAAAAGTGTGGAGATGGAGACACCCCAATCGACTCGCCAGgtaaacaaa 8253
M N S L E M Q R K V W R W R H P N R L A s

...cctgttctgtctgttgttagTCTACAGGTGTATCCAGCAGCTCCAAAGAGACAGCAACCAGCAAGAATGGGCCATAG 10480
L Q V Y P A A P K R Q Q P A R M G H S

TGACGATGGTGGTTTTGTCAAAAAGAAAAGGGGGGGATATGTAAGGAAAAGAGAGATCAGACTTTCACTGTGTCTATGTA 10560
D D G G F V K K K R G G Y V R K R E I R L S L C L C R

GAAAAGGAAGACATAAGAAACTCCATTTTGATCTGTACTAA 10601
K G R H K K L H F D L Y *

PCAP3 is thus similar to PCAP2, but the shift into +2 reading frame for PCAP3 is caused by small deletions in a type 2 genome rather than the large deletion seen in type 1 genomes for PCAP2.

cDNA prepared from prostate cancer cell line MDA Pca-2b included PCAP3 transcripts, as did prostate cancer mRNA *e.g.* more than 2-fold in 79% of patient samples and more than 5-fold in 53%. These figures support the view that PCAP3 is involved in many prostate cancers. Furthermore, the figures do not reflect the whole relationship between cancer and PCAP3 expression - if patients are grouped according to Gleason grades, grade 3 tumors show high up-regulation of PCAP3 whereas more developed grade 4 tumors seem to show PCAP3 suppression (Figure 18). A similar pattern is seen with gag expression (Figure 42), suggestion that PCAV expression is involved in the early stages of prostate cancer.

The subcellular localization of PCAP3 was studied in the same way as described above for PCAP2. The protein was relatively stable and was seen in the nucleoplasm. The concentration of this small protein in this cellular location shows that it is specifically interacting with a target in the nucleus.

### PCAP4

As mentioned above, PCAP4 activates expression from the PCAV LTR and also from the HIV LTR PCAP4 is generated following splicing involving a 5' splice site 52 bases upstream of the normal cORF splce site. This splicing event causes a shift into the third reading frame in the last exon.

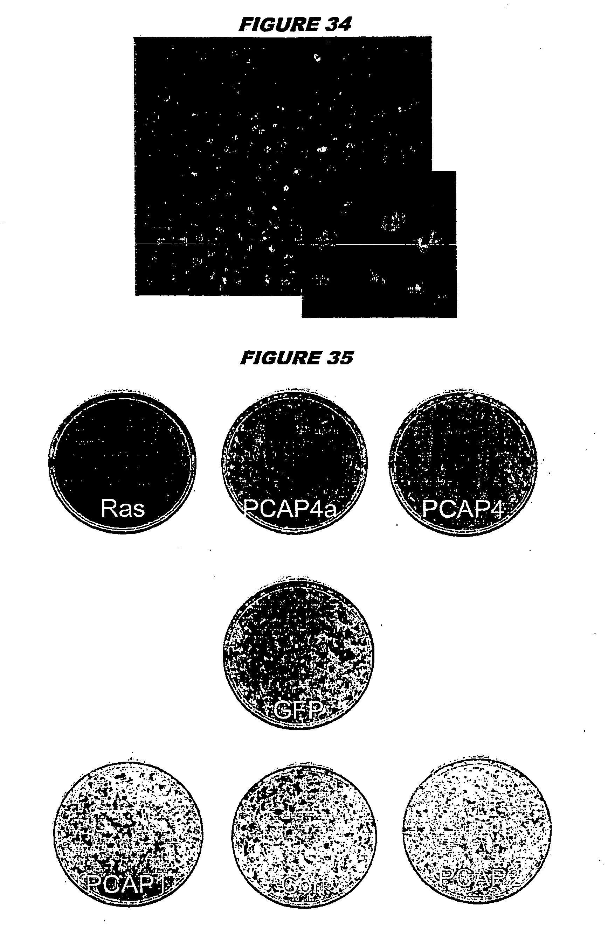

Staining of PCAP4 as described above for PCAP2 and PCAP3 shows nucleolar location (Figure 34). In keeping with nuclear location, PCAP4 shows other activities that suggest a role in cell division. In one experiment, NIH3T3 cells were transiently transfected with expression plasmids encoding GFP, ras with a V12 activating mutation, cORF, PCAP1, PCAP2, PCAP4 or PCAP4a (a splicing variant of PCAP4). These cells were then cultured for three weeks and the overall effect on cell growth was measured by staining the cells with methylene blue (Figure 35). Using the GFP for comparison purposes, PCAP4 and 4a induced proliferation of NIH3T3 cells in the same way as activated ras, whereas the other genes either had no effect or inhibited cell growth.

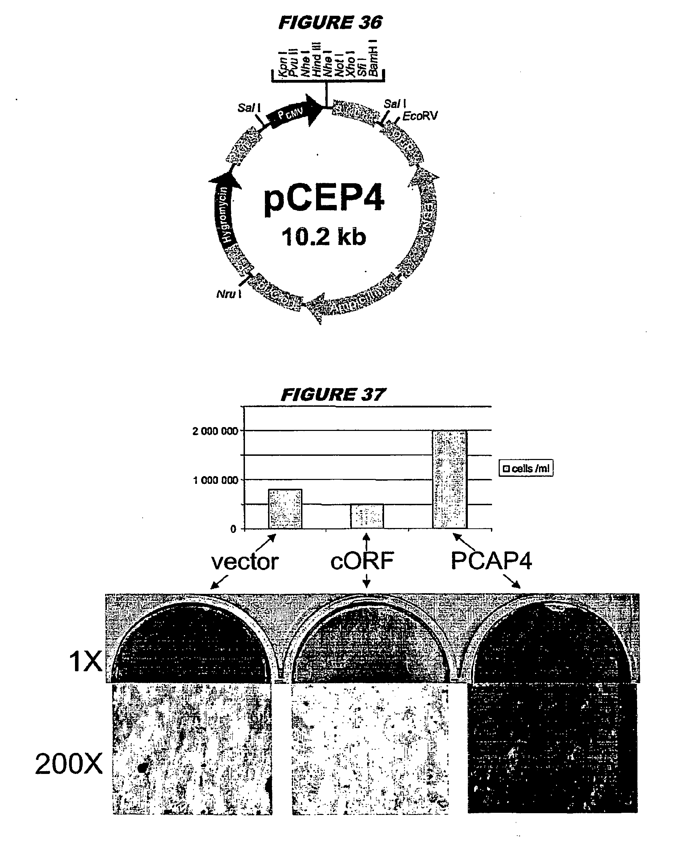

To explore this finding further, stable NIH3T3 cell lines expressing either no extra gene, PCAP4 or cORF were made by inserting the genes in pCEP4, a plasmid with a hygromycin marker (Figure 36). Stable cell pools of each were collected, counted and allowed to grow for 4 days in duplicate wells. One well was stained and photographed, and the other was trypsin treated and counted (Figure 37). Again, PCAP4 promoted growth of NIH3T3 cells and cORF may have slightly suppressed growth. A similar experiment with PCAP3 gave a population of cells that did not expand, but instead appeared to have off-setting high rates of death and division.

Like PCAP2, PCAP4 was able to make RWPE1 cells behave like DU145 cancer cells (Figure 31).

### PCAP PROTEINS AND SENESCENCE

The above data show that PCAP2, PCAP3 and PCAP4, all of which use the third reading frame of exon 3, have a strong effect on the growth properties of immortal cell lines, including on approximately-normal human prostate epithelial cells. This oncogenic potential, combined with their expression in tumor tissue but not normal tissue, suggests a clear link with cancer.

Prostate cancer is believed to arise in the luminal epithelial layer, but normal luminal epithelial cells are capable of very few cell divisions. In contrast, MH3T3 and RWPE1 cells are immortal. Because PCAV seems to be involved in early stages of cancer (see above), the effects of PCAP polypeptides on primary prostate epithelial cells (PrEC), which normally senesce rapidly, were tested.

Primary human epithelial cells have a very limited division potential. After a certain number of divisions the cells will enter senescence. Senescence is distinct from quiescence (immortal or pre-senescent cells enter quiescence when a positive growth signal is withdrawn, or when an inhibitory signal such as cell-cell contact is received, but can be induced to divide again by adding growth factors or by re-plating the cells at lower density) and is a permanent arrest in division, although senescent cells can live for many months without dividing if growth medium is regularly renewed.

Certain genes, particularly viral oncogenes (*e.g*. SV40 T-antigen) force cells to ignore senescence signals. T-antigen stimulates cells to continue division up to a further expansion barrier termed 'replicative crisis'. Two processes occur in crisis: cells continue to divide, but cells die in parallel at a very high rate from accumulated genetic damage. When cell death exceeds division then virtually all cells die in a short period. The rare cells which grow out after crisis have become immortal and yield cell lines. Cell lines typically have obvious genetic rearrangements: they are frequently close to tetraploid, there are frequent non-reciprocal chromosomal translocations, and many chromosomes have deletions and amplifications of multiple loci {205, 206, 207}.

Gene products that lead to crisis are particularly interesting because prostate cancers exhibit high genomic instability, which could be caused by post-senescence replication. Current theory holds that prostate cancer arises from lesions termed prostatic intraepithelial neoplasia (PIN) {208}. Genetic analyses of PIN show that many of the genetic rearrangements characteristic of prostate cancer have already occurred at this stage {209}. PIN cells were thus tested for PCAV expression to determine if the virus could play a role in the earliest stages of prostate cancer. PCAV gag was found to be abundantly expressed, indicating that PCAV expression is high at the time when the genetic changes associated with prostate cancer occur. As PCAP2 and PCAP3 was seen to be expressed in prostate tumors, their roles were investigated by seeing if they are capable of inducing cell division in PrEC after senescence.

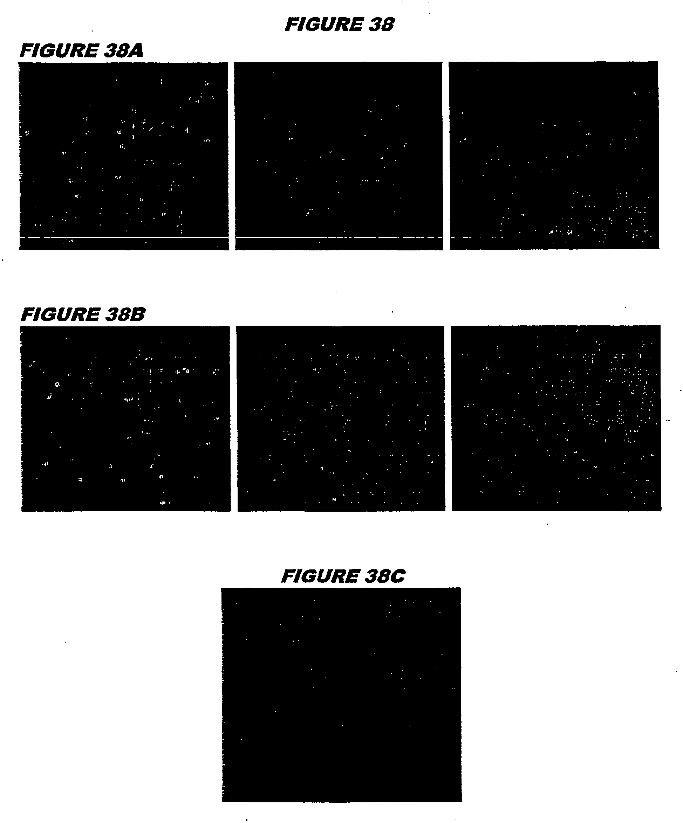

Initial attempts to select drug-resistant PrECs after transfection with PCAP expression plasmids failed. Analysis of PrEC after infection with adenovirus vectors expressing GFP, PCAP2 or PCAP3 revealed abundant cell death on day 4 post-infection in the PCAP cells. A dose-dependent increase in terminal deoxytransferase end labeling (TUNNEL), to mark nuclei with nicked DNA, confirmed that the cells were undergoing apoptosis (Figure 38). This apoptosis may explain the failure to isolate drug-resistant PrECs, and is consistent with engagement of cell division machinery by PCAP3, as an unbalanced growth signal is an inducer of apoptosis.

These results suggested that apoptosis would have to be blocked before the effect of PCAP expression in PrECs could be assessed. Plasmids encoding PCAPs 2, 3 and 4 plus neomycin markers were thus co-transfected with expression plasmids encoding either bcl-2 or bcl-X_{L} to block apoptosis. As controls, cells were transfected with plasmids expressing single proteins. After two weeks under selection, the bcl-2 and bcl-XL dishes all had numerous resistant cells that grew to fill in a fraction of the dish. When these cell were split they failed to divide further, but were viable and resembled senescent parental cells. In contrast, the cells which expressed PCAP2, PCAP3 or PCAP4 plus an anti-apoptosis protein yielded some colonies made up of small cells which divided to fill the initial plate and continued to divide when split.

In parallel to the above drug selections, the growth potential of cells was assessed. The parental PrECs went through seven population doublings before reaching senescence. In contrast, drug-resistant cells co-transfected with an anti-apoptotic gene plus a PCAP expanded well beyond the senescence point before ceasing to grow:

| PCAP product | BCL product | Doublings |
|---|---|---|
| None | None | 8 |
| 2 | Bcl-XL | 20 |
| 3 | Bcl-2 | 16 |
| 4 | Bcl-XI | 20 |

Cells transfected with PCAP4 grew rapidly for around two weeks. Expansion of the cells then slowed and finally ceased. Concomitantly, the number of floating and dead cells increased and the appearance of the cells changed - they no longer had the regular "cobblestone" appearance of epithelial cells, but instead had several morphologies, and there were many multinucleate cells. Cells died 2 weeks later, while the cells transfected with lacZ or lacZ+bcl-2 were still alive 1 month later.

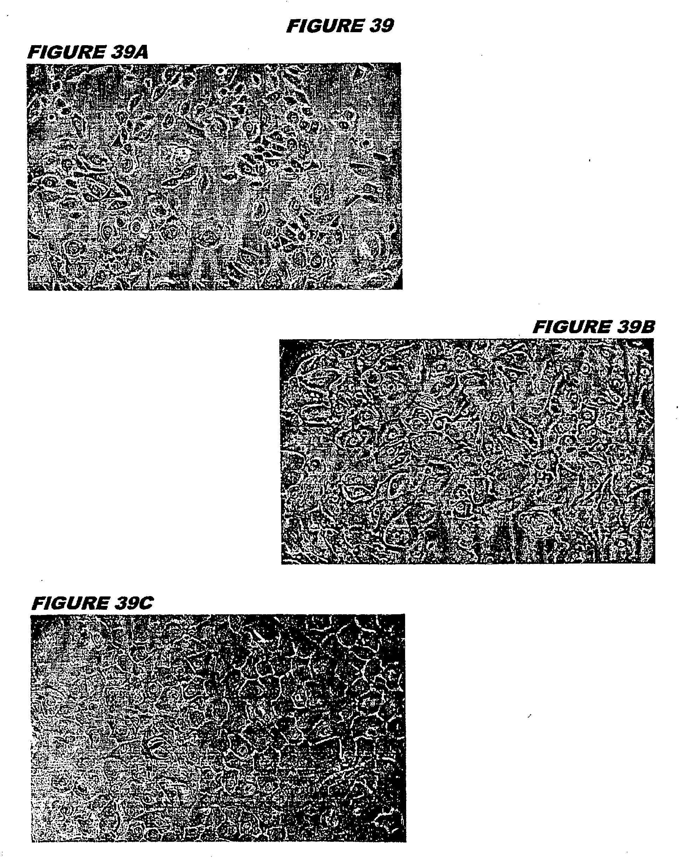

The PCAP2 and PCAP3 cells behaved similarly. Figure 39 shows cultures maintained in supplemented prostate epithelial growth media (PrEM) renewed twice per week (including G418 for transfected cells). Figure 39B shows the PCAP2 + bcl-X_{L} cells at the stage where expansion had ceased in comparison to control cells. Senescent PrEC (Figure 39A) and lacZ transfected cells (Figure 39C) are regular in appearance and have a central, single nucleus in each cell, whereas the PCAP2 cells are irregularly shaped and many have multiple nuclei.

Neither senescent cells nor cells approaching crisis expand in number. One difference between them, however, is that cells approaching crisis are dividing and dying at an appreciable rate, and so cell division can distinguish between the two states. After labeling with bromo-deoxyuridine, 30% of pre-senescent PrECs were labeled, as were 10% of PrEC transfected with either PCAP2 or PCAP3 (plus anti-apoptosis proteins), but none of the senescent lacZ or cORF + bcl-2 controls were labeled (Figure 40).

These results show that PCAP proteins are capable of inducing growth in prostate epithelial cells, and this growth could be an underlying cause of prostate cancer. The ability to drive cells past senescence is another property which matches tax rather than tat.

### PCAP PRODUCTS FROM OTHER HERV-K VIRUSES

The amino acid sequences encoded by the third reading frame of exon 3 for various HERV-Ks found in the human genome are given as SEQ IDs 78 to 277. Nucleotide sequences which encode these 200 amino acid sequences are given as SEQ IDs 278 to 477 although other nucleotide sequences, either found naturally in the human genome or designed artificially, can encode the same amino acid sequences due to codon degeneracy. The amino acid sequences are aligned below:

PCAP2_3rd -----------VYPTAPKRQRPSRTGHDDD----------GGFVEKK-----RGKCGEKQ 34
129 -----------VYPTAPKRQRPSRTGHDDD----------GGFVEKK-----RGKCGEKQ 34
55 -----------VYPTARKRQRPSRTGHDDD----------GGFVEKK-----RGKCGEKQ 34
9 -----------VYPTAPKRQRPSRTGHDDD----------GGFVEKK-----RGKCGEKQ 34
26 -----------VYPTAPKRQRPSRTGHDDD----------GGFVEKK-----RGKCGEKQ 34
54 -----------VYPTALKRQQPSRTGHDDD----------GGFVKKK-----RGKCGEKQ 34
98 -----------VYPTALKRQRPSRTGHDND----------GGFVEKK-----RGKCGEKQ 34
186 -----------LYPTAPKRQRPSRTGHDDD----------SGFVEKK-----RGKCGEKQ 34
224 -------------PTAPKRQRPSRTGHDYD----------GGFVEKK-----RGKCGEKQ 32
25 -----------VYPTAPKRQRPSRTGHDDD----------GGFVEKK-----RGKCGEKQ 34
219 -----------VYPTAPKRQRPSRTGHDDD----------GGFVEKK-----RGKCGEKQ 34
163 -----------VYPTAPKRQRPSRTRHDDD----------GSFVEKR-----RGKCGEKQ 34
164 -----------VYPTAPKRQRPSRTGQDDD----------GSFVEKR-----RGKCGEKQ 34
14 -----------VYRTALKRQRPSRMGHDDD----------GSFVEKK-----RGKCGEKK 34
329 -----------VYLTALKRQRPSRMGHDYD----------GSFVEKK-----RGKCGEKK 34
3 -----------VFPTALKRQRPSRMGHDDD----------GGFVEKK-----RGKCGEKK 34
123 ------------YPTALKRQRPSRTGHDDY----------GSFVKKK-----RGKCGEKK 33
327 -----------VYPTALKRQRPLRTGHDDN----------GSFVEKK-----RGKCGEKK 34
177 -------------PTALKRQRPSRTGHDDD----------GSFVEKK-----RGKCGEKK 32
99 -----------VHPTAPKRQRPSRTGHDDN----------GSFVEKK-----RGKCGEKK 34
148 -----------VYPTAPKRQRPSRTGHDDN----------GSFVEKK-----RGKCGEKK 34
79 -----------VYPTAPKRQRPSRTGHDDN----------GSFVEKK-----KGKCGEKK 34
244 -----------VYPTAPKRQRPSRMGHDDN----------GSFVEKK-----RGKCGEKK 34
228 -----------VYPTAPKRQRSSRMGHDDD----------GSFVEKK-----RGKCGEKK 34
320 -----------VYPTAPKRQRPSRMGHDDD----------GSFVEKK-----RGKCGEKK 34
52 -----------VYPTAPKRQRPSRTGHDDS----------GSFVKKK-----RGKCGEKK 34
240 ----------EVYPTAPKRQRPSRTGHDDN----------GSFVKKK-----RGKCGEKK 35
144 -----------VYPTAPKRQRPSRTGHDDD----------GSFVKNK-----RGKCGEKK 34
264 -----------VYPTAPKRQQPSRTGHDDD----------GSFVKKK-----RGKCGEKK 34
259 -----------VYPTAPKRQRPSRTGHDDD----------GSFVEKK-----RGKCGEKK 34
328 -----------VYPTAPKRQRPSRTGHDDD----------GSFVEKK-----RGKCGEKK 34

34 -----------VYPTAPKRQRPSRTGHDDD----------GGFVEKQ-----RGKCGEKK 34
321 -----------VYPTAPKRQRPSRTGHDDD----------GSFVEKQ-----RGKCGEKK 34
27 -----------VYPTAPKRQRPSRTGHDDS----------GGFVEKK-----RGKCGEKK 34
47 -----------VYPTAPKRQRPSRTGHDDN----------GGFVEKK-----RGKCGEKK 34
36 -----------VYPTAPKRQRPSRTGHDDD----------GGFVEKK-----RGKCGGKN 34
108 -----------VYPTAPKRQRPSRTGHDDD----------GGFVEKK-----RGKCGGKK 34
15 -----------VYPTAPKRQRPSRTGHDDD----------GGFVEEK-----RGKCGAKK 34
102 ----------EVYPIAPKRQRPSRTGHDDD----------GGFVEKK-----RGKCGEKK 35
322 -----------VYPAAPKRQRPSRTGHDDD----------GGFVEKK-----RGKCGEKK 34
31 -----------VYPTAPKRQRPSRTGHDDD----------GGFVEKK-----RGKCGEKK 34
PCAP3 -----------VYPAAPKRQQPARMGHSDD----------GGFVKKK-----RGGYVRKR 34
172 -----------VYPAAPKRQQPARMGHSDD----------GGFVKKK-----RGGYVRKR 34
302 ---------LQVYPAAPERQRPARRDHDDH----------GGFVKKK-----SGKCREKR 36
303 ---------LQVYPAAPERQRPARRDHDDH----------GGFVKKK-----SGKCREKR 36
296 ---------LQVYPAAPERQRPGRRGHDDH----------GGFVKKK-----SGKCREKR 36
CHR8_3rd ---------LQVYPAAPERQRPARRGHDDH----------GGFVKKK-----SGKCREKR 36
208 -----------VYPAAPERQRPVRRGHDDD----------GGFVKKK-----RGKCREKR 34
212 -----------LYPAAPERQRPARRGHDDG----------GGFFKTK-----RGICREKK 34
20 -----------VYPTAPKRQRPSRTGQYDD----------GSFVKKKRGRKEKGEMWGKE 39
32 -----------VYPTAPKRQRPSRTGHDDD----------GGFVEKK----EKGEMWGKE 35
140 -----------------RRERPSRTSHDDN----------GGFVEKK------GEMWGKE 27
156 -----------VYPIAPKRQRTSRTGHDDN----------GGFVEKK------REMWGKE 33
282 -----------VYPTAPKRQRPSRTGHDDD----------RGFVKKK-----WGKMWGKK 34
7 -----------VYPAAPKRQRPSRTSHDDD----------GGLSKRK---------WGNV 30
17 -----------VYPTAPKRQRPSRTGHDDD----------GGFVEKK------RRKSGEK 33
201 -----------VYPTAPKRQRPSRTGHDDD----------GGFVEKR------RGKCGEK 33
254 -----------LYPTAPKRQRPLRMGHDAD----------GGFVEKK-----RGKCGEKK 34
312 -----------LYPTAPKRQRPSRMGHDDD----------GGFVKKK-----RGKCGGKR 34
239 -----------VYPAAPKRQRPSRTGHDDD----------GSFVKNK-----RE-NVGKR 33
319 -----------VYPTAPKRQRPSRMGHDDD----------GGFVEKK-----GG-NVEKR 33
242 -----------VYPTAPKRQRPSRTGHDERAM-----MTMAVLLKRK-----GG-NAGKR 38
333 -----------VYSTAPKRQRPGRMGHDD---------V--AVLSKRK-----GG-NVGKR 33
213 -----------VYPTAPKRKRPSRMGHDDN----------GGFVEKK-----RG-NVGKR 33
190 -----------VYPTSPKRQRPSRTGHDDD----------GGFVEKK-----RG-NVGKR 33
84 -----------VYPTAPKKQQPSIMGHDDD----------GGFVKKK-----RGKCGEKR 34
149 -----------VYPTAPKRQQPSRTGHDDD----------GSFVKKK-----RGKCGEKR 34
135 -----------VYPTAPKRQRPSRTGHDDD----------GGFVQKK-----RGK-WEKR 33
226 -----------VYPTAPKRQRPSRTGHDDD----------GSFVIKK-----RGKRGEKR 34
51 -----------VYPTAPKRQRPSRTGHDDD----------GGFVEKK-----RGKCGEKR 34
71 -----------VYPTALKRQRPSRTGHDDD----------GGFVEKK-----REKCGEKK 34
176 -----------VYPTALKRQRPSRTGHDDD----------GSFVEKK-----RGKCGEKR 34
261 -----------VYPTAWKRQRPSRMGHDDD----------GGFVEKK-----RGKCGENR 34
94 -----------VYPTAPKRQRLSRTGHDDD----------GGFVEKK-----RGKCGEKK 34
233 -----------VYPTAPKRQRPSRTGHDDN----------GGFVEKK-----RGKCGEKK 34
69 ---------LQVYPTALKRQQPSRTGHDDD----------GSFVEKK-----RGKCGEKK 36
183 -----------VYPTAPKRQRPLRTGHDDD----------GSFVEKK-----RRKCGEKR 34
268 -----------VYPTAPKRQRPSRTGHDDD----------GAFVEKK-----RGKCGEKK 34
19 -----------VYPTAPKRQRPSRTGHDDD----------GGFVRKK-----RGKCGEKK 34
246 -----------VYATALKRQRPSRTGHDDD----------GGFVEKK-----RGKCGEKK 34
335 -----------VYPTAPKRQRPSRTGHDDD----------GGFAEKK-----RGKCGEKK 34
116 -----------VYPTAPKRQRPSRTGHDDD----------GGFVEKK-----RGKCGEKK 34
63 -----------VYPTAPKRQRPSRKGHDDD----------GGFVEKK-----RGKCGEKK 34
73 -----------VYPTAPKRQPPSRTGHDDD----------GGFVLKK-----RGKCGEKK 34
74 -----------VYPTAPKRQRPSRTGHDDD----------GGFVEKK-----RGKCGEKK 34
109 -----------VYPTALKRQRPSRTGHDDD----------GGFVEKK-----RRKCGEKK 34
111 -----------VYPTAPKRQRPSRTGHDDD----------GGFVEKK-----RRKCGEKK 34
83 -----------VYPTAPKRQRPSRTGSDDD----------GGFVEKK-----RGKCGEKK 34
235 -----------------RRDRPWRTGHDDD----------GGFVEKT-----RGKCGEKK 28
332 -----------MYPTPLKRQRPWRTGHDDN----------GGFVEKK-----RGKCGEKK 34
251 -----------VYPTALKRQRPWRTGHDDD----------GGFVEKK-----RGKCGEKK 34
162 -----------VYPTAPKRQRPWRTGHDDD----------GGFVEKK-----RGKCGEKK 34
315 -----------VYPTALKRQRPSRTGHDDD----------GGFVEKK-----RGKCGEKK 34
1 -----------VYPTAPKRQQPSRTGHDND----------GSFVEKR-----RGKCGEKK 34
203 -----------VYPTAPKRQQPSRTGHDDD----------GSFVEKK-----RGKCGEKK 34
13 -----------VYPTAPKRQQPSRTGHDDD----------GCFLEKK-----RGKCGEKK 34
96 -----------VYPTAPKRQQPSRTGHDDD----------GGFVKNK-----RGKRGEKK 34
217 -----------VYPTAPKRQQPSRTGHDDD----------GGFVEKK-----RGKRGEKK 34
198 ------------YPTAPKRQQPWRTGLDDL----------GGFFEKK-----RGNFGEKK 33

199 ------------VYPTAPKRQQPWRTGHDDH-----------GGFVEKK-----RGKCGEKK 34
269 ------------VYPTAPKRQQPLRTGHNDD-----------GGFVEKK-----RGKYGEKK 34
35 ------------VYPTAPKRQQPSRTGHDED-----------GGFVERK-----RGNCGEKK 34
24 ------------VYPTAPKRQQPSRMGHDDD-----------GGFVKKK-----RGKCGEKK 34
113 -----------EVYPTSPKRQQPSRMGHDDD-----------GGFVAKK-----RGKCGEKK 35
130 ------------VYPTAPKRQQPSRMGHDDN-----------GGFVEKK-----RGKCGEKK 34
318 ------------VYPTAPKRQQPSRMGHHDD-----------GGFVEKK-----RGKCGEKK 34
29 ------------VYPTARKRQQPSRTGHDDD-----------GGFVVKK-----RGKCGEKK 34
232 ------------VYPTALKRQQPSRTGHDDD-----------GGFVEKK-----RGKCGEKK 34
80 ------------VYPTAPKRQQPSRTGHDDD-----------GGFVKKK-----RGKCGEKK 34
160 ------------VYPTAPKRQQPSRTGHDDD-----------GGFVQKK-----RGKCGEKK 34
68 ------------VYPTAPKRQRPSRTGHDDD-----------GGFVEKK-----REKCGEKK 34
249 ------------VYPTAPKRQSSRTGHDDD-----------GGFVEKK-----REKCGEKK 34
231 ------------VYPTAPKRQRPSRMAHDDD-----------GGFVENK-----SGKCGEKK 34
234 ------------VYPTAPKRQRPSRTGHDDD-----------GGFVEKK-----RGKCGEKK 34
42 ------------VYPAAPKRQRPSRTSHDDD-----------GSFVKKK-----RVMWG--K 32
236 ------------VYLAAPKRQRPSRTSHDDN-----------GGFVKKK-----RGKCGEEK 34
16 ------------VYPTAPKRQQPSRNSHDDD-----------GGFV-EK-----GEMWG--K 31
134 -------------YPTAPKRQRPSRKSHDDD-----------GGFVEKK-----RGKYGEKK 33
90 ------------VYPTAPKRQRPSRTGHDDD-----------GGFVEKK-----MGKFGEKK 34
28 ------------VYPTAPKRQRPSRMGHDDD-----------GGFVEKK-----RGKCGEKK 34
200 ------------VYPTAPKRQRPSRMGHDDD-----------GGFVEKK-----RGKCGEKK 34
23 ------------VYPTAPKRQRPSRMGHDDY-----------GGFVEKK-----RGKCGEKK 34
37 ------------VYPTAPKRQRPSRMGHDDD-----------GGFVEKK-----RGKCGEKQ 34
324 ------------VYPTAPKRQRPSRMGHDDD-----------GGFVEKK-----RGKCGEKK 34
53 ------------VYPTAPKRQRPSRMGHDDD-----------GGFVEKQ-----RGKCREKK 34
191 ------------VYPTAPKRQRPLRMGHGDD-----------GGFVEKK-----RGKCREKK 34
117 ------------VYPTAPKRQRPLRMGHDDD-----------GGFVEKK-----MGKCGEKK 34
230 ------------VYPTAPKRQRPLRMGHDDD-----------GGFVEKK-----RGKCGEKK 34
120 ------------VYPTAPKRQRPWRMGHDDD-----------GGFVEKK-----RGKCGEKK 34
121 ------------VYPTAPKRQRPWRMGHDDD-----------GGFVEKK-----RGKCGEKK 34
252 ------------VYPTAPKRQRPSRAGHDDD-----------RGFVEKK-----RGKCGEKK 34
258 ------------VYPTAPKRQRPSRAGHDDD-----------GGFVEKK-----RGKCGEKE 34
314 ------------VYPTAPKRQRPSRRGHDDD-----------GGFVEKK-----RGKCEEKK 34
323 ------------VYPTAPKRQRPSRRGHDDD-----------GGFVKKK-----RGKCGEKK 34
131 ------------VYPTAPKRQRPSRTGHDDD-----------GGFVEKK-----RGKCREKK 34
326 ------------VYPTAPKRQRPSRTGHDDD-----------GGFVEKK-----RGKCREKK 34
151 ------------VYPTAPKRQRPLRTGHDDD-----------GGFVEKK-----RGKCGEKK 34
248 ------------VYPTAPKRQRPSRTGHDDD-----------GGFVEKK-----RGKCGENK 34
75 ------------VYSTAPKRQRPSRTGHDDD-----------GGFVEKK-----RGKCGEKK 34
56 ----------------KP--RRTKTQH---------TRISGTHS-------------TCGEKQ 23
311 -------------PLCP--RLKQSSR---------LSLSSSRD-------------CCGEKQ 25
166 ------------PPEQRP--REMNGCH---------SGPDPRHSQE----------GPCGEKK 30
174 ------------PPEQRP--REMNGCH---------SGPDPRHSQE----------GPCGEKK 30
155 ------------PPEQRP--REMNGCH---------SGPDLRHSQE----------GPCGEKK 30
82 ------------PSEQRP--RETNGCH---------SGPDPRHSQE----------GPCGEKK 30
245 ----------------------------P----GNPRRKLPQGQG---------HHCGEKQ 20
310 LHPLSPSQLAPPQPGHPAWATPSDCHNPR----AYGQDELHQVKM----------VECGEKQ 48
87 ----------------SPSAQRPPRLGGVPNSSLRTGHDDDGGFVEWR-----GGKCGEKI 40
189 ----------------SPSAQRPPRLGGVPNSSLRTGHDADGGFVEWK-----RGKCGEKI 40
132 -----------------PSGRCAQQLI-------EKGHDDNGGLVEWR-----RGKCGEKR 32
317 -------------WPAAPSGRCTQQL---------RTGHDDNGGFVEWK-----GGKGGEKI 35
125 -----------------APTRQPPCLRGVPNSSLRTGHDDDGGFVEQK-----RGKCREKK 39
146 ------------VYPAAP--KRQRPLR---------TGHDDDGGFVEKK-----RGKCGEKK 34
256 ------------VYPTAP--KRQRPLR---------TGHDDDSGFVEKK-----RGKCGEKK 34
210 ------------VYPTAPKRQRPSRTGHDDD-----------GGFVEKK-----RGKCGEKK 34
211 ------------VYPTAPKRQRPSRTGHDDD-----------GGFVEKK-----RGKCGEKK 34
265 ------------VYPTAPKRQRPWRTGHDDD-----------GGFVKKK-----RGKCGEKK 34
93 ------------VYPTAPKRQRPLRMGHDDD-----------GSFVKKK-----RGKCGEKK 34
106 ------------VYPTAPKRQRPLRMGHDDD-----------GGFVKKK-----RGKCGEKK 34
291 ------------VYPTAPKRQRPLRRGHDDD-----------GGSVKKK-----RGKCGEKK 34
12 ------------VYPTAPKRQRPLRTGHDDD-----------GGFVKKK-----RGKCGEKK 34
223 ------------VYPTAPKRQRPTRTGHDDD-----------GGFVKKK-----RGKCGEKK 34
86 ------------VYPTAPKRQRPSRTGHDDD-----------GGFVKKK-----RGKCGEKK 34
88 ------------VYPTAPKRQRPSRTGHDDD-----------GGFVKKK-----RGKCGEKK 34
205 ------------VYPTAPKRQRSSRTGRDND-----------GGFVKKK-----RGKCGEKK 34
126 ------------VYPTAPKRQRPSRTGHDDD-----------GGFVKKK-----RGKCGEKK 34
165 ------------VYPTAPKRQRPSRTGHEDD-----------GGFVKKK-----RGKCGEKK 34

330 -----------VYPTVPKRQRPSRKGHEDD----------GCFVKKK-----RGKFGEKK 34
247 -----------VYPTSPKRQRPSRTGHDDD----------GGFVKKK-----RGKCGEKK 34
4 -----------VYPTAPKRQQPSRTGHDDD----------GGFVKKK-----RGKCGEKK 34
10 -----------VYLTAPKRQRPSRTGHDDD----------GGFVKKK-----RGKCGEKK 34
81 -----------VYPTASKRQPPSGTDHDDD----------GGFVKKK-----RGKCGEKK 34
115 -----------VYPTASKRQPPSGTDHDDD----------GSFVKKK-----RGKCGEKE 34
175 -----------VYPTAVKRQRPSRTGHDDD----------GGFVKKK-----RGKCGEKK 34
275 -----------VYPTARKRQRPSRTGHDDD----------GGFVKKK-----RGKCGEKK 34
270 -----------VYPIALKRQRPSRTGHDDD----------GGFVKKK-----RGKCGEKK 34
chrY_3rd ---------LQVYPAAPERQQPARTGHDDYGS----------FVKKK-----RDICREKK 36
272 ---------LQVYPAAPERQRLARTDHDDDGG----------FVKKK-----RGICREKR 36
308 ---------LQVYPAAPERQQPAKTGHNDYGG----------FVKKK-----RGICTAKK 36
40 ---------LQVYPTAPKRQQPARTGHNDDGS----------FVKKK-----RGICREKK 36
170 -----------VFTTAEQGRTPAPGTQRDFAKG-----MDLAGPRGC-----L--CREKK 37
266 -------------PAWPTWRNPVSTKNTKLAR----------HG-AAC-----LQSCREKK 32
5 ---------LQVYPAAPKRERPVRTGHDDDGG----------FLKKK-----RGICREKK 36
64 ---------LQVYTTAPERQRPARTGHDDDGG----------FVKKK-----RGKCREKK 36
221 -----------VYPAASETQRPARTGHDDDGG----------FVKKK-----RGICREKK 34
43 ---------LQVYPAAPERQRPGRRGHDDGGG----------FVKTK-----RGICRGKK 36
50 ---------LQVYPAAPERQRPGRRGHDDGGG----------FVKTK-----RGICRGKK 36
60 ---------LQVYPAAPERQRPARRGHDDGGG----------FVKTK-----MGICREKK 36
192 ---------LQVYPAAQERHRPARRGHDDGGG----------FVKTK-----RGIYREKK 36
57 -----------VYPAAPERQRPARRGHDDGGG----------FVKTK-----RGICRVKK 34
187 -----------------DSDRPERRGHDDGGG----------FVKTK-----RGICREKK 28
293 -----------VYPAAPERQRPARRGHDDGGG----------FVKMK-----RGICREKK 34
299 ---------LQVYPAAPERQRPARRGHDDGGG----------FVKTK-----RGICREKK 36
292 -----------VYPAAPERQRPARRGHDDGGG----------FVKTK-----RGICREKK 34
207 -----------VYPAAPERQQPARRGHDDGGG----------FVKKK-----RGICREKK 34
178 -----------VYPAAPERQRPARRGHNDGGG----------FVKKK-----RGICREKK 34
152 -----------VYAAALERQRPARNGHDDDGG----------FVKKK-----RGIYREKK 34
195 -----------VYPAATEKQRPARTGHDDDGG----------VVKKK-----RGKCREKK 34
138 ---------LQVYPAAPKRQRPLRMGDDDDGG----------FVKKK-----RGKCGEKK 36
204 -----------VYPAAPERQRPARMGHDDDGG----------FVKKK-----RGKCREKK 34
22 -----------VYPAAPKRQRPVRMGHNDDVS----------FVKKK-----RGICREKK 34
11 -----------VYPTALKRQRPKRMGHDDYGS----------SVKKK-----RGICRGKK 34

PCAP2_3rd ERSDCYCVCVERSRHRRLHFVLY------------------------------------- 57
129 ERSDCYCVCVERSRHGRLHFVLY------------------------------------- 57
55 ERSDCYCVCVERSRHRRLHFVLY------------------------------------- 57
9 ERSDCYCVCVERSRHGRLHFVM-------------------------------------- 56
26 ERSDCHCVCVERSRHGRLHFVMY------------------------------------- 57
54 ERSDCHCVCVERSRHRRLHFVMY------------------------------------- 57
98 ERSDCHCVCVERSRHRRLHFVMY------------------------------------- 57
186 ERSDCYCVCVERSRHRRLHFVMY------------------------------------- 57
224 ERSDCCCVCVERSRHRRLHFVMY------------------------------------- 55
25 ERSDCYCVCVERSRHRRLHFVMY------------------------------------- 57
219 ERSNCYCVCVERSRHRRLHFVM-------------------------------------- 56
163 ERSDCYCVCVERSRHRRLHLVMY------------------------------------- 57
164 ERSDCYCVCVERSRHRRLHFLMY------------------------------------- 57
14 ERSDCYCVCVERSRHRRLHFVLY------------------------------------- 57
329 ERSDCYCVCVERSRHRRLHFVLY------------------------------------- 57
3 ERSDCYCVCVERSRHRRLHFVLY------------------------------------- 57
123 ERSDCYCVCVERSRHSRLHFVLY------------------------------------- 56
327 ERSDCYCVCVERSRHRRLHFVLY------------------------------------- 57
177 ERTDCYCVCVERSRHRRLHFVLY------------------------------------- 55
99 ERSDCYCVCVERSRHRRLHFVLY------------------------------------- 57
148 ERSDCYCVCVERSRHRRLHFVLY------------------------------------- 57
79 ERSDCYCVCVERSRHRRLHFVLY------------------------------------- 57
244 ERSDCYYVCVERSRHRRLHFVLY------------------------------------- 57
228 ERSDCYCVYVERSRHRRLHFVLY------------------------------------- 57
320 ERSDCYCVCVERSRHRRLHFVLY------------------------------------- 57
52 ERSDCYCVCVERSRHRRLRFVLY------------------------------------- 57
240 ERSDCYCVCVERRRHRRLHFVLYQEMFFCLGMLLIYNLTPNPLLSETCAV---------- 85
144 ERSDCCCVCVERSRHRRLHFVLY------------------------------------- 57
264 ERSDCCCVCVERSRHRRLHFVLY------------------------------------- 57
259 ERSDCSCVCVERSRHRRLHFVLY------------------------------------- 57
328 ETSDCYCVCVERSRHRRLHFVLY------------------------------------- 57
34 ERSDCYCVCVERSRHRRLHFVLY------------------------------------- 57

321 ERSDCYCVCVERSRHRRLHFVLY------------------------------------ 57
27 ERSDCYCVCVERSRHRRLHF--------------------------------------- 54
47 ERSDCYCVCVERSRHRRLHFVLY------------------------------------ 57
36 ERSDCYCVCVERSRHRRLHFVLY------------------------------------ 57
108 ERSDCSCVCVERSRHRRLHFVLY------------------------------------ 57
15 ERSDCYCVCVERSRHRRLHFVLY------------------------------------ 57
102 ERSDCYCVCVERSRYRRLHFVLYLEKFFCLGMLLIYNLTPNPVLSETCAV---------- 85
322 ERSDCYCVCVERSRHRRLHFVL------------------------------------- 56
31 ERSDCYCVCVERSRHRRLHFVLY------------------------------------ 57
PCAP3 E----IRLSLCLCRKGRHKKLHFV--LY------------------------------- 56
172 E----IRLSLCLCRKGRHKKLHFD--LY------------------------------- 56
302 E----IRLSLCLCRKGRHKRLHFEKDLYSNNCFAEMLFICSFAPATLPQSLCPNLEFTKI 92
303 E----IRLSLCLCRKGRHKRLHFEKDLYSNNCFAEMLFICSFAPATLPQSLCPNLEFTKT 92
296 Q----IRLSLCLCRKGRHKRLHFEKDLYSNNCFAEMLFICSFAPATLPQSLCPNLEFTKT 92
CHR8_3rd E----IRLSLCLCRKGRHKRLHFEKDLYSNYCFAEMLFICSFAPATLPQSLCPNLEFTKT 92
208 E----IRLSLCLCRKGRHKRLHF------------------------------------ 53
212 ERSDSYRLLLCLHRKGRHKRLH------------------------------------- 56
20 ---REIRLLLCLCRK-------------------------------------------- 51
32 ---REIRLLLCLC---------------------------------------------- 45
140 ---RDIRLLLCLC---------------------------------------------- 37
156 ---REIRLLLCLC---------------------------------------------- 43
282 ---REIRLLLCLCRK-------------------------------------------- 46
7 GK-REIRLLLCLC---------------------------------------------- 42
17 ---REIRLLLCLCRK-------------------------------------------- 45
201 ---KEIRLLLCLCRK-------------------------------------------- 45
254 E----IRLLLCLC---------------------------------------------- 43
312 ------------------------------------------------------------
239 K----RDQIVTVSMQKRK----------------------------------------- 47
319 K----REQIVTVSV--------------------------------------------- 43
242 E----IR---------------------------------------------------- 41
333 K----RNQIVTVSV--------------------------------------------- 43
213 Q---------------------------------------------------------- 34
190 K---------------------------------------------------------- 34
84 DQ--------------------------------------------------------- 36
149 DQMLLCLCRK------------------------------------------------- 44
135 DQ--------------------------------------------------------- 35
226 DQ--------------------------------------------------------- 36
51 DQ--------------------------------------------------------- 36
71 DQIVTVSVERSRHRRLHFVLY-------------------------------------- 55
176 DQ--------------------------------------------------------- 36
261 DQ--------------------------------------------------------- 36
94 DQ--------------------------------------------------------- 36
233 DQ--------------------------------------------------------- 36
69 ERSDCYCVCVERSRHRRFQKKK------------------------------------- 58
183 EQ--------------------------------------------------------- 36
268 E----RSDCYCV---------------------------------------------- 42
19 ERSDCYCVCVERTRHRRFHFVLY------------------------------------ 57
246 ERSDCYCVCIERSRHRRHHFVLY------------------------------------ 57
335 ERSDFYCVCAERSRHRRHHFVLY------------------------------------ 57
116 ERSDCYCVCVERSRHRRHHFVLY------------------------------------ 57
63 ERSDCYCVCVERSRHRRHHFVLY------------------------------------ 57
73 ERSDCYHVCVERSRHRRHHFVLY------------------------------------ 57
74 ERSDCYCVCLERSRHRRLHFVL------------------------------------- 56
109 ERTDCYCVCVERSRHRGLHFVLY------------------------------------ 57
111 ERTDCYCVCVERSRHRGLHFVLY------------------------------------ 57
83 ERTDCYCVCVERSRHRRLHFVLY------------------------------------ 57
235 ERSDCYCVCVERSRHRRHHFVLY------------------------------------ 51
332 ERSDCYCVCVERSRHRRLHFVLY------------------------------------ 57
251 ERSDCYCVCVERSRHRRLHFVLY------------------------------------ 57
162 ERSDCYCVCVERSRHRRLHFVLY------------------------------------ 57
315 ERSDCYCVCVERSRHRRLHFVLY------------------------------------ 57
1 ERSDCYCVCVERSRHRRLHFVLY------------------------------------ 57
203 ERSDCYCVCVERSRHRRLHFVLY------------------------------------ 57
13 ERSDCYCVCVERSRHRRLHFVLY------------------------------------ 57
96 ERSDCYCVCVERSRHRRHPFVLY------------------------------------ 57
217 ERSDCYCVCVERSRHRRLPFVLY------------------------------------ 57
198 GGSDFYSVCVERSRHRGPHFVLY------------------------------------ 56
199 ERSDCYYVCVERSRHRRLHFVLY------------------------------------ 57

269 ERSDCYCVCVERSRHRRLHFVLY------------------------------------- 57
35 ERSDCYCVCVERSRHRRLHFALY------------------------------------- 57
24 ERSDCYFVCVERSRHRRLHFVLY------------------------------------- 57
113 ERSDCYCVCVERSRHRRLHFVLYLEKFFCLGMLLIYNFTPNHVLSETC------------ 83
130 ERSDYYCVCVERSRHRRLHFVLY------------------------------------- 57
318 ERSDCYCVCVERSRHRRLHFVLY------------------------------------- 57
29 ERSDCYCVCVERSRHRRLHFVLY------------------------------------- 57
232 ERSDCYCVCVERSRHRRLHFVLY------------------------------------- 57
80 ERSDCYCVCVERSRHRRLHFVLY------------------------------------- 57
160 ERSDCYCVCVERSRHRRLHFVLH------------------------------------- 57
68 ERSNCYCVCVERSRHRRLHFVLY------------------------------------- 57
249 ERSDCYRVCVERSRHRRLHFVLY------------------------------------- 57
231 ERSDCYRVCVERSRHRRLHFVLY------------------------------------- 57
234 ERSDCYRVCVERSRHRRLHFVLY------------------------------------- 57
42 ERSDCYCVYVERSRHKRLHFVLY------------------------------------- 55
236 ERSDCYCVYVERSRHKRLHFVLY------------------------------------- 57
16 ERSDCYCVCVERSRHRRLHFVLY------------------------------------- 54
134 ERSDCYCVCVERSRHRRLHFVLY------------------------------------- 56
90 ERSDCYCVCVERSRHRRLHFVLY------------------------------------- 57
28 ERSDCYCVCVERSRHRRLHFVLY------------------------------------- 57
200 ERSDCYCVCVES-RHRRLHFVLY------------------------------------- 56
23 ERSDCYCVCVERSRHRRLHFVLY------------------------------------- 57
37 ERSDCYCVCIERSRHRRLHFVLY------------------------------------- 57
324 ERTDCYCVYIERSRHRRLHFVLY------------------------------------- 57
53 ERSDCYCVCVERSWHRRLHFVLY------------------------------------- 57
191 ERSDCYCVCVERSRHRRLHFVLY------------------------------------- 57
117 ERSDCYCVCVERSRHRRLHFVLY------------------------------------- 57
230 ERSDCYCVCVERSRHRRLHFVLY------------------------------------- 57
120 ERSDCHCVCVERSRHRRLHFVLY------------------------------------- 57
121 ERSDCYCVCVERSRHRRLHFVLY------------------------------------- 57
252 ERSDCYCVCVERSRHRRLHFVLY------------------------------------- 57
258 ERSDLYCVCVERSRHRRLHFVLY------------------------------------- 57
314 ERSDCYCVCVERSRHRRLHFILY------------------------------------- 57
323 ERSDCYCVCVERSRHRRLHFVLY------------------------------------- 57
131 ERSDCYCVCVERSRHRRLHFVLY------------------------------------- 57
326 ERSDCYCVCVERRRHRRLHFVLY------------------------------------- 57
151 ERSDCYCVCVERSRHKRLHFVLY------------------------------------- 57
248 ERSDCYCVCVERSRHKRLHFVLY------------------------------------- 57
75 ERSDCYCVCVERSRHSRLHFVLY------------------------------------- 57
56 ERSDCYCVCVERSRHRRLHFVLY------------------------------------- 46
311 ERSDCYCVCIERSRHRRLHFVLY------------------------------------- 48
166 EISDCYCVYVERSRRKRLHFVV-------------------------------------- 52
174 EISDCYCVYVERSRRKRLHFVL-------------------------------------- 52
155 EISDCYCVYVERSRRKRLHFVLY------------------------------------- 53
82 EISDCYCVYVERSRHKRLHFVV-------------------------------------- 52
245 EGSDCYCVCVERSRHRRLHFVLH------------------------------------- 43
310 ERSECHCICVERSRHGRLHFVMY------------------------------------- 71
87 DKSDCCCVCVEGSRRRRLHFVLY------------------------------------- 63
189 ERSDCYCVCIERSRHRRLHFVLY------------------------------------- 63
132 ERSDCCCVCVEGGRRGRLHFVLY------------------------------------- 55
317 EKSDGCRVCVERGRHGR-FFILF------------------------------------- 57
125 ERSDCYCVCVERSRHRRLHFVLY------------------------------------- 62
146 QKSDCYCVCVERDRHRRLHFVLY------------------------------------- 57
256 ERSDCYCVCVERSKHRRLHFVLY------------------------------------- 57
210 KRSDCYCVCVERSRCRRLRFVLY------------------------------------- 57
211 KRSDCYCVCVERSRCRRLHFVLY------------------------------------- 57
265 KRSDCYCVCVERSRHGRLRFVLY------------------------------------- 57
93 ERSDCYCVCVERSRHRRLHFVLY------------------------------------- 57
106 ERSDCYCVCVERSRHRRLHFVLY------------------------------------- 57
291 ERSDCYCVCVERSRHKRLHFVLY------------------------------------- 57
12 ERSDCYCVCVERSRHRRLHFVLY------------------------------------- 57
223 ERSGCYCACVERSRHRRLHFVLY------------------------------------- 57
86 EKSDCYCVCVERSRHRRLHFVLY------------------------------------- 57
88 ERSDCYCVCVERSRHRRLHFVLY------------------------------------- 57
205 ERSDCYCVCVGRSRHRRLHFVLY------------------------------------- 57
126 ERSDCYCVCVERSIHRRLHFVLY------------------------------------- 57
165 ERSDCYCVCVERNRHRRLHFVLY------------------------------------- 57
330 ERSDCYCVCVERSRHRRLHFVLY------------------------------------- 57

247                ERSDCYCVCVERSRHRRLHFVLY------------------------------------- 57
4                  ERSDCYCVCVERSRHRILHFVLY------------------------------------- 57
10                 ERSDCYCVCVERSRHRILHFVLY------------------------------------- 57
81                 ERSDCYCVCVERSRHRRLHFLLY------------------------------------- 57
115                ERSDCYCVCVERSRHRRLHFLLY------------------------------------- 57
175                ERSDCYCVCVERSRHRRLHFL--------------------------------------- 55
275                ERSDCYCVCVERSRHRRLHFVL-------------------------------------- 56
270                ERSDCYCVYVERSRHRRLHFVLY------------------------------------- 57
chrY_3rd           ERSDCYCVYVEKKDIRDSILKKTCTLNNCFAEMLLICSFAPATLTQPGAHKNMCCMESRL 96
272                ERSDCYCVYVEREDIRDSILKKTCTLNNCFAQMLLICSFAPATLTQPGAHKNMCCMKSRF 96
308                ERSDCYCVYVEREDIRNSIL--TCTLNNCFAEMLLICNFAPATLPQ-------------- 80
40                 EISDCYCIFVEKEDIRNSIL--TCTVNNCFA----------------------------- 65
170                ERSHCYCVYVEKEDI-NSILS--CTKKNYFA----------------------------- 65
266                ERSDCYCVCVEREDIRNSILT--CTLNNWLAEMLLICDFAPNLSSQ-------------- 76
5                  ERSDGYCVYVEKEDIRNFILI--CTLNNCFA----------------------------- 65
64                 ERSDCHCAYVEREDIRDSILKKTCTLNNCFAEMLLICSF--------------------- 75
221                VRSDCYCIYVER------------------------------------------------ 46
43                 ERSDCYCVYIEREDIRDSILKKICTLSNCFAEMLLICSFAPATLPQP------------- 83
50                 ERSDCYCVYIEREDIRDSILKKNCTLNNCFAEMFLICSFAPATFPQP------------- 83
60                 ERSDCYCVYIEREDIRDSILKKTCTLNNCFAEMLLICSFAPATLPQP------------- 83
192                ERSDCYCVYTEREDIRDSILKKTCTLNNCFAEMLLICSFAPATLP--------------- 81
57                 ERSDCYCVYIER------------------------------------------------ 46
187                ERSDCYCVYIEREDIRDSILKKTCTLNSCFDRDSCLSAFMCLLLPQ-------------- 74
293                ERSDCYCVYIEREAIR-------------------------------------------- 50
299                ERSDCYCVYIEREAIRDSILKKTCTLNNCLLRCCLSVALPQPLCPN-------------- 82
292                ERSDSYCVYIER------------------------------------------------ 46
207                ERSDSYCVYIER------------------------------------------------ 46
178                ERSDCYCVYIER------------------------------------------------ 46
152                ERSDCYCVYVER------------------------------------------------ 46
195                EGSDCHCVYAER------------------------------------------------ 46
138                ERSDCYCVYVEKEDIRNSILICIKKNCSALRC---------------------------- 68
204                ERSDCHSVYVEK------------------------------------------------ 46
22                 ERSDCYCVYVEK------------------------------------------------ 46
11                 ERSDCYCVYVEK------------------------------------------------ 46

302                CVV-------- 95
303                CVV-------- 95
296                CVV-------- 95
CHR8_3rd           CVV-------- 95
chrY_3rd           KGSRAVQDVPC 107
272                KGSRAVQDVPC 107

The foregoing description of preferred embodiments of the invention has been presented by way of illustration and example for purposes of clarity and understanding. It is not intended to be exhaustive or to limit the invention to the precise forms disclosed. It will be readily apparent to those of ordinary skill in the art in light of the teachings of this invention that many changes and modifications may be made thereto without departing from the spirit of the invention. It is intended that the scope of the invention be defined by the appended claims and their equivalents.

**TABLE 1 - DESCRIPTION OF SPLICE VARIANTS**

| Consensus sequences of splice forms A to J (Figure 17) are given as SEQ IDs 18-27. These represent consensus sequences of all cDNA clones characterized and in general represent the sequence variability observed in the genomic alignments of figures 19-22. | | |
|---|---|---|
| **SPLICE FORM (SEQ ID)** | **DESCRIPTION** | **PROTEIN SEQ ID** |
| A 18 | Splice variant that joins exons 1 (Splice Site I) and 3 (Splice Site XIII), without exon 2. Probably does not encode any protein, as its only methionines are from the ORFs in exon 3. This splice form was detected in prostate cancer cell lines LNCaP and PC3. By agarose gel analysis, a product corresponding to this splice form was detected in all cell line and tumor samples analyzed. | - |
| B 19 | PCAP2 Splice variant that joins exon 1 (Splice Site I) to exon 2 (Splice Site IV to VI) and to exon 3 (Splice Site XIII). Derived from a type I PCAV. Encodes a 74aa protein from the env start codon and ends in the +2 frame termination codon in exon 3. Detected in all prostate cancer cell lines tested and in tissue samples from prostate tumors. | 28 |
| C 20 | PCAP2 Splice variant that joins exon 1 (Splice Site I) to exon 2 (Splice Site V to VI) and to exon 3 (Splice Site XIII). Derived from a type I PCAV. Encodes a 74aa protein identical to that encoded by Splice Form B, from the env start codon to the +2 frame termination codon in exon 3. Detected in all prostate cancer cell lines tested and in tissue samples from prostate cancer tumors. | 29 |
| D 21 | PCAP2 Splice variant that joins exon 1 (Splice Site I) to exon 1.5 (Splice Site II and III), to exon 2 (Splice Site V to VI) and to exon 3 (Splice Site XIII). Derived from a type I PCAV. Exon 1.5 contains a potential initiation methionine and so this splice form potentially encodes an additional polypeptide different from the one initiated by the env methionine in exon 2. It also encode for the 74aa protein identical to that encoded by Splice Form B, from the env start codon to the +2 frame termination codon in exon 3. Detected in prostate cancer cell line MDA Pca-2b. | 30 |
| | | 31 |
| E 22 | PCAP1 Splice variant that joins exon 1 (Splice Site I) to exon 2 (Splice Site V to VIII) and to exon 3 (Splice Site XIII). Derived from a type II PCAV. Encodes a 76aa protein from the env start codon to the cORF termination codon in exon 3. Detected in most prostate cancer cell lines tested and in tissue samples from prostate cancer tumors. | 32 |
| F 23 | Splice variant that joins exon 1 (Splice Site I) to exon 2 (Splice Site V to IX) and to exon 3 (Splice Site XIII). Derived from a type II PCAV. Encodes a 95aa protein from the env start codon to the cORF termination codon in exon 3. Detected in most prostate cancer cell lines tested and in the tissue samples from prostate cancer tumors. | 33 |
| G 24 | Splice variant that joins exon 1 (Splice Site I) to exon 2 (Splice Site V to XI) and to exon 3 (Splice Site XIII). Derived from a type I PCAV. If the env start codon is used, the ORF is very short and ends with a TAA termination codon at position 6922 in the type I PCAV (Figure 21). However, a second potential initiation methionine is found at position 6918 in the type I PCAV (Figure 21), and this ORF encodes a 127aa protein that ends at the +2 frame termination codon in exon 3. Detected in most prostate cancer cell lines tested and in the tissue samples from prostate cancer tumors. | 34 |
| H 25 | Splice variant that joins exon 1 (Splice Site I) to exon 2 (Splice Site V to X) and to exon 3 (Splice Site XIII). Derived from a type I PCAV. As for splice form G, if the env start codon is used then the ORF is very short. Using the second initiation methionine at position 6918 in the type I PCAV (Figure 21), however, this ORF encodes a 105aa protein that ends at the +2 frame termination codon in exon 3. Detected in most prostate cancer cell lines tested and in the tissue samples from prostate cancer tumors. | 35 |
| I 26 | PCAP3 Splice variant that joins exon 1 (Splice Site I) to exon 2 (Splice Site V to VII) and to exon 3 (Splice Site XIII). Derived from a type II PCAV. Encodes a 79aa protein from the env start codon and ends at the +2 frame termination codon in exon 3. Detected in prostate cancer cell line MDA Pca-2b. | 36 |
| J 27 | This cDNA clone was identified using a PCR forward primer in exon 2 and a reverse primer in exon 3. The mRNA from which this splice form is derived probably does not have intron 1 spliced out. Splicing of intron 2 was between Splice Sites XII & XIII. | 37 |
| K 38 | Created by joining exon 2 at 'Potential Splice Site A' (figure 21) with exon 3 at Splice Site XIII. Encodes PCAP4 | 39 |
| L 40 | Created by joining exon 2 at Splice Site VIII (figure 21) with exon 3 at 'Potential Splice Site B'. Encodes PCAP4a. | 41 |
| M 42 | Created by joining exon 2 at Splice Site IX (figure 21) with exon 3 at 'Potential Splice Site B'. Encodes PCAP5. | 43 |

**TABLE 2 - SEQUENCE LISTING**

| **SEQ ID** | **DESCRIPTION** |
|---|---|
| 1 | U5 region of herv-k(hml-2.hom) {GenBank AF074086} |
| 2 | U3 region of herv-k(hml-2.hom) |
| 3 | R region of herv-k(hml-2.hom) |
| 4 | RU5 region of herv-k(hml-2.hom) |
| 5 | U3R region of herv-k(hml-2.hom) |
| 6 | Non-coding region between U5 and first 5' splice site of herv-k(hml-2.hom) |
| 7 | PCAP4 |
| 8 | PCAP4a |
| 9 | PCAP5 |
| 10 | PCAP1 |
| 11 | PCAP2 |
| 12 | PCAP3 |
| 13 | cORF |
| 14 | TAR |
| 15 | Figure 15, forward primer |
| 16 | Figure 15, reverse primer 1 |
| 17 | Figure 15, reverse primer 2 |
| 18 | Splice form A |
| 19 | Splice form B |
| 20 | Splice form C |
| 21 | Splice form D |
| 22 | Splice form E |
| 23 | Splice form F |
| 24 | Splice form G |
| 25 | Splice form H |
| 26 | Splice form I |
| 27 | Splice form J |
| 28 | Splice form A protein |
| 29 | Splice form B protein |
| 30 | Splice form C protein |
| 31 | Splice form D protein |
| 32 | Splice form E protein |
| 33 | Splice form F protein |
| 34 | Splice form G protein |
| 35 | Splice form H protein |
| 36 | Splice form I protein |
| 37 | Splice form J protein |
| 38 | Splice form K |
| 39 | Splice form K protein' |
| 40 | Splice form L |
| 41 | Splice form L protein |
| 42 | Splice form M |
| 43 | Splice form M protein |
| 44 | LTR of herv-k(hml-2.hom) |
| 45 | HML-2 LTR |
| 46 | HML-2 LTR |
| 47 | HML-2 LTR |
| 48 | HML-2 LTR |
| 49 | Putative TAR of herv-k(hml-2.hom) |
| 50 | Remainder of herv-k(hml-2.hom) LTR |
| 51 | Region downstream of 'potential splice site B' in Figure 22 |
| 52-59 | Oligos and primers used in mapping 5' end of mRNA |
| 60 | V5 tag |
| 61-67 | Motifs common to exon 3 of various PCAPs |
| 68 | Exon 3 region of PCAP3 |
| 69-76 | Optional disclaimed amino acid sequences |
| 77 | MDALTR |
| 78-477 | Third frame sequences from human genome |
| 478 | SEQ ID 49, excluding its 77 5' nucleotides |

### REFERENCES

{1} International patent application WO02/46477 (PCT/US01/47824. filed December 7,2001).
{2} US patent application 10/016,604 (filed December 7,2001).
{3} Magin-Lachmann (2001) J. Virol. 75(21):10359-71.
{4} Löwer et al. (1995) J. Virol. 69:141-149.
{5} Magin et al. (1999) J. Virol. 73:9496-9507.
{6} GenBank entry Y17832.
{7} Mayer et al. (1999) Nat. Genet. 21 (3), 257-258 (1999)
{8} Farrell (1998) RNA Methodologies (Academic Press; ISBN 0-12-249695-7).
{9} Robbins et al. (1997) Clin Lab Sci 10(5):265-71.
{10} Ylikoski et al. (1999) Clin Chem 45(9): 1397-407
{11} Ylikoski et al. (2001) Biotechniques 30:832-840
{12} Shirahata & Pegg (1986) J. Biol. Chem. 261(29):13833-7.
{13} Suzuki et al. (2001) Br J Cancer 85:1731-1737.
{14} Revillion et al. (2000) Eur J Cancer 36:1038-1042.
{15} Miyakis et al. (1998) Biochem Biophys Res Commun 251:609-612.
{16} Berois et al. (1997) Anticancer Res 17(4A):2639-2646.
{17} Sambrook et al. (1989) Molecular Cloning: A Laboratory Manual (New York, Cold Spring Harbor Laboratory)
{18} Short protocols in molecular biology (4th edition, 1999) Ausubel et al. eds. ISBN 0-471-32938-X.
{19} US patent 5,707,829
{20} Current Protocols in Molecular Biology (F.M. Ausubel et al., eds., 1987) Supplement 30.
{21} EP-B-0509612
{22} EP-B-0505012
{23} Hashido et al. (1992) Biochem. Biophys. Res. Comm. 187:1241-1248.
{24} Geysen et al. (1984) PNAS USA 81:3998-4002.
{25} Carter (1994) Methods Mol Biol 36:207-23.
{26} Jameson, BA et al., 1988, CABIOS 4(1):181-186.
{27} Raddrizzani & Hammer (2000) Brief Bioinform 1(2):179-89.
{28} De Lalla et al. (1999) J. Immunol. 163:1725-29.
{29} Brusic et al. (1998) Bioinformatics 14(2):121-30
{30} Meister et al. (1995) Vaccine 13(6):581-91.
{31} Roberts et al. (1996) AIDS Res Hum Retroviruses 12(7):593-610.
{32} Maksyutov & Zagrebelnaya (1993) Comput Appl Biosci 9(3):291-7.
{33} Feller & de la Cruz (1991) Nature 349(6311):720-1.
{34} Hopp (1993) Peptide Research 6:183-190.
{35} Welling et al. (1985) FEBSLett. 188:215-218.
{36} Davenport et al. (1995) Immunogenetics 42:392-297.
{37} Morris et al. (2001) Vaccine 20(1-2):12-15.
{38} Goldstein et al. (2001) Vaccine 19(13-14):1738-46
{39} Tosi et al. (2000) Eur. J Immunol. 30(4):1120-6
{40} Tahtinen et al. (1997) Biomed Pharmacother 51(10):480-7.
{41} Nath et al. (1996) J. Virol. 70:1475-1480*.*
{42} Pardi et al. (1995) J Infect Dis 172:554-557.
{43} Lairmore et al. (1995) Biomed Pept Proteins Nucleic Acids 1:117-122.
{44} Sakakibara et al. (1998) J Vet Med Sci 60:599-605.
{45} Smith and Waterman, Adv. Appl. Math. (1981) 2: 482-489.
{46} Go et al, Int. J. Peptide Protein Res. (1980) 15:211
{47} Querol et al., Prot. Eng. (1996) 9:265
{48} Olsen and Thomsen, J. Gen. Microbiol. (1991) 137:579
{49} Clarke et al., Biochemistry (1993) 32:4322
{50} Wakarchuk et al., Protein Eng. (1994) 7:1379
{51} Toma et al., Biochemistry (1991) 30:97
{52} Haezerbrouck et al., Protein Eng. (1993) 6:643
{53} Masul et al., Appl. Env. Microbiol. (1994) 60:3579
{54} US patent 4,959,314
{55} Breedveld (2000) Lancet 355(9205):735-740.
{56} Gorman & Clark (1990) Semin. Immunol. 2:457-466
{57} Jones et al., Nature 321:522-525 (1986)
{58} Morrison et al., Proc. Natl. Acad. Sci, US.A., 81:6851-6855 (1984)
{59} Morrison and Oi, Adv. Immunol., 44:65-92 (1988)
{60} Verhoeyer et al., Science 239:1534-1536 (1988)
{61} Padlan, Molec. Immun. 28:489-498 (1991)
{62} Padlan, Molec. Immunol. 31(3):169-217 (1994).
{63} Kettleborough, C.A. et al., Protein Eng. 4(7):773-83 (1991).
{64} Chothia et al., J. Mol. Biol. 196:901-917 (1987)
{65} Kabat *et al.,* U.S. Dept. of Health and Human Services NIH Publication No. 91-3242 (1991)
{66} US patent 5,530,101.
{67} US patent 5,585,089.
{68} WO 98/24893
{69} WO 91/10741
{70} WO 96/30498
{71} WO 94/02602
{72} US Patent 5,939,598.
{73} WO 96/33735
{74} Johnston et al. (2001) Ann Neurol 50(4):434-42.
{75} Medstrand et al. (1998) J Virol 72(12):9782-7.
{76} Lisziewicz et al. (1993) PNAS USA 90:8000-8004
{77} Chang et al. (1994) Gene Ther 1(3):208-16.
{78} Fraisier et al. (1998) Gene Ther 5(7):946-54.
{79} Fraisier et al. (1998) Gene Ther 5(12):1665-1676.
{80} Wyszko et al. (2001) Int J Biol Macromol 28(5):373-80.
{81} Rao et al. (2000) J. Biomolec. Struc. Dynam. 11(1).
{82} Tamilarasu et al. (2000) Bioorg Med Chem Lett 10(9):971-4.
{83} Hamy et al. (1998) Biochemistry 37(15):5086-95
{84} Yamamoto et al. (2000) Genes Cells 5:371-388
{85} Coburn & Cullen (2002) J. Virol. 76:9225-9231.
{86} Cantor et al. (1993) Proc Natl Acad Sci USA 90:10932-10936.
{87} Miyano-Kurosaki et al. (1996) Virus Genes 12:205-217.
{88} Cantor & Palmer (1992) Antisense Res Dev 2:147-152.
{89} Radrizzani et al. (1999) Medicina (B Aires) 59(6):753-8
{90} Bianchini et al. (2001) J Immunol Methods 252(1-2):191-197
{91} Zamore (2001) Nat Struct Biol 8:746-750.
{92} Carthew (2001) Curr Opin Cell Biol 13:244-248.
{93} Billy et al. (2001) PNAS USA 98:14428-14433.
{94} Yang et al. (2001) Mol Cell Biol 21:7807-7816.
{95} Carmichael (2002) Nature 418:379-380.
{96} Xia et al. (2002) Nature Biotech 20:1006-1010.
{97} Mei et al. (1998) Biochemistry 37:14204-12.
{98} An et al. (1998) Bioorg Med Chem Lett 8(17):2345-50
{99} McSharry (1999) Antiviral Res 43(1):1-21.
{100} Kuhelj et al. (2001) J Biol Chem 276(20):16674-82.
{101} Schommer et al. (1996) J Gen Virol 77:375-379.
{102} Magin et al. (2000) Virology 274:11-16.
{103} Boese et al. (2001) FEBS Lett 493(2-3):117-21.
{104} Further details: Rational drug design: novel methodology and practical applications, ACS Symposium Series vol. 719 (Parrill & Reddy eds., 1991).
{105} Available from Tripos Inc (http://www.tripos.com).
{106} Available from Oxford Molecular (http://www.oxmol.co.uk/).
{107} Available from Molecular Simulations Inc (http://www.msi.com/).
{108} Available from Hypercube Inc (http://www.hyper.com/).
{109} Available from Pyramid Learning (http://www.chemsite.org/).
{110} Filikov et al. (2000) J Comput Aided Mol Des 14(6):593-610
{111} Re et al. (2001) New Microbiol 24(2):197-205.
{112} Ensoli & Cafaro (2000) Peptides 21:1839-1847.
{113} Ensoli & Cafaro (2000) J Biol Regul Homeost Agents 14(1):22-6
{114} Boykins et al. (2000) Peptides 21:1839-1847.
{115} Dezzutti et al. (1990) J Med Primatol 19:305-316.
{116} Cafaro et al. (2001) Vaccine 19(20-22):2862-77
{117} Ohashi et al. (2000) J Virol 74:9610-9616.
{118} WO90/14837
{119} Vaccine Design - the subunit and adjuvant approach (1995) ed. Powell & Newman
{120} WO00/07621
{121} GB-2220221
{122} EP-A-0689454
{123} EP-A-0835318
{124} EP-A-0735898
{125} EP-A-0761231
{126} WO99/52549
{127} WO01/21207
{128} WO01/21152
{129} WO00/62800
{130} WO00/23105
{131} WO99/11241
{132} WO98/57659
{133} WO93/13202.
{134} Gennaro (2000) Remington: The Science and Practice of Pharmacy. 20th edition, ISBN: 0683306472.
{135} WO 93/14778
{136} Findeis et al., Trends Biotechnol. (1993) 11:202
{137} Chiou et al., Gene Therapeutics: Methods And Applications Of Direct Gene Transfer (J.A. Wolff, ed.) (1994)
{138} Wu et al., J. Biol. Chem. (1988) 263:621
{139} Wu et al., J. Biol. Chem. (1994) 269:542
{140} Zenke et al., Proc. Natl. Acad. Sci. (USA) (1990) 87:3655
{141} Wu et al., J. Biol. Chem. (1991) 266:338
{142} Jolly, Cancer Gene Therapy (1994) 1:51
{143} Kimura, Human Gene Therapy (1994) 5:845
{144} Connelly, Human Gene Therapy (1995) 1:185
{145} Kaplitt, Nature Genetics (1994) 6:148
{146} WO 90/07936
{147} WO 94/03622
{148} WO 93/25698
{149} WO 93/25234
{150} US patent 5,219,740
{151} WO 93/11230
{152} WO 93/10218
{153} US patent 4,777,127
{154} GB Patent No. 2,200,651
{155} EP-A- 0 345 242
{156} WO 91/02805
{157} WO 94/12649
{158} WO 93/03769
{159} WO 93/19191
{160} WO 94/28938
{161} WO 95/11984
{162} WO 95/00655
{163} Curiel, Hum. Gene Ther. (1992) 3:147
{164} Wu, J. Biol. Chem. (1989) 264:16985
{165} US patent 5,814,482
{166} WO 95/07994
{167} WO 96/17072
{168} WO 95/30763
{169} WO 97142338
{170} WO 90/11092
{171} US patent 5,580,859
{172} US patent 5,422,120
{173} WO 95/13796
{174} WO 94/23697
{175} WO 91/14445
{176} EP 0524968
{177} Philip, Mol. Cell Biol. (1994) 14:2411
{178} Woffendin, Proc. Natl. Acad. Sci. (1994) 91:11581
{179} US patent 5,206,152
{180} WO 92/11033
{181} US patent 5,149,655
{182} WO 92/11033
{183} Larsson, E., et al., Current Topics in Microbiology and Immunology 148:115 (1989)
{184} Mariani-Costantini, et al., J. Virol. 63:4982 (1989) and Shih, et al., Virology 182:495 (1991)
{185} Tönjes et al. (1996) J. AIDS Hum. Retrovir. 13(Suppl 1):S261-S267.
{186} Barbulescu et al., Curr. Biol. 9:861 (1999)
{187} Ono, et al., J. Virol. 58:937 (1986)
{188} Löwer et al., Proc. Natl. Acad. Sci USA 90:4480 (1993)
{189} Ono et al., (1986) J. Virol. 60:589
{190} Boller, et al., Virol. 196:349 (1993)
{191} Yang et al., Proc. Natl. Acad. Sci USA 96:13404 (1999)
{192} Mueller- Lantzsch et al., AIDS Research and Human Retroviruses 9:343-350 (1993)
{193} Herbst et al., Amer. J. Pathol. 149:1727 (1996)
{194} US patent 5,858,723
{195} Löwer et al., Proc. Natl. Acad. Sci USA 93:5177 (1996)
{196} Löwer et al., Virology 192:501 (1993)
{197} Blomberg et al., J. Cancer. res. Clin. Oncol. 121: Supp. 1, 3 (1995)
{198} Genbank accession number AB047240
{199} Andersson et al. (1999) J. Gen. Virol. 80:255-260.
{200} Zsiros et al. (1998) J. Gen. Virol. 79:61-70.
{201} Tönjes et al. (1999) J. Virol. 73:9187-9195.
{202} Yoshida (X) Annual Review of Immunology 19:475-496.
{203} Bello et al. (1997) Carcinogenesis 18:1215-1223.
{204} Armbruester et al. (2002) Clinical Cancer Research 8:1800-1807.
{205} Sedivy (1998) Proc Natl Acad Sci USA 95:9078-9081.
{206} Hahn et al. (2002) Mol Cell Biol. 22(7):2111-2123.
{207} Hahn et al. (1999) Nature 400(6743):464-468.
{208} De Marzo et al. (1998) J Urol. 160:2381-2392.
{209} Sakr & Partin (2001) Urology 57(4 Suppl 1):115-120.

### SEQUENCE LISTING

SEQ ID 1 - U5 region of herv-k(hml-2.hom) [GenBank AF074086]
   CTTTGTCTCTGTGTCTTTTTCTTTTCCAAATCTCTCGTCCCACCTTACGAGAAACACCCACAGGTGTGTAGGGGCAACCCACCCCTACA
SEQ ID 2 - U3 region of herv-k(hml-2.hom) [GenBank AF074086] 
TGTGGGAAAAGCAAGAGAGATCAGATTGTTACTGTGTCTGTGTAGAAAGAAGTAGACATAGGAGACTCCATTTGTTATGTACTAAGA
AAAATTCTTCTGCCTTGAGATTCTGTTAATCTATGACCTTACCCCCAACCCCGTGCTCTCTGAAACATGTGCTGTGTCCACTCAGGGTT
AAATGGATTAAGGGCGGTGCAGGATGTGCTTTGTTAAACAGATGCTTGAAGGCAGCATGCTCCTTAAGAGTCATCACCACTCCCTAATC
TCAAGTACCCAGGGACACAAAAACTGCGGAAGGCCGCAGGGACCTCTGCCTAGGAAAGCCAGGTATTGTCCAACGTTTCTCCCCATGTG
ATAGCCTGAAATATGGCCTCGTGGGAAGGGAAAGACCTGACCGTCCCCCAGCCCGACACCCGTAAAGGGTCTGTGCTGAGGAGGATTAG
TAAAAGAGGAAGGAATGCCTCTTGCAGTTGAGACAAGAGGAAGGCATCTGTCTCCTGCCTGTCCCTGGGCAATGGAATGTCTCGGTATA
AAACCCGATTGTATGCTCCATCTACT
SEQ ID 3 - R region of herv-k(hml-2.hom) [GenBank AF074086] 
GAGATAGGGAAAAACCGCCTTAGGGCTGGAGGTGGGACCTGCGGGCAGCAATACTGCTTTGTAAAGCACTGAGATGTTTATGTGTATGC
ATATCTAAAAGCACAGCACTTAATCCTTTACATTGTCTATGATGCAAAGACCTTTGTTCACATGTTTGTCTGCTGACCCTCTCCCCACA
ATTGTCTTGTGACCCTGACACATCCCCCTCTTCGAGAAACACCCACAGATGATCAGTAAATACTAAGGGAACTCAGAGGCTGGCGGGAT
CCTCCATATGCTGAACGCTGGTTCCCCGGGTCCCCTTCTTTCTTTCTCTATA
SEQ ID 4 - RU5 region of herv-k(hml-2.hom) [GenBank AF074086] 
GAGATAGGGAAAAACCGCCTTAGGGCTGGAGGTGGACCTGCGGGCAGCAATACTGCTTTGTAAAGCACTGAGATGTTTATGTGTATGC
ATATCTAAAAGCACAGCACTTAATCCTTTACATTGTCTATGATGCAAAGACCTTTGTTCACATGTTTGTCTGCTGACCCTCTCCCCACA
ATTGTCTTGTGACCCTGACACATCCCCCTCTTCGAGAAACACCCACAGATGATCAGTAAATACTAAGGGAACTCAGAGGCTGGCGGGAT
CCTCCATATGCTGAACGCTGGTTCCCCGGGTCCCCTTCTTTCTTTCTCTATACTTTGTCTCTGTGTCTTTTTCTTTTCCAAATCTCTCG
TCCCACCTTACGAGAAACACCCACAGGTGTGTAGGGGCAACCCACCCCTACA
SEQ ID 5 - U3R region of herv-k(hml-2.hom) [GenBank AF074086] 
TGTGGGAAAAGCAAGAGAGATCAGATTGTTACTGTGTCTGTGTAGAAAGAAGTAGACATAGGAGACTCCATTTGTTATGTACTAAGA
AAAATTCTTCTGCCTTGAGATTCTGTTAATCTATGACCTTACCCCCAACCCCGTGCTCTCTGAAACATGTGCTGTGTCCACTCAGGGTT
AAATGGATTAAGGGCGGTGCAGGATGTGCTTTGTTAAACAGATGCTTGAAGGCAGCATGCTCCTTAAGAGTCATCACCACTCCCTAATC
TCAAGTACCCAGGGACACAAAAACTGCGGAAGGCCGCAGGGACCTCTGCCTAGGAAAGCCAGGTATTGTCCAACGTTTCTCCCCATGTG
ATAGCCTGAAATATGGCCTCGTGGGAAGGGAAAGACCTGACCGTCCCCCAGCCCGACACCCGTAAAGGGTCTGTGCTGAGGAGGATTAG
TAAAAGAGGAAGGAATGCCTCTTGCAGTTGAGACAAGAGGAAGGCATCTGTCTCCTGCCTGTCCCTGGGCAATGGAATGTCTCGGTATA
AAACCCGATTGTATGCTCCATCTACTGAGATAGGGAAAAACCGCCTTAGGGCTGGAGGTGGGACCTGCGGGCAGCAATACTGCTTTGTA
AAGCACTGAGATGTTTATGTGTATGCATATCTAAAAGCACAGCACTTAATCCTTTACATTGTCTATGATGCAAAGACCTTTGTTCACAT
GTTTGTCTGCTGACCCTCTCCCCACAATTGTCTTGTGACCCTGACACATCCCCCTCTTCGAGAAACACCCACAGATGATCAGTAAATAC
TAAGGGAACTCAGAGGCTGGCGGGATCCTCCATATGCTGAACGCTGGTTCCCCGGGTCCCCTTCTTTCTTTCTCTATA
SEQ ID 6 - Non-coding region between U5 and first 5' splice site 
TCTGGTGCCCAACGTGGAGGCTTTTCTCTAGGGTGAAGGTACGCTCGAGCGTGGTCATTGAGGACAAGTCGACGAGAGATCCCGAGTAC
ATCTACAGTCAGCCTTACG
SEQ ID 7 - PCAP4 
MNPSEMQRKAPPRRRRHRNRAPLTHKMNKMVTSEEQMKLPSTKKAEPPTWAQLKKLTQLATKYLENTKVILQVYPTAPKRQRPSRTGHD
DDGGFVEKKRGKCGEKQERSDCYCVCVERSRHRRLHFVLY
SEQ ID 8- PCAP4a 
MNPSEMQRKAPPRRRRHRNRAPLTHKMNKMVTSEEQMKLPSTKKAEPPTWAQLKKLTQLATKYLENTKVYPTAPKRQRPSRTGHDDDGG
FVEKKRGKCGEKQERSDCYCVCVERSRHRRLHFVLY
SEQ ID 9- PCAP5 
MNPSEMQRKAPPRRRRHRNRAPLTHKMNKMVTSEEQMKLPSTKKAEPPTWAQLKKLTQLATKYLENTKVTQTPESMLLAALMIVSMVVY
PTAPKRQRPSRTGHDDDGGFVEKKRGKCGEKQERSDCYCVCVERSRHRRLHFVLY
SEQ ID 10 - PCAP1
   MNPSEMQRKAPPRRRRHRNRAPLTHKMNKMVTSEEQMKLPSTKKAEPPTWAQLKKLTQLATKYLENTKSAGVPNSSEETATIENGP
SEQ ID 11 - PCAP2
   MNPSEMQRKGPPQRCLQVYPTAPKRQRPSRTGHDDDGGFVEKKRGKCGEKQERSDCYCVCVERSRHRRLHFVLY
SEQ ID 12 - PCAP3
   MNSLEMQRKVWRWRHPNRLASLQVYPAAPKRQQPARMGHSDDGGFVKKKRGGYVRKREIRLSLCLCRKGRHKKLHFVLY
SEQ ID 13 - cORF 
MNPSEMQRKAPPRRRRHRNRAPLTHKMNKMVTSEEQMKLPSTKKAGPPTWAQLKKLTQLATKYLENTKVTQTPESMLLAALMIVSMVSA
GVPNSSEETATIENGP
SEQ ID 14 - TAR 
GAGATAGGGAAAAACCGCCTTAGGGCTGGAGGTGGACCTGCGGGCAGCAATACTGCTTTTTAAAGCATTGAGATGTTTATGTGTATGC
ATATCTAAAAGCACAGCACTTAATCCTTTACCTTGTCTATGATGCAAAGATCTTTGTTCAC
SEQ ID 15 - Figure 15, forward primer
   CATCTGGTGCCCAACGTGGAGGCTTTTCTCT
SEQ ID 16 - Figure 15, reverse primer 1
   AACCGCCATCGTCATCATGGCCCGTTCTCGA
SEQ ID 17 - Figure 15, reverse primer 2
   ACAGAATCTCAAGGCAGAAGAATTTTTCTT
SEQ ID 18 - Splice form A 
CATCTGGTGCCCAACGTGGAGGCTTTTCTCTAGGGTGAAGGTACGCTCGAGCGTGGTCATTGAGGACAAGTCGACGAGAGAATCCCGAG
TACATCTACAGTCAGCCTTACGTCTGCAGGTGTACCCAACAGCTCCGAAGAGACAGCGACCATCGAGAACGGCCATGATGACGATGGC
GGTTTTGTCGAAAAGAAAAGGGGGAAATGTGGGAAAAGCAAGAGAGATCAGATTGTTACTGTGTCTGTGTAGAAAGAAGTAGACATAG
GAGACTCCATTTTGTTATGTACTAAGAAAAATTCTT
SEQ ID 19 - Splice form B 
CATCTGGTGCCCAACGTGGAGGCTTTTCTCTAGGGTGAAGGTACGCTCGAGCGTGGTCATTGAGGACAAGTTGACGAGAGATCCCGAGT
ACATCTACAGTCAGCCTTGCGGAGAAAATCAGCTTCCTGTTTGGATACCCACTAGACATTTGAAGTTCTACAATGAACCCATCGGAGAT
GCAAAGAAAAGGGCCTCCACAGAGATGTCTGCAGGTGTACCCAACAGCTCCGAAGAGACAGCGACCATCGAGAACGGGCCATGATGACG
ATGGCGGTTTTGTCGAAAAGAAAAGGGGGAAATGTGGGGAAAAGCAAGAGAGATCAGATTGTTACTGTGTCTGTGTAGAAAGAAGTAGA
CATAGGAGACTCCATTTTGTTCTGTACTAAGAAAAATTCTTCTGCCTTGAGATTCTGT
SEQ ID 20 - Splice form C 
TGCCCAACGTGGAGGCTTTTCTCTAGGGTGAAGGTACGCTCGAGCGTGGTCATTGAGGACAAGTTGACGAGAGATCCCGAGTACATCTA
CAGTCAGCCTTGCGACATTTGAAGTTCTACAATGAACCCATCGGAGATGCAAAGAAAAGGCCTCCACAGAGATGTCTGCAGGTGTACC
CAACAGCTCCGAAGAGACAGCGACCATCGAGAACGGGCCATGATGACGATGGCGGTTTTGTCGAAAAGAAAAGGGGGAAATGTGGGAA
AAGCAAGAGAGATCAGATTGTTACTGTGTCTGTGTAGAAAGAAGTAGACATAGGAGACTCCATTTTGTTCTGTACTAAGAAAAATTCTT
CTGCCTTGAGATTCTGT
SEQ ID 21 - Splice form D 
CATCTGGTGCCCAACGTGGAGGCTTTTCTCTAGGGTGAAGGTACGCTCGAGCGTGGTCATTGAGGACAAGTTGACGAGAGATCCCGAGT
ACATCTACAGTCAGCCTTGCGTATCTACAGTTTAAAACCTGGTGGATTGATGGAGTACAAGAACAGACATTTGAAGTTCTACAATGAAC
CCATCGGAGATGCAAAGAAAAGGGCCTCCACAGAGATGTCTGCAGGTGTACCCAACAGCTCCGAAGAGACAGCGACCATCGAGAACGGG
CCATGATGACGATGGCGGTTTTGTCGAAAAGAAAAGGGGGAAATGTGGGGAAAAGCAAGAGAGATCAGATTGTTACTGTGTCTGTGTAG
AAAGAAGTAGACATAGGAGACTCCATTTGTTCTGTACTAAGAAAAATTTCTTCTGCCTTGAGATTCTGT
SEQ ID 22 - Splice form E 
CATCTGGTGCCCAACGTGGAGGCTTTCTCTAGGGTGAAGGTACGCTCGAGCGTGGTCATTGAGGACAAGTCGACGAGAGATCCCGAGT
ACATCTACAGTCAGCCTTACGACATTTGAAGTTCTACAATGAACCCATCAGAGATGCAAAGAAAAGCWCCTCCGCGGAGACGGAGACAT
CGCAATCGAGCACCGTTGACTCACAAGATGAACAAAATGGTGACGTCAGAAGAACAGATGAAGTTGCCATCCACCAAGAAGGCAGAGCC
GCCAACTTGGGCACAACTAAAGAAGCTGACGCAGTTAGCTACAAAATATCTAGAGAACACAAAGTCTGCAGGTGTACCCAACAGCTCCG
AAGAGACAGCGACCATCGAGAACGGGCCATGATGACGATGGCGGTTTTGTCGAAAAGAAAAGGGGGAAATGTGGGAAAAGCAAGAGAG
ATCAGATTGTTACTGTGTCTGTGTAGAAAGAAGTAGACATAGGAGACTCCATTTTGTTATGTACTAAGAAAAATTCTTCTGCCTTGAGA
TTCTGT
SEQ ID 23 - Splice form F 
CATCTGGTGCCCAACGTGGAGGCTTTTCTCTAGGGTGAAGGTACGCTCGAGCGTGGTCATTGAGGACAAGTCGACGAGAGATCCCGAGT
ACATCTACAGTCAGCCTTACGACATTTGAAGTTCTACAATGAACCCATCAGAGATGCAAAGAAAAGCACCTCCGCGGAGACGGAGACAT
CGCAATCGAGCACCGTTGACTCACAAGATGAACAAAATGGTGACGTCAGAAGAACAGATGAAGTTGCCATCCACCAAGAAGGCAGAGCC
GCCAACTTGGGCACAACTAAAGAAGCTGACGCAGTTAGCTACAAAATATCTAGAGAACACAAAGGTGACACAAACCCCAGAGAGTATGC
TGCTTGCAGCCTTGATGATTGTATCAATGGTGTCTGCAGGTGTACCCAACAGCTCCGAAGAGACAGCGACCATCGAGAACGGGCCATGA
TGACGATGGCGGTTTTGTCGAAAAGAAAAGGGGGAAATGTGGGGAAAAGCAAGAGAGATCAGATTGTTACTGTGTCTGTGTAGAAAGAA
GTAGACATAGGAGACTCCATTTTGTTATGTACTAAGAAAAATTCTTCTGCCTTGAGATTCTGT
SEQ ID 24 - Splice form G 
TCATCTGGTGCCCAACGTGGAGGCTTTTCTCTAGGGTGAAGGTACGCTCGAGCGTGGTCATTGAGGACAAGTTGACGAGAGATCCCGAG
TACATCTACAGTCAGCCTTGCGACATTTGAAGTTCTACAATGAACCCATCGGAGATGCAAAGAAAAGGCCTCCACAGAGATGGTAACC
CCAGTCACATGGATGGATAATCCTATAGAAGTATATGTTAATGATAGTGTATGGGTACCTGGCCCCACAGATGATCGCTGCCCTGCCAA
ACCTGAGGAAGAAGGGATGATGATAAATATTTCCATTGTGTATCGTTATCCTCCTATTTGCCTAGGGAGAGCACCAGGATGTTTAATGC
CTGCAGTCCAAAATTGTCTGCAGGTGTACCCAACAGCTCCGAAGAGACAGCGACCATCGAGAACGGGCCATGATGACGATGGCGGTTTT
GTCGAAAAGAAAAGGGGGAAATGTGGGGAAAAGCAAGAGAGATCAGATTGTTACTGTGTCTGTGTAGAAAGAAGTAGACATAGGAGACT
CCATTTTGTTCTGTACTAAGAAAAATTCTTCTGCCTTGAGATTCTGT
SEQ ID 25 - Splice form H 
CATCTGGTGCCCAACGTGGAGGCTTTTCTCTAGGGTGAAGGTACGCTCGAGCGTGGTCATTGAGGACAAGTCGACGAGAGATCCCGAGT
ACGTCTACAGTCAGCCTTACGACATTTGAAGTTCTACAATGAACCCATCGGAGATGCAAAGAAAAGGCCTCCACGGAGATGGTAACAC
CAGTCACATGGATGGATAATCCTATAGAAGTATATGTTAATGATAGCGAATGGGTACCTGGCCCCACAGATGATCGCTGCCCTGCCAAA
CCTGAGGAAGAAGGGATGATGATAAATATTTCCATTGGTCTGCAGGTGTACCCAACGGCTCCGAAGAGACAGCGACCATCGAGAACGGG
CCATGATGACGATGGCGGTTTTGTCGAAAAGAAAAGGGGGAAATGTGGGGAAAAGCAAGAGAGATCAGATTGTTACTGTGTCTGTGTAG
AAAGAAGTAGACATAGGAGACTCCATTTTGTTATGTGCTAAGAAAAATTCTTCTGCCTTGAGATTCTGT
SEQ ID 26 - Splice form I 
GGGTGAAGGTACTCTACAGTGTGGTCATTGAGGACAAGTTGACGAGAGAGTCCCAAGTACGTCCACGGTCAGCCTTGCGACATTTAAAG
TTCTACAATGAACTCACTGGAGATGCAAAGAAAAGTGTGGAGATGGAGACACCCCAATCGACTCGCCAGTCTACAGGTGTATCCAGCAG
CTCCAAAGAGACAGCAACCAGCAAGAATGGGCCATAGTGACGATGGTGGTTTTGTCAAAAAGAAAAGGGGGGGATATGTAAGGAAAAGA
GAGATCAGACTTTCACTGTGTCTATGTAGAAAAGGAAGACATAAGAAACTCCATTTTGTTCTGTACTAAGAAAAATTGTTTTGCCTTGA
GATGCTGT
SEQ ID 27 - Splice form J 
YGGAGATGCAAAGAAAAGCACCTCCGCGGAGACGGAGACATCGCAATCGAGCACCGTTGACTCACAAGATGAACAAAATGGTGACGTCA
GAAGAACAGATGAAGTTGTCATCCACCAAGAAGGCAGAGCCGCCAACTTGGGCACAACTAAAGAAGCTGACGCAGTTAGCTACAAAATA
TCTAGAGAACACAAAGGTGACACAAACCCCAGAGAGTATGCTGCTTGCAGCCTTGATGATTGTATCAATGGTGGTAAGTCTCCCTATGC
CTGCAGGAGCAGCTGCAGCTAACTATACCTACTGGGCCTATGTGCCTTTCCCGCCCTTAATTCGGGCAGTCACATGGATGGATAATCCT
ACAGAAGTATATGTTAATGATAGTGTATGGGTACCTGGCCCCATAGATGATCGCTGCCCTGCCAAACCTGAGGAAGAAGGGATGATGAT
AAATATTTCCATTGGGTATCATTATCCTCCTATTTGCCTAGGGAGAGCACCAGGATGTTTAATGCCTGCAGTCCAAAATTGGTTGGTAG
AAGTACCTACTGTCAGTCCCATCTGTAGATTCACTTATCACATGTCTGCAGGTGTACCCAACAGCTCCGAAGAGACAGCGACCATCGAG
AACGGGCCATGATGACGATGGCGGTTTTGTCGAAAAGAAAAGGGGGAAATGTGGGGAAAAGCMAGARAGATCAGATTGKTACTGKGTCT
GTGTAGAAAGAAGTAGACATAGGAGACTCCWTTTTGC
SEQ ID 28 - Splice form B protein
   MNPSEMQRKGPPQRCLQVYPTAPKRQRPSRTGHDDDGGFVEKKRGKCGEKQERSDCYCVCVERSRHRRLHFVLY
SEQ ID 29 - Splice form C protein
   MNPSEMQRKGPPQRCLQVYPTAPKRQRPSRTGHDDDGGFVEKKRGKCGEKQERSDCYCVCVERSRHRRLHFVLY
SEQ ID 30 - Splice form D protein
   MEYKNRHLKFYNEPIGDAKKRASTEMSAGVPNSSEETATIENGP
SEQ ID 31 - Splice form D protein
   MNPSEMQRKGPPQRCLQVYPTAPKRQRPSRTGHDDDGGEVEKKRGKCGEKQERSDCYCVCVERSRHRRLHFVLY
SEQ ID 32 - Splice form E protein
   MNPSEMQRKAPPRRRRHRNRAPLTHKMNKMVTSEEQMKLPSTKKAEPPTWAQLKKLTQLATKYLENTKSAGVPNSSEETATIENGP
SEQ ID 33 - Splice form F protein 
MNPSEMQRKAPPRRRRHRNRAPLTHKMNKMVTSEEQMKLPSTKKAEPPTWAQLKKLTQLATKYLENTKVTQTPESMLLAALMIVSMVSA
GVPNSSEETATIENGP
SEQ ID 34 - Splice form G protein 
MVTPVTWMDNPIEVYVNDSVWVPGPTDDRCPAKPEEEGMMINISIVYRYPPICLGRAPGCLMPAVQNCLQVYPTAPKRQRPSRTGHDDD
GGFVEKKRGKCGEKQERSDCYCVCVERSRHRRLHFVLY
SEQ ID 35 - Splice form H protein 
MVTPVTWMDNPIEVYVNDSEWVPGPTDDRCPAKPEEEGMMINISIGLQVYPTAPKRQRPSRTGHDDDGGFVEKKRGKCGEKQERSDCYC
VCVERSRHRRLHFVMC
SEQ ID 36 - Splice form I protein
   MNSLEMQRKVWRWRHPNRLASLQVYPAAPKRQQPARMGHSDDGGFVKKKRGGYVRKREIRLSLCLCRKGRHKKLHFVLY
SEQ ID 37 - Splice form J protein 
MNSLEMQRKAPPRRRRHRNRAPLTHKMNKMVTSEEQMKLSSTKKAEPPTWAQLKKLTQLATKYLENTKVTQTPESMLLAALMIVSMVVS
LPMPAGAAAANYTYWAYVPFPPLIRAVTWMDNPTEVYVNDSVWVPGPIDDRCPAKPEEEGMMINISIGYHYPPICLGRAPGCLMPAVQN
WLVEVPTVSPICRFTYHMSAGVPNSSEETATIENGP
SEQ ID 38 - Splice form K 
ACATTTGAAGTTCTACAATGAACCCATCRGAGATGCAAAGAAAAGCACCTCCGCGGAGACGGAGACATCGCAATCGAGCACCGTTGACT
CACAAGATGAACAAAATGGTGACGTCAGAAGAACAGATGAAGTTGCCATCCACCAAGAAGGCAGAGCCGCCAACTTGGGCACAACTAAA
GAAGCTGACGCAGTTAGCTACAAAATATCTAGAGAACACAAAGGTGACTCTGCAGGTGTACCCAACAGCTCCGAAGAGACAGCGACCAT
CGAGAACGGGCCATGATGACGATGGCGGTTTTGTCGAAAAGAAAAGGGGGAAATGTGGGGAAAAGCAAGAGAGATCAGATTGTTACTGT
GTCTGTGTAGAAAGAAGTAGACATAGGAGACTCCATTTTGTTATGTACTAAGAAAAATTCTT
SEQ ID 39 - Splice form K protein 
MNPSEMQRKAPPRRRRHRNRAPLTHKMNKMVTSEEQMKLPSTKKAEPPTWAQLKKLTQLATKYLENTKVTLQVYPTAPKRQRPSRTGHD
DDGGFVEKKRGKCGEKQERSDCYCVCVERSRHRRLHFVMY
SEQ ID 40 - Splice form L 
ACATTTGAAGTTCTACAATGAACCCATCRGAGATGCAAAGAAAAGCACCTCCGCGGAGACGGAGACATCGCAATCGAGCACCGTTGACT
CACAAGATGAACAAAATGGTGACGTCAGAAGAACAGATGAAGTTGCCATCCACCAAGAAGGCAGAGCCGCCAACTTGGGCACAACTAAA
GAAGCTGACGCAGTTAGCTACAAAATATCTAGAGAACACAAAGGTGTACCCAACAGCTCCGAAGAGACAGCGACCATCGAGAACGGGCC
ATGATGACGATGGCGGTTTTGTCGAAAAGAAAAGGGGGAAATGTGGGGAAAAGCAAGAGAGATCAGATTGTTACTGTGTCTGTGTAGAA
AGAAGTAGACATAGGAGACTCCATTTTGTTATGTACTAAGAAAAATTCTT
SEQ ID 41 - Splice form L protein 
MNPSEMQRKAPPRRRHRNRAPLTHKMNKMVTSEEQMKLPSTKKAEPPTWAQLKKLTQLATKYLENTKVYPTAPKRQRPSRTGHDDDGG
FVEKKRGKCGEKQERSDCYCVCVERSRHRRLHFVMY
SEQ ID 42 - Splice form M 
ACATTTGAAGTTCTACAATGAACCCATCAGAGATGCAAAGAAAAGCACCTCCGCGGAGACGGAGACATCGCAATCGAGCACCGTTGACT
CACAAGATGAACAAAATGGTGACGTCAGAAGAACAGATGAAGTTGCCATCCACCAAGAAGGCAGAGCCGCCAACTTGGGCACAACTAAA
GAAGCTGACGCAGTTAGCTACAAAATATCTAGAGAACACAAAGGTGACACAAACCCCAGAGAGTATGCTGCTTGCAGCCTTGATGATTG
TATCAATGGTGGTGTACCCAACAGCTCCGAAGAGACAGCGACCATCGAGAACGGGCCATGATGACGATGGCGGTTTTGTCGAAAAGAAA
AGGGGGAAATGTGGGGAAAAGCAAGAGAGATCAGATTGTTACTGTGTCTGTGTAGAAAGAAGTAGACATAGGAGACTCCATTTTGTTAT
GTACTAAGAAAAATTCTT
SEQ ID 43 - Splice form M protein 
MNPSEMQRKAPPRRRRHRNRAPLTHKMNKMVTSEEQMKLPSTKKAEPPTWAQLKKLTQLATKYXLENTKVTQTPESMLLAALMIVSMVV
YPTAPKRQRPSRTGHDDDGGFVEKKRGKCGEKQERSDCYCVCVERSRHRRLHFVMY
SEQ ID 44 - LTR of herv-k(hml-2.hom) 
TGTGGGAAAAGCAAGAGAGATCAGATTGTTACTGTGTCTGTGTAGAAAGAAGTAGACATAGGAGACTCCATTTTGTTATGTACTAAGA
AAAATTCTTCTGCCTTGAGATTCTGTTAATCTATGACCTTACCCCCAACCCCGTGCTCTCTGAAACATGTGCTGTGTCCACTCAGGGTT
AAATGGATTAAGGGCGGTGCAGGATGTGCTTTGTTAAACAGATGCTTGAAGGCAGCATGCTCCTTAAGAGTCATCACCACTCCCTAATC
TCAAGTACCCAGGGACACAAAAACTGCGGAAGGCCGCAGGGACCTCTGCCTAGGAAAGCCAGGTATTGTCCAACGTTTCTCCCCATGTG
ATAGCCTGAAATATGGCCTCGTGGGAAGGGAAAGACCTGACCGTCCCCCAGCCCGACACCCGTAAAGGGTCTGTGCTGAGGAGGATTAG
TAAAAGAGGAAGGAATGCCTCTTGCAGTTGAGACAAGAGGAAGGCATCTGTCTCCTGCCTGTCCCTGGGCAATGGAATGTCTCGGTATA
AAACCCGATTGTATGCTCCATCTACTGAGATAGGGAAAAACCGCCTTAGGGCTGGAGGTGGGACCTGCGGGCAGCAATACTGCTTTGTA
AAGCACTGAGATGTTTATGTGTATGCATATCTAAAAGCACAGCACTTAATCCTTTACATTGTCTATGATGCAAAGACCTTTGTTCACAT
GTTTGTCTGCTGACCCTCTCCCCACAATTGTCTTGTGACCCTGACACATCCCCCTCTTCGAGAAACACCCACAGATGATCAGTAAATAC
TAAGGGAACTCAGAGGCTGGCGGGATCCTCCATATGCTGAACGCTGGTTCCCCGGGTCCCCTTCTTTCTTTCTCTATACTTTGTCTCTG
TGTCTTTTTCTTTTCCAAATCTCTCGTCCCACCTTACGAGAAACACCCACAGGTGTGTAGGGGCAACCCACCCCTACA
SEQ ID 45 - HML-2 LTR (i) 
TGTGGGAAAAGCAAGAGAGATCAGATTGTCACTGTATCTGTGTAGAAAGAAGTAGACATGGAGACTCCATTTGTTATGTACTAAGA
AAAATTCTTCTGCCTTGAGATTCTGTGACCTTACCCCCAACCCCGTGCTCTCTGAAACATGTGCTGTGTCAAACTCAGGGTTAAATGGA
TTAAGGGCGGTGCAGGATGTGCTTTGTTAAACAGATGCTTGAAGGCAGCATGCTCCTTAAGAGTCATCACCACTCCCTAATCTCAAGTA
CCCAGGGACACAAACACTGCGGAAGGCCGCAGGGACCTCTGCCTAGGAAAGCCAGGTATTGTCCAAGGTTTCTCCCCATGTGATAGTCT
GAAATATGGCCTCGTGGGAAGGGAAAGACCTGACCGTCCCCCAGCCCGACACCCGTAAAGGGTCTGTGCTGAGGAGGATTAGTAAAAGA
GGAAGGCATGCCTCTTGCAGTTGAGACAAGAGGAAGGCATCTGTCTCCTGCCCGTCCCTGGGCAATGGAATGTCTCGGTATAAAACCGG
ATTGTACGTTCCATCTACTGAGATAGGGAAAAACCGCCTTAGGGCTGGAGGTGGGACCTGCGGGCAGCAATACTGCTTTTTAAAGCATT
GAGATGTTTATGTGTATGCATATCTAAAAGCACAGCACTTAATCCTTTACCTTGTCTATGATGCAAAGATCTTTGTTCACGTGTTTGTC
TGCTGACCCTCTCCCCACTATTGTCTTGTGACCCTGACACATCCCCCTCTCGGAGAAACACCCACGAATGACCAATAAATACTAAAGGG
AACTCAGAGGCTGGCGGGATCCTCCATATGCTGAACGCTGGTTCCCCGGGCCCCCTTATTTCTTTCTCTACACTTTGTCTCTGTGTCTT
TTTCTTTCCTAAGTCTCTCGTTCCACCTTACGAGAAACACCCACAGGTGTGGAGGGGCAACCCACCCCTACA
SEQ ID 46 - HML-2 LTR (ii) 
TGTGGGAAAAGCAAGAGAGATCAGATTGTTACTGTGTCTGTGTAGAAAGAAGTAGACATGGAGACTCCATTTGTTATGTGCTAAGA
AAAATTCTTCTGCCTTGAGATTCTGTTAATCTATGACCTTACCCCCAACCCCGTGCTCTCTGAAACATGTGCTGTGTCAACTCAGGGTT
GAATGGATTAAGGGCGGTGCAGGATGTGCTTTGTTAAACAGATGCTTGAAGGCAGCATGCTCCTTAAGAGTCATCACCACTCCCTAATC
TCAAGTACCCAGGGACACAAAAACTGCGGAAGGCCGCAGGGACCTCTGCCTAGGAAAGCCAGGTATTGTCCAAGGTTTCTCCCCATGTG
ATAGTCTGAAATATGGCCTCGTGGGAAGGGAAAGACCTGACCATCCCCCAGCCCGACACCCATAAAGGGTCTGTGCTGAGGAGGATTAG
TATAAGAGGAAGGCATGCCTCTTGCAGTTGAGACAAGAGGAAGGCATCTGTCTCCTGCCTGTCCCTGGGCAATGGAATGTCTCGGTATA
AAACCCGATTGTATGCTCCATCTACTGAGATAGGGAAAAACCGCCTTAGGGCTGGAGGTGGGACCTGCGGGCAGCAATACTGCCTTGTA
AAGCATTGAGATGTTTATGTGTATGCATATCTAAAAGCACAGCACTTAATCCTTTACATTGTCTATGATGCAAAGACCTTTGTTCACGT
GTTTGTCTGCTGACCCTCTCCCCACAATTGTCTTGTGACCCTGACACATCCCCCTCTTTGAGAAACACCCACAGATGATCAATAAATAC
TAAGGGAACTCAGAGGCTGGCGGGATCCTCCATATGCTGAACGCTGGTTCCCCGGTTCCCCTTATTTCTTTCTCTATACTTTGTCTCTG
TGTCTTTTTCTTTTCCAAATCTCTCGTCCCACCTTACGAGAAACACCCACAGGTGTGTAGGGGCAACCCACCCCTACA
SEQ ID 47 - HML-2 LTR (iii) 
TGTGGGGAAAAGCAAGAGAGATCAGATTGTTACAGTGTCTGTGTAGAAAGAAGTAGACATAGGAGACTCCATTTTGTTCTGTACTAAGA
AAAATTCTTCTGCCTTGAAATTCTGTTAATCTATAACCTTACCCCCAACCCCGTGCTCTTTGAAACATGTGCTGTGTCAACTCAGAGTT
AAATGGATTAAGTGCGGTGCAAGATGTGCTTTGTTAAACAGATGCTTGAAGGCAGCATGCTCCTTGAGAGTCATCACCACTCCCTAATC
 
TCAAGTACCCAGGGACACAAAAACTGCGGAAGGCCTCAGGGACCTCTGCCTAGGAAAGCCAGGTATTGTCCAAGGTTTCTCCCCATGTG
ATAGTCTGAAATATGGCCTCGTGGAAGGGAAAGACCTGACCATCCCCCAGCCCGACACCCGTAAAGGTCTGTGCTGAGGAGGATTAG
TAAAAGAGGAAGGAACGCCTCTTGCAGTTGAGACAAGAGGAAGGCATCTGTCTCCTGCCTGTCCCTGGGCAATGGAATGTCCCGGTATA
AAACCCGATTGTATGCTCCATCTACTGAGATAGGGAAAAACCGCCTTAGGGCTGGAGGTGGGACCTGCGGGCAGCAATACTGCTTTGTA
AAGCATTGAGCTGTTTATGTGTATGCATATCTAAAAGCACAGCACTTAATCCTTTACATTGTCTATGATGCAAAGACCTTTGTTCACGT
GTTTGTCTGCTGACCCTCTCCCCACAATTGTCTTGTGACCCTGACACATCCCCCTCTTCGAGAAACACCCACGAATGATGAATAAATAC
TAAGGGAACTCAGAGGCTGGCGGGATCCTCCATATGCTGAACGCTGGTTCCCCGGGTCCCCTTACTTCTTTCTCTGTACTTTGTCTCTG
TGTCTTTTTCTTTCCTAAGTCTCTCGTTCCACCTTACGAGAAATACCCACAGGTGTGGAGGGGCAACCCACCCCTACA
SEQ ID 48 - HML-2 LTR (iv) 
TGTGGGAAAAGCAAGAGAGATCAGATTGTTACTGTGTCTGTGTAGAAAGAAGTAGACATAGGAGACTCCATTTTGTTCTGTACTAAGA
AAAATTCTTCTGCCTTGAGATTCTGTTAATCTATAACCTTACCCCCAACCCCGTGCTCTCTGAAACATGTGCTATGTCAACTCAGAGTT
GAATGGATTAAGGGCGGTGCAAGATGTGCTTTGTTAAACAGATGCTTGAAGGCAGCACGCTCCTTAAGAGTCATCACCACTCCCTAATC
TCAAGTACCCAGGGACACAAAAACTGCGGAAGGCCGCAGGGACCTCTGCCTAGGAAAGCCAGGTATTGTCCAAGGTTTCTCCCCATGTG
ATAGTCTGAAATATGGCCTCGTGGGAAGGGAAAGACCTGACCATCCCCCAGCCCGACACCTGTAAAGGGTCTGTGCTGAGGAGGATTAG
TATAAGAGGAAGGCATGCCTCTTGCAGTTGAGACAAGAGGAAGGCATCTGTCTCCTGCCCGTCCCTGGCAATGGAATGTCTCGGTATA
AAACCCGATTGTATGTTCCATCTACTGAGATAGGGAAAAACCGCCTTAGGGCTGGAGGTGGGACCTGCGGGCAGCAATACTGCTTTGTA
AAGCATTGAGATGTTTATGTGTATGCATATCTAAAAGCACAGCACTTAATCCTTTACCTTGTCTATGATGCAAAGACCTTTGTTCACGT
GTTTGTCTGCTGACCCTCTCCCCACGATTGTCTTGTGACCCTGACACATCCCCGTCTTCGAGAAACACCCACGAATGATCAATAAATAC
TAAGGGAACTCAGAGGCTGGCGGGATCCTCCATATGCTGAACGCTGGTTCCCCAGGTCCCCTTATTTCTTTCTCTATACTTTGTCTCTG
TGTCTTTTTCTTTTCCAAGTCTCTCGTTCCATCTTACGAGAAACACCCACAGGTGTGGAGGGCAACCCACCCCTACA
SEQ ID 49 - TAR of herv-k(hml-2.hom) [GenBank AF074086] 
GAGATAGGGAAAAACCGCCTTAGGGCTGGAGGTGGACCTGCGGGCAGCAATACTGCTTTGTAAAGCACTGAGATGTTTATGTGTATGC
ATATCTAAAAGCACAGCACTTAATCCTTTACATTGTCTATGATGCAAAGACCTTTGTTCAC
SEQ ID 50 - Rest of RU5 of herv-k(hml-2.hom) [GenBank AF074086] 
ATGTTTGTCTGCTGACCCTCTCCCCACAATTGTCTTGTGACCCTGACACATCCCCCTCTTCGAGAAACACCCACAGATGATCAGTAAAT
ACTAAGGGAACTCAGAGGCTGGCGGATCCTCCATATGCTGAACGCTGGTTCCCCGGGTCCCCTTCTTTCTTTCTCTATACTTTGTCTC
TGTGTCTTTTTCTTTTCCAAATCTCTCGTCCCACCTTACGAGAAACACCCACAGGTGTGTAGGGGCAACCCACCCCTACA
SEQ ID 51 - Downstream of 'potential splice site B' 
GTGTACCCAACAGCTCCGAAGAGACAGCGACCATCGAGAACGGGCCATGATGACGATGGCGGTTTTGTCGAAAAGAAAAGGGGAAATG
TGGGGAAAAGCAAGAGAGATCAGATTGTTACTGTGTCTGTGTAGAAAGAAGTAGACATAGGAGACTCCATTTGTTATGTACTAA
SEQ ID 52 - Downstream of 'potential splice site B'
   GTGTACCCAACAGCTCCGAAGAGACAGCGACCATCGAGAACGGGCCATGA
SEQ ID 53 - Antisense oligo for mapping 5' end of mRNA
   AGAGAAAAGCCTCCACGTTGGGCACC
SEQ ID 54 - Antisense primer
   GTAGGGGTGGGTTGCCCC
SEQ ID 55 - Sense primer
   AAACCGCCTTAGGGCTGGAGGTGGGAC
SEQ ID 56 - Sense primer
   TGCGGGCAGCAATACTGC
SEQ ID 57 - Sense primer
   TAAAGCACTGAGATGTTTATGTGTATGC
SEQ ID 58 - Sense primer
   GCACAGCACTTAATCCTTTACATT
SEQ ID 59 - Sense primer
   GTTTGTCTGCTGACCCTCTCCC
SEQ ID 60 - V5 tag
   GKPIPNPLLGLDST
SEQ ID 61 - Motif common to exon 3 of PCAP2, PCAP3 and PCAP4
   LQVYP
SEQ ID 62 - Motif common to exon 3 of PCAP2, PCAP3, PCAP4 and PCAP4a
   APKRQ
SEQ ID 63 - Motif common to exon 3 of PCAP2, PCAP3, PCAP4 and PCAP4a
   DDGGFV
SEQ ID 64 - Motif common to exon 3 of PCAP2, PCAP3, PCAP4 and PCAP4a
   KKRG
SEQ ID 65 - Motif common to exon 3 of PCAP2, PCAP3, PCAP4 and PCAP4a
   LHFVLY
SEQ ID 66 - Motif common to exon 3 of PCAP2, PCAP4 and PCAP4a
   YPTAPKRQRPSRTGHDDDGGFVEKKRGKCGEKQERSDCYCVCVERSRHRRLHFVLY
SEQ ID 67 - Motif common to exon 3 of PCAP2 and PCAP4
   LQVYPTAPKRQRPSRTGHDDDGGFVEKKRGKCGEKQERSDCYCVCVERSRHRRLHFVLY
SEQ ID 68 - Exon 3 region of PCAP3
   LQVYPAAPKRQQPARMGHSDDGGFVKKKRGGYVRKREIRLSLCLCRKGRHKKLHFVLY
SEQ ID 69 - Optional disclaimer
   MNPSEMQRKGPPQRCLQVYPTAPKRQRPSRTGHDDDGGFVEKKRGKCGEKQERSDCYCVCVERSRHRRLHFVLY
SEQ ID 70 - Optional disclaimer
   MNPSEMQRKGPPQRCLQVYPTAPKRQRPSRTGHDDDGGFVEKKRGKCGEKQERSDCYCVCVERSRHRRLHFVMY
SEQ ID 71 - Optional disclaimer
   MNPSEMQRKGPPQRCLQVYPTAPKRQRPSRTGHDDDGGFVEKKRGKCGEKQERSDCYCVCVERSRHRRLHFVLC
SEQ ID 72 - Optional disclaimer
   MNPSEMQRKGPPQRCLQVYPTAPKRQRPSRTGHDDDGGFVEKKRGKCGEKQERSDCYCVCVERSRHRRLHFVMC
SEQ ID 73 - Optional disclaimer
   MNPSEMQRKGPPRRCLQVYPTAPKRQRPSRTGHDDDGGFVEKKRGKCGEKQERSDCYCVCVERSRHRRLHFVLY
SEQ ID 74 - Optional disclaimer
   MNPSEMQRKGPPRRCLQVYPTAPKRQRPSRTGHDDDGGFVEKKRGKCGEKQERSDCYCVCVERSRHRRLHFVMY
SEQ ID 75 - Optional disclaimer
   MNPSEMQRKGPPRRCLQVYPTAPKRQRPSRTGHDDDGGFVEKKRGKCGEKQERSDCYCVCVERSRHRRLHFVLC
SEQ ID 76 - Optional disclaimer
   MNPSEMQRKGPPRRCLQVYPTAPKRQRPSRTGHDDDGGFVEKKRGKCGEKQERSDCYCVCVERSRHRRLHFVMC
SEQ ID 77 - MDALTR 
TGTAGGGAAAAGAAAGAGAGATCACACTGTTACTGTGTCTATGTAGAAAAAGGAAGACATAAGAAACTCCATTTTGATCTGTACTAAGA
AAAATTCTTCTGCTTTGAAATGCTATTAATCTGTAACCCTAGCCCCAACCCTGTGCTCACAGAAACATGCGCTGTATTGACTCAAGGTT
AATGGATTTAGGGCTGTGCAGGATGTGCTTTGTTAACAATGTGTTTGAAGGCAGTATGCTTGGTAAAGGTCATCGCCATTCTCCAGTCT
TGAGTACCCAGGGACACAATGCACTGTGGAAAGCCATGGGGACCTCTGCCCAAGAAAGCCTGGGTGTTGTCCAGGCTTCCCCACACTGA
GACAGCCTGAGATGTGGCCTCGTTGGAAGGGAAAGACCTTACATTATAGTCCCCCAGCCGGACACCCATAAAAGGTCTGTGCTGAGGAG
GATTACTGAAAGAGGAAGGCCTCTTTGCAGTTAAGAGGAAAGCATCTGTCTCATGATCCCCTGGGAATGGAATGTCTTGGTGTAAAACC
TGATCGTACATTCTATTTACTGAGATAGGAGAAAACCGCCCTATGGCTGGAGGTGAGACATGCTGGTGGCAATACCGATCTTTACTGCA
CGGCAATACTGATCTTTACTGCACTGAGATGTTTATGTAAAGTTAAACATAAATCTAGCCTACGTGCACATTCAGGCATAGCACCTTTC
CTTAAACTTATTTATGACACAGAGTCTTTTGTTCACGTGTTTTCCTGTTGACCCTCTCTCCACCATTACCCTATAGTCCTGCCACATCC
CCCTCACTGAGATAGTAGAGATAATGATCAATAAATACTGAGGGAATTCAGAAACCAGTGCCGGTGCAGGTCCTCACTTGCTGAGTGCC
GGTCCCCTGGGCCCACTTTTCTTCCTCTATGCTTTACCTCTGTGTCTTATTTCTTTTCTCAGTCTCTCGTCTCCACCTTGCGAGAAATA
CCCACAGGTGTGGAGGGGCTGGCCCCCTTCA
SEQ ID 78 - Third frame amino acid sequence from HERV-K
   VYPTAPKRQQPSRTGHDNDGSFVEKRRGKCGEKKERSDCYCVCVERSRHRRLHFVLY
SEQ ID 79 - Third frame amino acid sequence from HERV-K
   VFPTALKRQRPSRMGHDDDGGFVEKKRGKCGEKKERSDCYCVCVERSRHRRLHFVLY
SEQ ID 80 - Third frame amino acid sequence from HERV-K
   VYPTAPKRQQPSRTGHDDDGGFVKKKRGKCGEKKERSDCYCVCVERSRHRILHFVLY
SEQ ID 81 - Third frame amino acid sequence from HERV-K
   LQVYPAAPKRERPVRTGHDDDGGFLKKKRGICREKKERSDGYCVYVEKEDIRNFILICTLNNCFA
SEQ ID 82 - Third frame amino acid sequence from HERV-K
   VYPAAPKRQRPSRTSHDDDGGLSKRKWGI3VGKREIRLLLCLC
SEQ ID 83 - Third frame amino acid sequence from HERV-K
   VYPTAPKRQRPSRTGHDDDGGFVEKKRGKCGEKQERSDCYCVCVERSRHGRLHFVM
SEQ ID 84 - Third frame amino acid sequence from HERV-K
   VYLTAPKRQRPSRTGHDDDGGFVKKKRGKCGEKKERSDCYCVCVERSRHRILHFVLY
SEQ ID 85 - Third frame amino acid sequence from HERV-K
   VYPTALKRQRPKRMGHDDYGSSVKKKRGICRGKKERSDCYCVYVEK
SEQ ID 86 - Third frame amino acid sequence from HERV-K
   VYPTAPKRQRPLRTGHDDDGGFVKKKRGKCGEKKERSDCYCVCVERSRHRRLHFVLY
SEQ ID 87 - Third frame amino acid sequence from HERV-K
   VYPTAPKRQQPSRTGHDDDGCFLEKKRGKCGEKKERSDCYCVCVERSRHRRLHFVLY
SEQ ID 88 - Third frame amino acid sequence from HERV-K VYRTALKRQRPSRMGHDDDGSFVEKKRGKCGEKKERSDCYCVCVERSRHRRLHFVLY
SEQ ID 89 - Third frame amino acid sequence from HERV-K
   VYPTAPKRQRPSRTGHDDDGGFVEEKRGKCGAKKERSDCYCVCVERSRHRRLHFVLY
SEQ ID 90 - Third frame amino acid sequence from HERV-K
   VYPTAPKRQQPSRNSHDDDGGFVEKGEMWGKERSDCYCVCVERSRHRRLHFVLY
SEQ ID 91 - Third frame amino acid sequence from HERV-K
   VYPTAPKRQRPSRTGHDDDGGFVEKKRRKSGEKREIRLLLCLCRK
SEQ ID 92 - Third frame amino acid sequence from HERV-K
   VYPTAPKRQRPSRTGHDDDGGFVRKKRGKCGEKKERSDCYCVCVERTRHRRFHFVLY
SEQ ID 93 - Third frame amino acid sequence from HERV-K
   VYPTAPKRQRPSRTGQYDDGSFVKKKRGRKEKGEMWGKEREIRLLLCLCRK
SEQ ID 94 - Third frame amino acid sequence from HERV-K
   VYPAAPKRQRPVRMGHNDDVSFVKKKRGICREKKERSDCYCVYVEK
SEQ ID 95 - Third frame amino acid sequence from HERV-K
   VYPTAPKRQRPSRMGHDDYGGFVEKKRGKCGEKKERSDCYCVCVERSRHRRLHFVLY
SEQ ID 96 - Third frame amino acid sequence from HERV-K
   VYPTAPKRQQPSRMGHDDDGGFVKKKRGKCGEKKERSDCYFVCVERSRHRRLHFVLY
SEQ ID 97 - Third frame amino acid sequence from HERV-K
   VYPTAPKRQRPSRTGHDDDGGFVEKKRGKCGEKQERSDCYCVCVERSRHRRLHFVMY
SEQ ID 98 - Third frame amino acid sequence from HERV-K
   VYPTAPKRQRPSRTGHDDDGGFVEKKRGKCGEKQERSDCHCVCVERSRHGRLHFVMY
SEQ ID 99 - Third frame amino acid sequence from HERV-K
   VYPTAPKRQRPSRTGHDDSGGFVEKKRGKCGEKKERSDCYCVCVERSRHRRLHF
SEQ ID 100 - Third frame amino acid sequence from HERV-K
   VYPTAPKRQRPSRMGHDDDGGFVEKKRGKCGEKKERSDCYCVCVERSRHRRLHFVLY
SEQ ID 101 - Third frame amino acid sequence from HERV-K
   VYPTARKRQQPSRTGHDDDGGFVVKKRGKCGEKKERSDCYCVCVERSRHRRLHFVLY
SEQ ID 102 - Third frame amino acid sequence from HERV-K
   VYPTAPKRQRPSRTGHDDDGGFVEKKRGKCGEKKERSDCYCVCVERSRHRRLHFVLY
SEQ ID 103 - Third frame amino acid sequence from HERV-K
   VYPTAPKRQRPSRTGHDDDGGFVEKKEKGEMWGKEREIRLLLCLC
SEQ ID 104 - Third frame amino acid sequence from HERV-K
   VYPTAPKRQRPSRTGHDDDGGFVEKQRGKCGEKKERSDCYCVCVERSRHRRLHFVLY
SEQ ID 105 - Third frame amino acid sequence from HERV-K
   VYPTAPKRQQPSRTGHDEDGGFVERKRGNCGEKKERSDCYCVCVERSRHRRLHFALY
SEQ ID 106 - Third frame amino acid sequence from HERV-K
   VYPTAPKRQRPSRTGHDDDGGFVEKKRGKCGGKNERSDCYCVCVERSRHRRLHFVLY
SEQ ID 107 - Third frame amino acid sequence from HERV-K
   VYPTAPKRQRPSRMGHDDDGGFVEKKRGKCGEKQERSDCYCVCIERSRHRRLHFVLY
SEQ ID 108 - Third frame amino acid sequence from HERV-K
   LQVYPTAPKRQQPARTGHNDDGSFVKKKRGICREKKEISDCYCIFVEKEDIRNSILTCTVNNCFA
SEQ ID 109 - Third frame amino acid sequence from HERV-K
   VYPAAPKRQRPSRTSHDDDGSFVKKKRVMWGKERSDCYCVYVERSRHKRLHFVLY
SEQ ID 110 - Third frame amino acid sequence from HERV-K
   LQVYPAAPERQRPGRRGHDDGGGFVKTKRGICRGKKERSDCYCVYIEREDIRDSILKKICTLSNCFAEMLLICSFAPATLPQP
SEQ ID 111 - Third frame amino acid sequence from HERV-K
   VYPTAPKRQRPSRTGHDDNGGFVEKKRGKCGEKKERSDCYCVCVERSRHRRLHFVLY
SEQ ID 112 - Third frame amino acid sequence from HERV-K
   LQVYPAAPERQRPGRRGHDDGGGFVKTKRGICRGKKERSDCYCVYIEREDIRDSILKKNCTLNNCFAEMFLICSFAPATFPQP
SEQ ID 113 - Third frame amino acid sequence from HERV-K
   VYPTAPKRQRPSRTGHDDDGGFVEKKRGKCGEKRDQ
SEQ ID 114 - Third frame amino acid sequence from HERV-K
   VYPTAPKRQRPSRTGHDDSGSFVKKKRGKCGEKKERSDCYCVCVERSRHRRLRFVLY
SEQ ID 115 - Third frame amino acid sequence from HERV-K
   VYPTAPKRQRPSRMGHDDDGGFVEKQRGKCREKKERSDCYCVCVERSWHRRLHFVLY
SEQ ID 116 - Third frame amino acid sequence from HERV-K
   VYPTALKRQQPSRTGHDDDGGFVKKKRGKCGEKQERSDCHCVCVERSRHRRLHFVMY
SEQ ID 117 - Third frame amino acid sequence from HERV-K
   VYPTARKRQRPSRTGHDDDGGFVEKKRGKCGEKQERSDCYCVCVERSRHRRLHFVLY
SEQ ID 118 - Third frame amino acid sequence from HERV-K
   KPRRTKTQHTRISGTHSTCGEKQERSDCYCVCVERSRHRRLHFVLY
SEQ ID 119 - Third frame amino acid sequence from HERV-K
   VYPAAPERQRPARRGHDDGGGFVKTKRGICRVKKERSDCYCVYIER
SEQ ID 120 - Third frame amino acid sequence from HERV-K
   LQVYPAAPERQRPARRGHDDGGGFVKTKMGICREKKERSDCYCVYIEREDIRDSILKKTCTLNNCFAEMLLICSFAPATLPQP
SEQ ID 121 - Third frame amino acid sequence from HERV-K
   VYPTAPKRQRPSRKGHDDDGGFVEKKRGKCGEKKERSDCYCVCVERSRHRRHHFVLY
SEQ ID 122 - Third frame amino acid sequence from HERV-K
   LQVYTTAPERQRPARTGHDDDGGFVKKKRGKCREKKERSDCHCAYVEREDIRDSILKKTCTLNNCFAEMLLICSF
SEQ ID 123 - Third frame amino acid sequence from HERV-K
   VYPTAPKRQRPSRTGHDDDGGFVEKKREKCGEKKERSNCYCVCVERSRHRRLHFVLY
SEQ ID 124 - Third frame amino acid sequence from HERV-K
   LQVYPTALKRQQPSRTGHDDDGSFVEKKRGKCGEKKERSDCYCVCVERSRHRRFQKKK
SEQ ID 125 - Third frame amino acid sequence from HERV-K
   VYPTALKRQRPSRTGHDDDGGFVEKKREKCGEKKDQIVTVSVERSRHRRLHFVLY
SEQ ID 126 - Third frame amino acid sequence from HERV-K
   VYPTAPKRQPPSRTGHDDDGGFVLKKRGKCGEKKERSDCYHVCVERSRHRRHHFVLY
SEQ ID 127 - Third frame amino acid sequence from HERV-K
   VYPTAPKRQRPSRTGHDDDGGFVEKKRGKCGEKKERSDCYCVCLERSRHRRLHFVL
SEQ ID 128 - Third frame amino acid sequence from HERV-K
   VYSTAPKRQRPSRTGHDDDGGFVEKKRGKCGEKKERSDCYCVCVERSRHSRLHFVLY
SEQ ID 129 - Third frame amino acid sequence from HERV-K
   VYPTAPKRQRPSRTGHDDNGSFVEKKKGKCGEKKERSDCYCVCVERSRHRRLHFVLY
SEQ ID 130 - Third frame amino acid sequence from HERV-K
   VYPTAPKRQQPSRTGHDDDGGFVKKKRGKCGEKKERSDCYCVCVERSRHRRLHFVLY
SEQ ID 131 - Third frame amino acid sequence from HERV-K
   VYPTASKRQPPSGTDHDDDGGFVKKKRGKCGEKKERSDCYCVCVERSRHRRLHFLLY
SEQ ID 132 - Third frame amino acid sequence from HERV-K
   PSEQRPRETNGCHSGPDPRHSQEGPCGEKKEISDCYCVYVERSRHKRLHFVV
SEQ ID 133 - Third frame amino acid sequence from HERV-K
   VYPTAPKRQRPSRTGSDDDGGFVEKKRGKCGEKKERTDCYCVCVERSRHRRLHFVLY
SEQ ID 134 - Third frame amino acid sequence from HERV-K
   VYPTAPKKQQPSIMGHDDDGGFVKKKRGKCGEKRDQ
SEQ ID 135 - Third frame amino acid sequence from HERV-K
   VYPTAPKRQRPSRTGHDDDGGFVKKKRGKCGEKKEKSDCYCVCVERSRHRRLHFVLY
SEQ ID 136 - Third frame amino acid sequence from HERV-K
   SPSAQRPPRLGGVPNSSLRTGHDDDGGFVEWRGGKCGEKIDKSDCCCVCVEGSRRRRLHFVLY
SEQ ID 137 - Third frame amino acid sequence from HERV-K
   VYPTAPKRQRPSRTGHDDDGGFVKKKRGKCGEKKERSDCYCVCVERSRHRRLHFVLY
SEQ ID 138 - Third frame amino acid sequence from HERV-K
   VYPTAPKRQRPSRTGHDDDGGFVEKKMGKFGEKKERSDCYCVCVERSRHRRLHFVLY
SEQ ID 139 - Third frame amino acid sequence from HERV-K
   VYPTAPKRQRPLRMGHDDDGSFVKKKRGKCGEKKERSDCYCVCVERSRHRRLHFVLY
SEQ ID 140 - Third frame amino acid sequence from HERV-K
   VYPTAPKRQRLSRTGHDDDGGFVEKKRGKCGEKKDQ
SEQ ID 141 - Third frame amino acid sequence from HERV-K
   VYPTAPKRQQPSRTGHDDDGGFVKNKRGKRGEKKERSDCYCVCVERSRHRRHPFVLY
SEQ ID 142 - Third frame amino acid sequence from HERV-K
   VYPTALKRQRPSRTGHDNDGGFVEKKRGKCGEKQERSDCHCVCVERSRHRRLHFVMY
SEQ ID 143 - Third frame amino acid sequence from HERV-K
   VHPTAPKRQRPSRTGHDDNGSFVEKKRGKCGEKKERSDCYCVCVERSRHRRLHFVLY
SEQ ID 144 - Third frame amino acid sequence from HERV-K
   EVYPIAPKRQRPSRTGHDDDGGFVEKKRGKCGEKKERSDCYCVCVERSRYRRLHFVLYLEKFFCLGMLLIYNLTPNPVLSETCAV
SEQ ID 145 - Third frame amino acid sequence from HERV-K
   VYPTAPKRQRPLRMGHDDDGGFVKKKRGKCGEKKERSDCYCVCVERSRHRRLHFVLY
SEQ ID 146 - Third frame amino acid sequence from HERV-K
   VYPTAPKRQRPSRTGHDDDGGFVEKKRGKCGGKKERSDCSCVCVERSRHRRLHFVLY
SEQ ID 147 - Third frame amino acid sequence from HERV-K
   VYPTALKRQRPSRTGHDDDGGFVEKKRRKCGEKKERTDCYCVCVERSRHRGLHFVLY
SEQ ID 148 - Third frame amino acid sequence from HERV-K
   VYPTAPKRQRPSRTGHDDDGGFVEKKRRKCGEKKERTDCYCVCVERSRHRGLHFVLY
SEQ ID 149 - Third frame amino acid sequence from HERV-K
   EVYPTSPKRQQPSRMGHDDDGGFVAKKRGKCGEKKERSDCYCVCVERSRHRRLHFVLYLEKFFCLGMLLIYNFTPNHVLSETC
SEQ ID 150 - Third frame amino acid sequence from HERV-K
   VYPTASKRQPPSGTDHDDDGSFVKKKRGKCGEKEERSDCYCVCVERSRHRRLHFLLY
SEQ ID 151 - Third frame amino acid sequence from HERV-K
   VYPTAPKRQRPSRTGHDDDGGFVEKKRGKCGEKKERSDCYCVCVERSRHRRHHFVLY
SEQ ID 152 - Third frame amino acid sequence from HERV-K
   VYPTAPKRQRPLRMGHDDDGGFVEKKMGKCGEKKERSDCYCVCVERSRHRRLHFVLY
SEQ ID 153 - Third frame amino acid sequence from HERV-K
   VYPTAPKRQRPWRMGHDDDGGFVEKKRGKCGEKKERSDCHCVCVERSRHRRLHFVLY
SEQ ID 154 - Third frame amino acid sequence from HERV-K
   VYPTAPKRQRPWRMGHDDDGGFVEKKRGKCGEKKERSDCYCVCVERSRHRRLHFVLY
SEQ ID 155 - Third frame amino acid sequence from HERV-K
   YPTALKRQRPSRTGHDDYGSFVKKKRGKCGEKKERSDCYCVCVERSRHSRLHFVLY
SEQ ID 156 - Third frame amino acid sequence from HERV-K
   APTRQPPCLRGVPNSSLRTGHDDDGGFVEQKRGKCREKKERSDCYCVCVERSRHRRLHFVLY
SEQ ID 157 - Third frame amino acid sequence from HERV-K
   VYPTAPKRQRPSRTGHDDDGGFVKKKRGKCGEKKERSDCYCVCVERSIHRRLHFVLY
SEQ ID 158 - Third frame amino acid sequence from HERV-K
   VYPTAPKRQRPSRTGHDDDGGFVEKKRGKCGEKQERSDCYCVCVERSRHGRLHFVLY
SEQ ID 159 - Third frame amino acid sequence from HERV-K
   VYPTAPKRQQPSRMGHDDNGGFVEKKRGKCGEKKERSDYYCVCVERSRHRRLHFVLY
SEQ ID 160 - Third frame amino acid sequence from HERV-K
   VYPTAPKRQRPSRTGHDDDGGFVEKKRGKCREKKERSDCYCVCVERSRHRRLHFVLY
SEQ ID 161 - Third frame amino acid sequence from HERV-K
   PSGRCAQQLIEKGHDDNGGLVEWRRGKCGEKRERSDCCCVCVEGGRRGRLHFVLY
SEQ ID 162 - Third frame amino acid sequence from HERV-K
   YPTAPKRQRPSRKSHDDDGGFVEKKRGKYGEKKERSDCYCVCVERSRHRRLHFVLY
SEQ ID 163 - Third frame amino acid sequence from HERV-K
   VYPTAPKRQRPSRTGHDDDGGFVQKKRGKWEKRDQ
SEQ ID 164 - Third frame amino acid sequence from HERV-K
   LQVYPAAPKRQRPLRMGDDDDGGFVKKKRGKCGEKKERSDCYCVYVEKEDIRNSILICIKKNCSALRC
SEQ ID 165 - Third frame amino acid sequence from HERV-K
   RRERPSRTSHDDNGGFVEKKGEMWGKERDIRLLLCLC
SEQ ID 166 - Third frame amino acid sequence from HERV-K
   VYPTAPKRQRPSRTGHDDDGSFVKNKRGKCGEKKERSDCCCVCVERSRHRRLHFVLY
SEQ ID 167 - Third frame amino acid sequence from HERV-K
   VYPAAPKRQRPLRTGHDDDGGFVEKKRGKCGEKKQKSDCYCVCVERDRHRRLHFVLY
SEQ ID 168 - Third frame amino acid sequence from HERV-K
   VYPTAPKRQRPSRTGHDDNGSFVEKKRGKCGEKKERSDCYCVCVERSRHRRLHFVLY
SEQ ID 169 - Third frame amino acid sequence from HERV-K
   VYPTAPKRQQPSRTGHDDDGSFVKKKRGKCGEKRDQMLLCLCRK
SEQ ID 170 - Third frame amino acid sequence from HERV-K
   VYPTAPKRQRPLRTGHDDDGGFVEKKRGKCGEKKERSDCYCVCVERSRHKRLHFVLY
SEQ ID 171 - Third frame amino acid sequence from HERV-K
   VYAAALERQRPARNGHDDDGGFVKKKRGIYREKKERSDCYCVYVER
SEQ ID 172 - Third frame amino acid sequence from HERV-K
   PPEQRPREMNGCHSGPDLRHSQEGPCGEKKEISDCYCVYVERSRRKRLHFVLY
SEQ ID 173 - Third frame amino acid sequence from HERV-K
   VYPIAPKRQRTSRTGHDDNGGFVEKKREMWGKEREIRLLLCLC
SEQ ID 174 - Third frame amino acid sequence from HERV-K
   VYPTAPKRQQPSRTGHDDDGGFVQKKRGKCGEKKERSDCYCVCVERSRHRRLHFVLH
SEQ ID 175 - Third frame amino acid sequence from HERV-K
   VYPTAPKRQRPWRTGHDDDGGFVEKKRGKCGEKKERSDCYCVCVERSRHRRLHFVLY
SEQ ID 176 - Third frame amino acid sequence from HERV-K
   VYPTAPKRQRPSRTRHDDDGSFVEKRRGKCGEKQERSDCYCVCVERSRHRRLHLVMY
SEQ ID 177 - Third frame amino acid sequence from HERV-K
   VYPTAPKRQRPSRTGQDDDGSFVEKRRGKCGEKQERSDCYCVCVERSRHRRLHFLMY
SEQ ID 178 - Third frame amino acid sequence from HERV-K
   VYPTAPKRQRPSRTGHEDDGGFVKKKRGKCGEKKERSDCYCVCVERNRHRRLHFVLY
SEQ ID 179 - Third frame amino acid sequence from HERV-K
   PPEQRPREMNGCHSGPDPRHSQEGPCGEKKEISDCYCVYVERSRRKRLHFVV
SEQ ID 180 - Third frame amino acid sequence from HERV-K
   VFTTAEQGRTPAPGTQRDFAKGMDLAGPRGCLCREKKERSHCYCVYVEKEDINSILSCTKKNYFA
SEQ ID 181 - Third frame amino acid sequence from HERV-K
   VYPAAPKRQQPARMGHSDDGGFVKKKRGGYVRKREIRLSLCLCRKGRHKKLHFDLY
SEQ ID 182 - Third frame amino acid sequence from HERV-K
   PPEQRPREMNGCHSGPDPRHSQEGPCGEKKEISDCYCVYVERSRRKRLHFVL
SEQ ID 183 - Third frame amino acid sequence from HERV-K
   VYPTAVKRQRPSRTGHDDDGGFVKKKRGKCGEKKERSDCYCVCVERSRHRRLHFL
SEQ ID 184 - Third frame amino acid sequence from HERV-K
   VYPTALKRQRPSRTGHDDDGSFVEKKRGKCGEKRDQ
SEQ ID 185 - Third frame amino acid sequence from HERV-K
   PTALKRQRPSRTGHDDDGSFVEKKRGKCGEKKERTDCYCVCVERSRHRRLHFVLY
SEQ ID 186 - Third frame amino acid sequence from HERV-K
   VYPAAPERQRPARRGHNDGGGFVKKKRGICREKKERSDCYCVYIER
SEQ ID 187 - Third frame amino acid sequence from HERV-K
   VYPTAPKRQRPLRTGHDDDGSFVEKKRRKCGEKREQ
SEQ ID 188 - Third frame amino acid sequence from HERV-K
   LYPTAPKRQRPSRTGHDDDSGFVEKKRGKCGEKQERSDCYCVCVERSRHRRLHFVMY
SEQ ID 189 - Third frame amino acid sequence from HERV-K
   DSDRPERRGHDDGGGFVKTKRGICREKKERSDCYCVYIEREDIRDSILKKTCTLNSCFDRDSCLSAFMCLLLPQ
SEQ ID 190 - Third frame amino acid sequence from HERV-K
   SPSAQRPPRLGGVPNSSLRTGHDADGGFVEWKRGKCGEKIERSDCYCVCIERSRHRRLHFVLY
SEQ ID 191 - Third frame amino acid sequence from HERV-K
   VYPTSPKRQRPSRTGHDDDGGFVEKKRGNVGKRK
SEQ ID 192 - Third frame amino acid sequence from HERV-K
   VYPTAPKRQRPLRMGHGDDGGFVEKKRGKCREKKERSDCYCVCVERSRHRRLHFVLY
SEQ I17 193 - Third frame amino acid sequence from HERV-K
   LQVYPAAQERHRPARRGHDDGGGFVKTKRGIYREKKERSDCYCVYTEREDIRDSILKKTCTLNNCFAEMLLICSFAPATLP
SEQ ID 194 - Third frame amino acid sequence from HERV-K
   VYPAATEKQRPARTGHDDDGGWKKKRGKCREKKEGSDCHCVYAER
SEQ ID 195 - Third frame amino acid sequence from HERV-K
   YPTAPKRQQPWRTGLDDLGGFFEKKRGNFGEKKGGSDFYSVCVERSRHRGPHFVLY
SEQ ID 196 - Third frame amino acid sequence from HERV-K
   VYPTAPKRQQPWRTGHDDHGGFVEKKRGKCGEKKERSDCYYVCVERSRHRRLHFVLY
SEQ ID 197 - Third frame amino acid sequence from HERV-K
   VYPTAPKRQRPSRMGHDDDGGFVEKKRGKCGEKKERSDCYCVCVESRHRRLHFVLY
SEQ ID 198 - Third frame amino acid sequence from HERV-K
   VYPTAPKRQRPSRTGHDDDGGFVEKRRGKCGEKKEIRLLLCLCRK
SEQ ID 199 - Third frame amino acid sequence from HERV-K
   VYPTAPKRQQPSRTGHDDDGSFVEKKRGKCGEKKERSDCYCVCVERSRHRRLHFVLY
SEQ ID 200 - Third frame amino acid sequence from HERV-K
   VYPAAPERQRPARMGHDDDGGFVKKKRGKCREKKERSDCHSVYVEK
SEQ ID 201 - Third frame amino acid sequence from HERV-K
   VYPTAPKRQRSSRTGRDNDGGFVKKKRGKCGEKKERSDCYCVCVGRSRHRRLHFVLY
SEQ ID 202 - Third frame amino acid sequence from HERV-K
   VYPAAPERQQPARRGHDDGGGFVKKKRGICREKKERSDSYCVYIER
SEQ ID 203 - Third frame amino acid sequence from HERV-K
   VYPAAPERQRPVRRGHDDDGGFVKKKRGKCREKREIRLSLCLCRKGRHKRLHF
SEQ ID 204 - Third frame amino acid sequence from HERV-K
   VYPTAPKRQRPSRTGHDDDGGFVEKKRGKCGEKKKRSDCYCVCVERSRCRRLRFVLY
SEQ ID 205 - Third frame amino acid sequence from HERV-K
   VYPTAPKRQRPSRTGHDDDGGFVEKKRGKCGEKKKRSDCYCVCVERSRCRRLHFVLY
SEQ ID 206 - Third frame amino acid sequence from HERV-K
   LYPAAPERQRPARRGHDDGGGFFKTKRGICREKKERSDSYRLLLCLHRKGRHKRLH
SEQ ID 207 - Third frame amino acid sequence from HERV-K
   VYPTAPKRKRPSRMGHDDNGGFVEKKRGNVGKRQ
SEQ ID 208 - Third frame amino acid sequence from HERV-K
   VYPTAPKRQQPSRTGHDDDGGFVEKKRGKRGEKKERSDCYCVCVERSRHRRLPFVLY
SEQ ID 209 - Third frame amino acid sequence from HERV-K
   VYPTAPKRQRPSRTGHDDDGGFVEKKRGKCGEKQERSNCYCVCVERSRHRRLHFVM
SEQ ID 210 - Third frame amino acid sequence from HERV-K
   VYPAASETQRPARTGHDDDGGF'VKKKRGICREKKVRSDCYCIYVER
SEQ ID 211 - Third frame amino acid sequence from HERV-K
   VYPTAPKRQRPTRTGHDDDGGFVKKKRGKCGEKKERSGCYCACVERSRHRRLHFVLY
SEQ ID 212 - Third frame amino acid sequence from HERV-K
   PTAPKRQRPSRTGHDYDGGFVEKKRGKCGEKQERSDCCCVCVERSRHRRLHFVMY
SEQ ID 213 - Third frame amino acid sequence from HERV-K
   VYPTAPKRQRPSRTGHDDDGSFVIKKRGKRGEKRDQ
SEQ ID 214 - Third frame amino acid sequence from HERV-K
   VYPTAPKRQRSSRMGHDDDGSFVEKKRGKCGEKKERSDCYCVYVERSRHRRLHFVLY
SEQ ID 215 - Third frame amino acid sequence from HERV-K
   VYPTAPKRQRPLRMGHDDDGGFVEKKRGKCGEKKERSDCYCVCVERSRHRRLHFVLY
SEQ ID 216 - Third frame amino acid sequence from HERV-K
   VYPTAPKRQRPSRMAHDDDGGFVENKSGKCGEKKERSDCYRVCVERSRHRRLHFVLY
SEQ ID 217 - Third frame amino acid sequence from HERV-K
   VYPTALKRQQPSRTGHDDDGGFVEKKRGKCGEKKERSDCYCVCVERSRHRRLHFVLY
SEQ ID 218 - Third frame amino acid sequence from HERV-K
   VYPTAPKRQRPSRTGHDDNGGFVEKKRGKCGEKKDQ
SEQ ID 219 - Third frame amino acid sequence from HERV-K
   VYPTAPKRQRPSRTGHDDDGGFVEKKRGKCGEKKERSDCYRVCVERSRHRRLHFVLY
SEQ ID 220 - Third frame amino acid sequence from HERV-K
   RRDRPWRTGHDDDGGFVEKTRGKCGEKKERSDCYCVCVERSRHRRHHFVLY
SEQ ID 221 - Third frame amino acid sequence from HERV-K
   VYLAAPKRQRPSRTSHDDNGGFVKKKRGKCGEEKERSDCYCVYVERSRHKRLHFVLY
SEQ ID 222 - Third frame amino acid sequence from HERV-K
   VYPAAPKRQRPSRTGHDDDGSFVKNKRENVGKRKRDQIVTVSMQKRK
SEQ ID 223 - Third frame amino acid sequence from HERV-K
   EVYPTAPKRQRPSRTGHDDNGSFVKKKRGKCGEKKERSDCYCVCVERRRHRRLHFVLYQEMFFCLGMLLIYNLTPNPLLSETCAV
SEQ ID 224 - Third frame amino acid sequence from HERV-K
   VYPTAPKRQRPSRTGHDERAMMTMAVLLKRKGGNAGKREIR
SEQ ID 225 - Third frame amino acid sequence from HERV-K
   VYPTAPKRQRPSRMGHDDNGSFVEKKRGKCGEKKERSDCYYVCVERSRHRRLHFVLY
SEQ ID 226 - Third frame amino acid sequence from HERV-K
   PGNPRRKLPQGQGHHCGEKQEGSDCYCVCVERSRHRRLHFVLH
SEQ ID 227 - Third frame amino acid sequence from HERV-K
   VYATALKRQRPSRTGHDDDGGFVEKKRGKCGEKKERSDCYCVCIERSRHRRHHFVLY
SEQ ID 228 - Third frame amino acid sequence from HERV-K
   VYPTSPKRQRPSRTGHDDDGGFVKKKRGKCGEKKERSDCYCVCVERSRHRRLHFVLY
SEQ ID 229 - Third frame amino acid sequence from HERV-K
   VYPTAPKRQRPSRTGHDDDGGFVEKKRGKCGENKERSDCYCVCVERSRHKRLHFVLY
SEQ ID 230 - Third frame amino acid sequence from HERV-K
   VYPTAPKRQQSSRTGHDDDGGFVEKKREKCGEKKERSDCYRVCVERSRHRRLHFVLY
SEQ ID 231 - Third frame amino acid sequence from HERV-K
   VYPTALKRQRPWRTGHDDDGGFVEKKRGKCGEKKERSDCYCVCVERSRHRRLHFVLY
SEQ ID 232 - Third frame amino acid sequence from HERV-K
   VYPTAPKRQRPSRAGHDDDRGFVEKKRGKCGEKKERSDCYCVCVERSRHRRLHFVLY
SEQ ID 233 - Third frame amino acid sequence from HERV-K
   LYPTAPKRQRPLRMGHDADGGFVEKKRGKCGEKKEIRLLLCLC
SEQ ID 234 - Third frame amino acid sequence from HERV-K
   VYPTAPKRQRPLRTGHDDDSGFVEKKRGKCGEKKERSDCYCVCVERSKHRRLHFVLY
SEQ ID 235 - Third frame amino acid sequence from HERV-K
   VYPTAPKRQRPSRAGHDDDGGFVEKKRGKCGEKEERSDLYCVCVERSRHRRLHFVLY
SEQ ID 236 - Third frame amino acid sequence from HERV-K
   VYPTAPKRQRPSRTGHDDDGSFVEKKRGKCGEKKERSDCSCVCVERSRHRRLHFVLY
SEQ ID 237 - Third frame amino acid sequence from HERV-K
   VYPTAWKRQRPSRMGHDDDGGFVEKKRGKCGENRDQ
SEQ ID 238 - Third frame amino acid sequence from HERV-K
   VYPTAPKRQQPSRTGHDDDGSFVKKKRGKCGEKKERSDCCCVCVERSRHRRLHFVLY
SEQ ID 239 - Third frame amino acid sequence from HERV-K
   VYPTAPKRQRPWRTGHDDDGGE'VKKKRGKCGEKKKRSDCYCVCVERSRHGRLRFVLY
SEQ ID 240 - Third frame amino acid sequence from HERV-K
   PAWPTWRNPVSTKNTKLARHGAACLQSCREKKERSDCYCVCVEREDIRNSILTCTLNNWLAEMLLICDFAPNLSSQ
SEQ ID 241 - Third frame amino acid sequence from HERV-K
   VYPTAPKRQRPSRTGHDDDGAFVEKKRGKCGEKKERSDCYCV
SEQ ID 242 - Third frame amino acid sequence from HERV-K
   VYPTAPKRQQPLRTGHNDDGGFVEKKRGKYGEKKERSDCYCVCVERSRHRRLHFVLY
SEQ ID 243 - Third frame amino acid sequence from HERV-K
   VYPIALKRQRPSRTGHDDDGGFVKKKRGKCGEKKERSDCYCVYVERSRHRRLHFVLY
SEQ ID 244 - Third frame amino acid sequence from HERV-K 
LQVYPAAPERQRLARTDHDDDGGFVKKKRGICREKRERSDCYCVYVEREDIRDSILKKTCTLNNCFAQMLLICSFAPATLTQPGAHKNM
CCMKSRFKGSRAVQDVPC
SEQ ID 245 - Third frame amino acid sequence from HERV-K
   VYPTARKRQRPSRTGHDDDGGFVKKKRGKCGEKKERSDCYCVCVERSRHRRLHFVL
SEQ ID 246 - Third frame amino acid sequence from HERV-K
   VYPTAPKRQRPSRTGHDDDRGFVKKKWGKMWGKKREIRLLLCLCRK
SEQ ID 247 - Third frame amino acid sequence from HERV-K
   VYPTAPKRQRPLRRGHDDDGGSVKKKRGKCGEKKERSDCYCVCVERSRHKRLHFVLY
SEQ ID 248 - Third frame amino acid sequence from HERV-K
   VYPAAPERQRPARRGHDDGGGFVKTKRGICREKKERSDSYCVYIER
SEQ ID 249 - Third frame amino acid sequence from HERV-K
   VYPAAPERQRPARRGHDDGGGFVKMKRGICREKKERSDCYCVYIEREAIR
SEQ ID 250 - Third frame amino acid sequence from HERV-K 
LQVYPAAPERQRPGRRGHDDHGGFVKKKSGKCREKRQIRLSLCLCRKGRHKRLHFEKDLYSNNCFAEMLFICSFAPATLPQSLCPNLEF
TKTCVV
SEQ ID 251 - Third frame amino acid sequence from HERV-K
   LQVYPAAPERQRPARRGHDDGGGFVKTKRGICREKKERSDCYCVYIEREAIRDSILKKTCTLNNCLLRCCLSVALPQPLCPN
SEQ ID 252 - Third frame amino acid sequence from HERV-K 
LQVYPAAPERQRPARRDHDDHGGFVKKKSGKCREKREIRLSLCLCRKGRHKRLHFEKDLYSNNCFAEMLFICSFAPATLPQSLCPNLEF
TKICVV
SEQ ID 253 - Third frame amino acid sequence from HERV-K 
LQVYPAAPERQRPARRDHDDHGGFVKKKSGKCREKREIRLSLCLCRKGRHKRLHFEKDLYSNNCFAEMLFICSFAPATLPQSLCPNLEF
TKTCVV
SEQ ID 254 - Third frame amino acid sequence from HERV-K
   LQVYPAAPERQQPAKTGHNDYGGFVKKKRGICTAKKERSDCYCVYVEREDIRNSILTCTLNNCFAEMLLICNFAPATLPQ
SEQ ID 255 - Third frame amino acid sequence from HERV-K
   LHPLSPSQLAPPQPGHPAWATPSDCHNPRAYGQDELHQVKMVECGEKQERSECHCICVERSRHGRLHFVMY
SEQ ID 256 - Third frame amino acid sequence from HERV-K
   PLCPRLKQSSRLSLSSSRDCCGEKQERSDCYCVCIERSRHRRLHFVLY
SEQ ID 257 - Third frame amino acid sequence from HERV-K
   LYPTAPKRQRPSRMGHDDDGGFVKKKRGKCGGKR
SEQ ID 258 - Third frame amino acid sequence from HERV-K
   VYPTAPKRQRPSRRGHDDDGGFVEKKRGKCEEKKERSDCYCVCVERSRHRRLHFILY
SEQ ID 259 - Third frame amino acid sequence from HERV-K
   VYPTALKRQRPSRTGHDDDGGFVEKKRGKCGEKKERSDCYCVCVERSRHRRLHFVLY
SEQ ID 260 - Third frame amino acid sequence from HERV-K
   WPAAPSGRCTQQLRTGHDDNGGFVEWKGGKGGEKIEKSDGCRVCVERGRHGRFFILF
SEQ ID 261 - Third frame amino acid sequence from HERV-K
   VYPTAPKRQQPSRMGHHDDGGFVEKKRGKCGEKKERSDCYCVCVERSRHRRLHFVLY
SEQ ID 262 - Third frame amino acid sequence from HERV-K
   VYPTAPKRQRPSRMGHDDDGGFVEKKGGNVEKRKREQIVTVSV
SEQ ID 263 - Third frame amino acid sequence from HERV-K
   VYPTAPKRQRPSRMGHDDDGSFVEKKRGKCGEKKERSDCYCVCVERSRHRRLHFVLY
SEQ ID 264 - Third frame amino acid sequence from HERV-K
   VYPTAPKRQRPSRTGHDDDGSFVEKQRGKCGEKKERSDCYCVCVERSRHRRLHFVLY
SEQ ID 265 - Third frame amino acid sequence from HERV-K
   VYPAAPKRQRPSRTGHDDDGGFVEKKRGKCGEKKERSDCYCVCVERSRHRRLHFVL
SEQ ID 266 - Third frame amino acid sequence from HERV-K
   VYPTAPKRQRPSRRGHDDDGGFVKKKRGKCGEKKERSDCYCVCVERSRHRRLHFVLY
SEQ ID 267 - Third frame amino acid sequence from HERV-K
   VYPTAPKRQRPSRMGHDDDGGFVEKKRGKCGEKKERTDCYCVYIERSRHRRLHFVLY
SEQ ID 268 - Third frame amino acid sequence from HERV-K
   VYPTAPKRQRPSRTGHDDDGGFVEKKRGKCREKKERSDCYCVCVERRRHRRLHFVLY
SEQ ID 269 - Third frame amino acid sequence from HERV-K
   VYPTALKRQRPLRTGHDDNGSFVEKKRGKCGEKKERSDCYCVCVERSRHRRLHFVLY
SEQ ID 270 - Third frame amino acid sequence from HERV-K
   VYPTAPKRQRPSRTGHDDDGSFVEKKRGKCGEKKETSDCYCVCVERSRHRRLHFVLY
SEQ ID 271 - Third frame amino acid sequence from HERV-K
   VYLTALKRQRPSRMGHDYDGSFVEKKRGKCGEKKERSDCYCVCVERSRHRRLHFVLY
SEQ ID 272 - Third frame amino acid sequence from HERV-K
   VYPTVPKRQRPSRKGHEDDGCFVKKKRGKFGEKKERSDCYCVCVERSRHRRLHFVLY
SEQ ID 273 - Third frame amino acid sequence from HERV-K
   MYPTPLKRQRPWRTGHDDNGGFVEKKRGKCGEKKERSDCYCVCVERSRHRRLHFVLY
SEQ ID 274 - Third frame amino acid sequence from HERV-K
   VYSTAPKRQRPGRMGHDDVAVLSKRKGGNVGKRKRNQIVTVSV
SEQ ID 275 - Third frame amino acid sequence from HERV-K
   VYPTAPKRQRPSRTGHDDDGGFAEKKRGKCGEKKERSDFYCVCAERSRHRRHHFVLY
SEQ ID 276 - Third frame amino acid sequence from HERV-K 
LQVYPAAPERQRPARRGHDDHGGFVKKKSGKCREKREIRLSLCLCRKGRHKRLHFEKDLYSNYCFAEMLFICSFAPATLPQSLCPNLEF
TKTCVV
SEQ ID 277 - Third frame amino acid sequence from HERV-K 
LQVYPAAPERQQPARTGHDDYGSFVKKKRDICREKKERSDCYCVYVEKKDIRDSILKKTCTLNNCFAEMLLICSFAPATLTQPGAHKNM
CCMESRLKGSRAVQDVPC
SEQ ID 278 - Third frame nucleotide sequence from HERV-K 
EQ ID 278 — Third frame nucleotide sequence from HERV-K
GTGTACCCAACAGCTCCGAAGAGACAGCAACCATCGAGAACGGGCCATGATAACGATGGCAGTTTTGTCGAAAAGAGAAGGGGAAATG
TGGGGAAAAGAAAGAGAGATCAGATTGTTACTGTGTCTGTGTAGAAAGAAGTAGACATAGGAGACTCCATTTGTTCTGTAC
SEQ ID 279 - Third frame nucleotide sequence from HERV-K 
GTGTTCCCAACAGCTCTGAAGAGACAGCGACCATCGAGAATGGCCATGATGACGATGGTGGTTTGTCGAAAAGAAAAGGGGAAATG
TGGGGAAAAGAAAGAGAGATCAGATTGTTACTGTGTCTGTGTAGAAAGAAGTAGACATAGGAGACTCCATTTTGTTCTGTAC
SEQ ID 280 - Third frame nucleotide sequence from HERV-K 
GTGTACCCAACAGCTCCGAAGAGACAGCAACCATCAAGAACTGGCCATGATGATGATGGCGGTTTTGTCAAAAAGAAAAGGGGGAAATG
TGGGGAAAAGAAAGAGAGATCAGATTGTTACTGTGTCTGTGTAGAAAGAAGTAGACATAGGATACTCCATTTTGTTCTGTAC
**SEQ ID 281 - Third frame nucleotide sequence from HERV-K** 
CTACAGGTGTATCCAGCAGCTCCAAAGAGAGAGCGACCAGTGAGAACGGGCCATGATGATGATGGCGGTTTTCTCAAAAAGAAAAGGGG
GATATGTAGGGAAAAGAAAGAGAGATCAGACGGTTACTGTGTCTATGTAGAAAAGGAAGACATAAGAAATTTCATTTTGATCTGTACCC
TGAACAATTGCTTTGCC
**SEQ ID 282 - Third frame nucleotide sequence from HERV-K** 
GTGTACCCAGCAGCTCCGAAGAGACAGCGACCATCGAGAACAAGCCATGATGATGATGGTGGTTTGTCGAAAAGGAAATGGGGAAATGT
GGGGAAAAGAGAGATCAGACTGTTACTGTGTCTGTGT
**SEQ ID 283 - Third frame nucleotide sequence from HERV-K** 
GTGTACCCAACAGCTCCGAAGAGACAGCGACCATCGAGAACGGGCCATGATGACGATGGCGGTTTTGTCGAAAAGAAAAGGGGAAATG
TGGGGAAAAGCAAGAGAGATCAGATTGTTACTGTGTCTGTGTAGAAAGAAGTAGACATGGAGACTCCATTTTGTTATG
**SEQ ID 284 - Third frame nucleotide sequence from HERV-K** 
GTGTACCTAACAGCTCCAAAGAGACAGCGACCATCGAGAACGGCCATGATGACGATGGCGGTTTTGTCAAAAAGAAAAGGGGAAATG
TGGGGAAAAGAAAGAGAGATCAGATTGTTACTGTGTCTGTGTAGAAAGAAGTAGACATAGGATACTCCATTTTGTTCTGTAC
**SEQ ID 285 - Third frame nucleotide sequence from HERV-K** 
GTGTATCCAACAGCTCTGAAGAGACAGAGACCAAAGAGAATGGGCCATGATGACTATGGCAGTTCTGTCAAAAAGAAAAGGGGATATG
TAGGGGAAAGAAAGAGAGATCAGACTGTTACTGTGTCTATGTAGAAAAG
**SEQ ID 286 - Third frame nucleotide sequence from HERV-K** 
GTGTACCCAACAGCTCCGAAGAGACAGCGACCATTGAGAACGGGCCATGATGACGATGGCGGTTTTGTCAAAAAGAAAAGGGGAAATG
TGGGGAAAAGAAAGAGAGATCAGATTGTTACTGTGTCTGTGTAGAAAGAAGTAGACATAGGAGACTCCATTTTGTTCTGTAC
**SEQ ID 287 - Third frame nucleotide sequence from HERV-K** 
GTGTACCCAACAGCACCGAAGAGACAGCAACCATCGAGAACGGGCCATGATGACGATGGCTGTTTTCTCGAAAAGAAAAGGGGGAAATG
TGGGGAAAAGAAAGAGAGATCAGATTGTTACTGTGTCTGTGTAGAAAGAAGTAGACATAGGAGACTCCATTTTGTTCTGTAC
**SEQ ID 288 - Third frame nucleotide sequence from HERV-K** 
GTGTACCGAACAGCTCTGAAGAGACAGCGACCATCGAGAATGGGCCACGATGATGATGGCAGTTTTGTCGAAAAGAAAAGGGGAAATG
TGGGGAAAAGAAAGAGAGATCAGATTGTTACTGTGTCTGTGTAGAAAGAAGTAGACATAGGAGACTCCATTTGTTCTGTAC
**SEQ ID 289 - Third frame nucleotide sequence from HERV-K** 
GTGTACCCAACAGCTCCGAAGAGACAGCGACCATCAAGAACGGGCCATGATGACGATGGTGGTTTTGTGGAAGAGAAAAGGGGAAATG
TGGGGCAAAGAAAGAGAGATCAGATTGTTACTGTGTCTGTGTAGAAAGAAGTAGACATAGGAGACTCCATTTTGTTCTGTAC
**SEQ ID 290 - Third frame nucleotide sequence from HERV-K** 
GTGTACCCAACAGCTCCAAAGAGACAGCAACCATCGAGAAACAGCCATGATGATGATGGCGGTTTTGTCGAAAAGGGGAAATGTGGGG
AAAAGAAAGATCAGATTGTTACTGTGTCTGTGTAGAAAGAAGTAGACATAGGAGACTCCATTTGTTCTGTAC
**SEQ ID 291 - Third frame nucleotide sequence from HERV-K** 
GTGTACCCAACAGCTCCGAAAAGACAGCGACCATCGAGAACGGGCCATGATGACGATGGCGGTTTTGTCGAAAAGAAAAGGAGGAAAAG
TGGGGAAAAGAGAGAGATCAGATTGTTACTGTGTCTGTGTAGAAAG
**SEQ ID 292 - Third frame nucleotide sequence from HERV-K** 
GTGTACCCAACAGCTCCAAAAAGACAGCGACCATCGAGAACGGGCCATGATGACGATGGCGGTTTTGTCAGAAAGAAAAGGGGAAATG
TGGGGAAAAGAAAGAGAGATCAGATTGTTATTGTGTCTGTGTAGAAAGAACTAGACATAGGAGATTCCATTTTGTTCTGTAC
**SEQ ID 293 - Third frame nucleotide sequence from HERV-K** 
GTGTACCCAACAGCTCCAAAGAGACAGCGACCATCGAGAACGGGCCAGTATGACGATGGCAGTTTTGTCAAAAAGAAAAGGGGAGAAA
AGAAAAGGGAGAAATGTGGGAAAAGAAAGAGAGATCAGATTGTTACTGTGTCTGTGTAGAAAG
**SEQ ID 294 - Third frame nucleotide sequence from HERV-K** 
GTGTATCCAGCAGCTCCGAAGAGACAGCGACCAGTGAGAATGGGCCATAATGACGATGTCAGTTTTGTCAAAAAGAAAAGGGGGATATG
TAGGGAAAAGAAAGAGAGATCAGACTGTTACTGTGTCTATGTAGAAAAG
**SEQ ID 295 - Third frame nucleotide sequence from HERV-K** 
GTGTATCCAACAGCTCCAAAGAGACAGCGACCATCAAGAATGGGCCATGATGACTATGGCGGTTTTGTCGAAAAGAAAAGGGGAAATG
TGGGGAAAAGAAAGAGAGATCAGATTGTTACTGTGTCTGTGTAGAAAGAAGTAGACATAGGAGACTCCATTTTGTTCTGTAC
**SEQ ID 296 - Third frame nucleotide sequence from HERV-K** 
GTGTACCCAACAGCTCCAAAGAGACAGCAACCATCGAGAATGGGCCATGATGACGATGGCGGTTTTGTCAAAAAGAAAAGGGGAAATG
TGGGGAAAAGAAAGAGAGATCAGATTGTTACTTTGTCTGTGTAGAAAGAAGTAGACATAGGAGACTCCATTTTGTTCTGTAC
**SEQ ID 297 - Third frame nucleotide sequence from HERV-K** 
GTGTACCCAACAGCTCCGAAGAGACAGCGACCATCGAGAACGGGCCATGATGACGATGGCGGTTTTGTCGAAAAGAAAAGGGGAAATG
TGGGGAAAAGCAAGAGAGATCAGATTGTTACTGTGTCTGTGTAGAAAGAAGTAGACATAGGAGACTCCATTTTGTTATGTAC
**SEQ ID 298 - Third frame nucleotide sequence from HERV-K** 
GTGTACCCAACAGCTCCGAAGAGACAGCGACCATCGAGAACGGGCCATGATGACGATGGCGGTTTTGTCGAAAAGAAAAGGGGAAATG
TGGGGAAAAGCAAGAGAGATCAGATTGTCACTGTGTCTGTGTAGAAAGAAGTAGACATGGAGACTCCATTTTGTTATGTAC
**SEQ ID 299 - Third frame nucleotide sequence from HERV-K** 
GTGTACCCAACAGCTCCGAAGAGACAGCGACCATCGAGAACGGGCCATGATGACAGTGGCGGTTTTGTCGAAAAGAAAAGGGGAAATG
TGGGGAAAAGAAAGAGAGATCAGATTGTTACTGTGTCTGTGTAGAAAGAAGTAGACATAGGAGACTCCATTTT
**SEQ ID 300 - Third frame nucleotide sequence from HERV-K** 
GTGTACCCAACAGCTCCGAAGAGACAGCGACCATCGAGAATGGGCCATGATGACGATGGTGGTTTGTTGAAAAGAAAAGGGGAAATG
TGGGGAAAAGAAAGAGAGATCAGATTGTTACTGTGTCTGTGTAGAAAGAAGTAGACATAGGAGACTCCATTTTGTTCTGTAC
**SEQ ID 301 - Third frame nucleotide sequence from HERV-K** 
GTGTACCCAACAGCTCGGAAGAGACAGCAACCATCAAGAACGGGCCATGATGATGATGGTGGTTTTGTCGTAAAGAAAAGGGGAAATG
TGGGGAAAAGAAAGAGAGATCAGATTGTTACTGTGTCTGTGTAGAAAGAAGTAGACATAGGAGACTCCATTTTGTTCTGTAC
**SEQ ID 302 - Third frame nucleotide sequence from HERV-K** 
GTGTACCCAACAGCTCCAAAGAGACAGCGACCATCAAGAACGGGCCATGATGACGATGGCGGTTTTGTCGAAAAGAAAAGGGGAAATG
TGGGGAAAAGAAAGAGAGATCAGATTGTTACTGTGTCTGTGTAGAAAGAAGTAGACATAGGAGACTCCATTTTGTTCTGTAC
**SEQ ID 303 - Third frame nucleotide sequence from HERV-K** 
GTGTACCCAACAGCTCCGAAGAGACAGCGACCATCAAGAACAGGCCATGATGACGATGGTGGTTTTGTCGAAAAAAAAGAAAAGGGGGA
AATGTGGGGAAAAGAAAGAGAGATCAGATTGTTACTGTGTCTGTGT
**SEQ ID 304 - Third frame nucleotide sequence from HERV-K** 
GTGTACCCAACAGCTCCGAAGAGACAGCGACCATCGAGAACAGGCCATGATGATGATGGCGGTTTTGTCGAAAAGCAAAGGGGAAATG
TGGGGAAAAGAAAGAGAGATCAGATTGTTACTGTGTCTGTGTAGAAAGAAGTAGACATAGGAGACTCCATTTTGTTCTGTAC
**SEQ ID 305 - Third frame nucleotide sequence from HERV-K** 
GTGTACCCAACAGCTCCGAAGAGACAGCAACCATCGAGAACGGGCCATGATGAGGATGGTGGTTTGTTGAAAGGAAAAGGGGAAATTG
TGGGGAAAAGAAAGAGAGATCAGATTGTTACTGTGTCTGTGTAGAAAGAAGTAGACATAGGAGACTCCATTTGCTCTGTAC
**SEQ ID 306 - Third frame nucleotide sequence from HERV-K** 
GTGTACCCAACAGCTCCGAAGAGACAGCGACCATCGAGAACGGGCCATGATGATGATGGCGGTTTTGTCGAAAAGAAAAGGGGAAATG
TGGGGGAAAGAATGAGAGATCAGATTGTTACTGTGTCTGTGTAGAAAGAAGTAGACATAGGAGACTCCATTTTGTTCTGTAC
**SEQ ID 307 - Third frame nucleotide sequence from HERV-K** 
GTGTACCCAACAGCTCCGAAGAGACAGCGACCATCGAGAATGGGCCATGATGACGATGGCGGTTTTGTCGAAAAGAAAAGGGGAAATG
TGGGGAAAAGCAAGAGAGATCAGATTGTTACTGTGTCTGTATAGAAAGAAGTAGACATAGGAGACTCCATTTTGTTCTGTAC
**SEQ ID 308 - Third frame nucleotide sequence from HERV-K** 
CTACAGGTGTATCCAACAGCTCCAAAGAGGCAGCAACCAGCGAGAACGGGCCATAATGACGATGGCAGTTTTGTCAAAAAGAAAAGGGG
GATATGTAGGGAAAAGAAAGAGATATCAGACTGTTACTGTATCTTTGTAGAAAAGGAAGACATAAGAAACTCCATTTTGACCTGTACCG
TGAACAATTGTTTTGCC
**SEQ ID 309 - Third frame nucleotide sequence from HERV-K** 
GTGTACCCAGCAGCTCCGAAGAGACAGCGACCGTCAAGAACGAGCCATGATGATGATGGCAGTTTTGTCAAAAAGAAAAGGGTTATGTG
GGGAAAAGAGAGATCAGACTGTTACTGTGTCTATGTAGAAAGAAGTAGACATAAGAGACTCCATTTGTTCTGTAC
**SEQ ID 310 - Third frame nucleotide sequence from HERV-K** 
CTACAGGTGTATCCAGCAGCTCCAGAGAGACAGCGACCAGGGAGAAGGGGCCATGATGATGGTGGTGGTTTTGTCAAAACGAAAAGGGG
GATATGTAGGGGAAAGAAAGAGAGATCAGACTGTTACTGTGTCTACATAGAAAGGGAAGACATAAGAGACTCCATTTTGAAAAAGATCT
GTACTTTAAGCAATTGCTTTGCTGAGATGTTGTTAATCTGTAGCTTTGCCCCAGCCACTTTGCCCCAACCA
**SEQ ID 311 - Third frame nucleotide sequence from HERV-K** 
CCAAAGAGACAGCGACCATCAAGAACTGGCCATGATGACAATGGTGGTTTTGTCGAAAAGAAAAGGGGAAATGTGGGAAAAGAAAGA
GAGATCAGATTGTTACTGTGTCTGTGTAGAAAGAAGTAGACATAGGAGACTCCACTTTGTTCTGTACTAAGAAAAATTCTTC
**SEQ ID 312 - Third frame nucleotide sequence from HERV-K** 
CTACAGGTGTATCCAGCAGCTCCAGAGAGACAGCGACCAGGGAGAAGGGGCCATGATGACGGTGGTGGTTTTGTCAAAACGAAAAGGGG
GATATGTAGGGGAAAGAAAGAGAGATCAGACTGTTACTGTGTCTACATAGAAAGGGAAGACATAAGAGACTCCATTTTGAAAAAGAACT
GTACTTTAAACAATTGCTTTGCTGAGATGTTTTTAATCTGTAGCTTTGCCCCAGCCACTTTTCCCCAACCA
**SEQ ID 313 - Third frame nucleotide sequence from HERV-K** 
GTGTACCCAACAGCTCCGAAGAGACAGCGACCATCGAGAACGGGCCATGATGACGATGGCGGTTTTGTCGAAAAGAAAAGGGGAAATG
TGGGGAAAAGAGAGATCAG
**SEQ ID 314 - Third frame nucleotide sequence from HERV-K** 
GTGTACCCAACAGCTCCAAAGAGACAGCGACCATCGAGAACGGCCATGATGACAGTGGCAGTTTTGTCAAAAAGAAAAGGGGAAATG
TGGGGAAAAGAAAGAGAGGTCAGATTGTTACTGTGTCTGTGTAGAAAGAAGTAGACATAGGAGACTCCGTTTTGTTCTGTAC
**SEQ ID 315 - Third frame nucleotide sequence from HERV-K** 
GTGTACCCAACAGCTCCAAAGAGACAGCGACCATCGAGAATGGGCCATGATGACGATGGCGGTTTTGTCGAAAAGCAAAGGGGGAAATG
CAGGGAAAAGAAAGAGAGATCAGATTGTTACTGTGTCTGTGTAGAAAGAAGTTGGCATAGGAGACTCCATTTTGTTCTGTAC
**SEQ ID 316 - Third frame nucleotide sequence from HERV-K** 
GTGTACCCAACAGCTCTGAAGAGACAGCAACCATCGAGAACGGGCCATGATGACGATGGCGGTTTTGTCAAAAAGAAAAGGGGAAATG
TGGGGAAAAGCAAGAGAGATCAGATTGTCACTGTGTCTGTGTAGAAAGAAGTAGACATAGGAGACTCCATTTTGTTATGTAC
**SEQ ID 317 - Third frame nucleotide sequence from HERV-K** 
GTGTACCCAACAGCTCGGAAGAGACAGAGACCATCGAGAACGGGCCATGATGACGATGGCGGTTTTGTCGAAAAGAAAAGGGGAAATG
TGGGGAAAAGCAAGAGAGATCAGATTGTTACTGTGTCTGTGTAGAAAGAAGTAGACATAGGAGACTCCATTTTGTTCTGTAC
**SEQ ID 318 - Third frame nucleotide sequence from HERV-K** 
AAACCAAGGAGAACAAAGACACAACATACCAGAATCTCTGGACACATTCAACGTGTGGGGAAAAGCAAGAGAGATCAGATTGTTACTG
TGTCTGTGTAGAAAGAAGTAGACATAGGAGACTCCATTTTGTTCTGTAC
**SEQ ID 319 - Third frame nucleotide sequence from HERV-K** 
GTGTATCCAGCAGCTCCAGAGAGACAGCGACCAGCGAGAAGGGGCCATGATGATGGTGGTGGTTTTGTCAAAACGAAAAGGGGGATATG
TAGGGTAAAGAAAGAGAGATCAGACTGTTACTGTGTCTACATAGAAAGG
**SEQ ID 320 - Third frame nucleotide sequence from HERV-K** 
CTACAGGTGTATCCAGCAGCTCCAGAGAGACAGCGACCAGCGAGAAGGGGCCATGATGATGGAGGTGGTTTGTCAAAACGAAAATGGG
GATATGTAGGGAAAAGAAAGAGAGATCAGACTGTTACTGTGTCTACATAGAAAGGAAGACATAAGAGACTCCATTTGAAAAAGACCT
GTACTTTAAACAATTGCTTTGCTGAGATGTTGTTAATCTGTAGCTTTGCCCCAGCCACTTTGCCCCAACCA
**SEQ ID 321 - Third frame nucleotide sequence from HERV-K** 
GTGTACCCAACAGCTCCGAAGAGACAGCGACCATCGAGAAAGGGCCATGATGACGATGGCGGTTTTGTCGAAAAGAAAAGGGGAAATG
TGGGGAAAAGAAAGAGAGATCAGATTGTTACTGTGTCTGTGTAGAAAGAAGCAGACATAGGAGACACCATTTTGTTCTGTAC
**SEQ ID 322 - Third frame nucleotide sequence from HERV-K** 
TATTAGTCTACAGGTGTATACAACAGCTCCGGAGAGACAGCGACCAGCGAGAACGGGTCATGATGACGATGGCGGTTTTGTCAAAAAGA
AAAGGGGGAAATGTAGGGAAAAGAAAGAGAGATCAGACTGTCACTGTGCCTATGTAGAAAGGGAAGACATAAGAGACTCCATTTTGAAA
AAGACCTGTACTTTAAACAATTGCTTTGCTGAGATGTTGTTAATTTG
**SEQ ID 323 - Third frame nucleotide sequence from HERV-K** 
GTGTACCCAACAGCTCCAAAGAGACAGCGACCATCGAGAACGGGCCATGATGACGATGGTGGTTTTGTCGAAAAGAAAAGGAGAAATG
TGGGGAAAAGAAAGAGAGATCAAATTGTTACTGTGTCTGTGTAGAAAGAAGCAGACATAGGAGACTCCATTTTGTTCTGTAC
**SEQ ID 324 - Third frame nucleotide sequence from HERV-K** 
CTGCAGGTGTACCCAACAGCTCTGAAGAGACAGCAACCATCGAGAACGGGCCATGATGACGATGGCAGTTTTGTCGAAAAGAAAAGGGG
GAAATGTGGGGAAAAGAAAGAGAGATCAGATTGTTACTGTGTCTGTGTAGAAAGAAGTAGACATAGGAGATTCCAAAAAAAAAAA
**SEQ ID 325 - Third frame nucleotide sequence from HERV-K** 
GTGTACCCAACAGCTCTGAAGAGACAGCGACCATCGAGAACGGGCCATGATGACGATGGCGGTTTTGTCGAAAAGAAAAGGAGAAATG
TGGGGAAAAGAAAGATCAGATTGTTACTGTGTCTGTAGAAAGAAGTAGACATAGGAGACTCCATTTTGTTCTGTAC
**SEQ ID 326 - Third frame nucleotide sequence from HERV-K** 
GTGTACCCAACAGCTCCGAAGAGACAGCCACCATCAAGAACGGGCCATGATGACGATGGCGGTTTTGTCCTAAAGAAAAGGGGAAATG
TGGGGAAAAGAAAGAGAGATCAGATTGTTACCATGTCTGTGTAGAAAGAAGTAGACATAGGAGACACCATTTTGTTCTGTAC
**SEQ ID 327 - Third frame nucleotide sequence from HERV-K** 
GTGTACCCAACAGCTCCGAAGAGACAGCGACCGTCGAGAACAGGCCATGATGACGATGGCGGTTTTGTTGAAAAGAAAAGGGGAAATG
TGGGGAAAAGAAAGAGAGATCAGATTGTTACTGTGTCTGTCTAGAAAGAAGTAGACATAGGAGACTCCATTTTGTTCTG
**SEQ ID 328 - Third frame nucleotide sequence from HERV-K** 
GTGTACTCAACAGCTCCGAAGAGACAGCGACCATCGAGAACGGGCCATGATGACGATGGCGGTTTTGTTGAAAAGAAAAGGGGAAATG
TGGGGAAAAGAAAGAGAGATCAGATTGTTACTGTGTCTGTGTAGAAAGAAGTAGACATAGCAGACTCCATTTTGTTCTGTAC
**SEQ ID 329 - Third frame nucleotide sequence from HERV-K** 
GTGTACCCAACAGCTCCGAAGAGACAGCGACCATCAAGAACGGGCCATGATGACAATGGCAGTTTTGTCGAAAAGAAAAAGGGAAATG
TGGGGAAAAGAAAGAGAGATCAGATTGTTACTGTGTCTGTGTAGAAAGAAGTAGACATAGGAGACTCCATTTTGTTCTGTAC
**SEQ ID 330 - Third frame nucleotide sequence from HERV-K** 
GTGTACCCAACAGCTCCGAAGAGACAGCAACCATCGAGAACGGGCCATGATGACGATGGCGGTTTTGTCAAAAAGAAAAGGGAAAATG
TGGGGAAAAGAAAGAGAGATCAGATTGTTACTGTGTCTGTGTAGAAAGAAGTAGACATAGGAGACTCCATTTTGTTCTGTAC
**SEQ ID 331 - Third frame nucleotide sequence from HERV-K** 
GTGTACCCAACAGCTTCGAAGAGACAGCCACCATCGGGAACGGACCATGATGACGATGGCGGTTTTGTCAAAAAGAAAAGAGGGAAATG
TGGGGAAAAGAAAGAGAGATCAGATTGTTACTGTGTCTGTGTAGAAAGAAGTAGACATAGGAGACTCCATTTTCTTCTGTAC
**SEQ ID 332 - Third frame nucleotide sequence from HERV-K** 
CCATCCGAGCAAAGGCCCAGGGAAACGAATGGGTGTCATTCTGGTCCTGACCCGAGGCACAGCCAGGAAGGTCCCTGTGGGGAAAAGAA
AGAGATATCAGACTGTTACTGTGTCTATGTAGAAAGAAGTAGACATAAGAGACTCCATTTGTTGTG
**SEQ ID 333 - Third frame nucleotide sequence from HERV-K** 
GTGTACCCAACAGCTCCGAAGAGACAGCGACCATCGAGAACGGGCAGTGATGACGATGGCGGTTTTGTAGAAAAGAAAAGGGGGAAATG
TGGGGAAAAGAAAGAGAGAACAGATTGTTACTGTGTCTGTGTAGAAAGAAGTAGACATAGGAGACTCCATTTTGTTCTGTAC
**SEQ ID 334 - Third frame nucleotide sequence from HERV-K** 
GTGTACCCAACAGCTCCGAAGAAACAGCAACCATCGATAATGGGCCATGATGACGATGGCGGTTTTGTCAAAAAGAAAAGGGGGAAATG
TGGGGAAAAGAGAGATCAG
**SEQ ID 335 - Third frame nucleotide sequence from HERV-K** 
GTGTACCCAACAGCTCCAAAGAGACAGCGACCATCGAGAACAGGCCATGATGATGATGGTGGTTTTGTCAAAAAGAAAAGGGGAAATG
TGGGGAAAAGAAAGAGAAATCAGATTGTTACTGTGTCTGTGTAGAAAGAAGTAGACATAGGAGACTCCATTTTGTTCTGTAC
**SEQ ID 336 - Third frame nucleotide sequence from HERV-K** 
AGCCCCTCTGCCCAGAGGCCACCCCGTCTGGGAGGTGTACCCAACAGCTCATTGAGAACAGGCCATGATGACGATGGCGGTTTTGTCGA
ATGGAGAGGGGGGAAATGTGGGGAAAAGATAGATAAATCAGATTGTTGCTGTGTCTGTGTAGAGGGAAGTAGACGTAGGAGACTCCATT
TTGTTCTGTAC
**SEQ ID 337 - Third frame nucleotide sequence from HERV-K** 
GTGTACCCAACAGCTCCGAAGAGACAGCGACCATCGAGAACGGGCCATGATGACGATGGCGGTTTTGTCAAAAAGAAAAGGGGAAATG
TGGGGAAAAGAAAGAGAGATCAGATTGTTACTGTGTCTGTGTAGAAAGAAGTAGACATAGGAGACTCCATTTTGTTCTGT
**SEQ ID 338 - Third frame nucleotide sequence from HERV-K** 
GTGTACCCAACAGCTCCGAAGAGACAGCGACCATCGAGAACGGGCCATGATGACGATGGCGGTTTTGTGGAAAAGAAAATGGGGAAATT
TGGGGAAAAGAAAGAGAGATCAGATTGTTACTGTGTCTGTGTAGAAAGAAGTAGACATAGGAGACTCCATTTGTTCTGTAC
**SEQ ID 339 - Third frame nucleotide sequence from HERV-K** 
GTGTACCCAACAGCTCCGAAGAGACAGCGACCATTGAGAATGGGCCATGATGACGATGGCAGTTTTGTCAAAAAGAAAAGGGGAAATG
- TGGGGAAAAGAAAGAGAGATCAGATTGTTACTGTGTCTGTGTAGAAAGAAGTAGACATAGGAGACTCCATTTTGTTCTGTAC
**SEQ ID 340 - Third frame nucleotide sequence from HERV-K** 
GTGTACCCAACAGCTCCGAAGAGACAGCGACTATCGAGAACGGGCCATGATGACGATGGCGGTTTTGTCGAAAAGAAAAGGGGAAATG
TGGGGAAAAGAAAGATCAG
**SEQ ID 341 - Third frame nucleotide sequence from HERV-K** 
GTGTACCCAACAGCTCCGAAGAGACAGCAACCATCGAGAACGGGCCATGATGACGATGGCGGTTTTGTCAAAAACAAAAGGGGGAAACG
TGGGGAAAAGAAAGAGAGATCAGATTGTTACTGTGTCTGTGTAGAAAGAAGTAGACATAGGAGACACCCTTTTGTTCTGTAC
**SEQ ID 342 - Third frame nucleotide sequence from HERV-K** 
GTGTACCCAACAGCTCTGAAGAGACAGCGACCATCGAGAACGGGCCATGACAACGATGGCGGTTTTGTGGAAAAGAAAAGGGGAAATG
TGGGGAAAAGCAAGAGAGATCAGATTGTCACTGTGTCTGTGTAGAAAGAAGTAGACATAGGAGACTCCATTTTGTTATGTAC
**SEQ ID 343 - Third frame nucleotide sequence from HERV-K** 
GTGCACCCAACAGCTCCGAAGAGACAGCGACCATCAAGAACGGGCCATGATGACAATGGCAGTTTTGTCGAAAAGAAAAGGGGAAATG
TGGGGAAAAGAAAGAGAGATCAGATTGTTACTGTGTCTGTGTAGAAAGAAGTAGACATAGGAGACTCCATTTTGTTCTGTAC
**SEQ ID 344 - Third frame nucleotide sequence from HERV-K** 
AGGTGTACCCAATAGCTCCGAAGAGACAGCGACCATCGAGAACGGGCCATGATGACGATGGCGGTTTTGTCGAAAAGAAAAGGGGGAAA
TGTGGGGAAAAGAAAGAGAGATCAGATTGTTACTGTGTCTGTGTAGAAAGAAGTAGATATAGGAGACTCCATTTGTTCTGTACTTAGA
AAAATTCTTCTGCCTTGGAATGCTGTTAATCTATAACCTTACCCCCAACCCTGTGCTCTCTGAAACATGTGCTGTGT
**SEQ ID 345 - Third frame nucleotide sequence from HERV-K** 
GTGTACCCAACAGCTCCGAAGAGACAGCGACCATTGAGAATGGGCCATGATGACGATGGCGGTTTTGTCAAAAAGAAAAGGGGGAAATG
TGGGGAAAAGAAAGAGAGATCAGATTGTTACTGTGTCTGTGTAGAAAGAAGTAGACATAGGAGACTCCATTTTGTTCTGTAC
**SEQ ID 346 - Third frame nucleotide sequence from HERV-K** 
GTGTACCCAACAGCTCCGAAGAGACAGCGACCATCGAGAACGGGCCATGATGACGATGGTGGTTTTGTCGAAAAGAAAAGGGGGAAATG
TGGGGGAAAGAAAGAGAGATCAGATTGTTCCTGTGTCTGTGTAGAAAGAAGTAGACATAGGAGACTCCATTTTGTTCTGTAC
**SEQ ID 347 - Third frame nucleotide sequence from HERV-K** 
GTGTACCCAACAGCTCTGAAGAGACAGCGACCATCAAGAACGGGCCATGATGACGATGGCGGTTTTGTAGAAAAGAAAAGGAGGAAATG
TGGAGAAAAGAAAGAGAGAACAGATTGTTACTGTGTCTGTGTAGAAAGAAGTAGACATAGGGGACTCCATTTTGTTCTGTAC
**SEQ ID 348 - Third frame nucleotide sequence from HERV-K** 
GTGTACCCAACAGCTCCGAAGAGACAGCGACCATCAAGAACAGGCCATGATGACGATGGTGGTTTTGTAGAAAAGAAAAGGAGGAAATG
TGGAGAAAAGAAAGAGAGAACAGATTGTTACTGTGTCTGTGTAGAAAGAAGTAGACATAGGGGACTCCATTTTGTTCTGT
**SEQ ID 349 - Third frame nucleotide sequence from HERV-K** 
GAGGCGCTCCCCAAATCCCAGACAGGGCGGCCGGGCAGAGGCACTCCTCACTTCCTAGATGGGGTGGTGGCCAGGCAGAGGCACTCCTC
ACTTCCCAGATGGGGCGGCTGGACAGAGGCGCTCCCCACTTCCCAGACGGGGCAGCCGGGCAGAGGCACTCCTCACTTCCTCCCAGATG
CAGGGCAGCCAGGCAGAGGCGCTCCTCACCTCCCAGATGGGGCGGCCGGGCAGAGGCACTCCTCACTTCCC
**SEQ ID 350 - Third frame nucleotide sequence from HERV-K** 
GTGTACCCAACAGCTTCGAAGAGACAGCCACCATCGGAACGGACCATGATGATGATGGCAGTTTTGTCAAAAAGAAAAGAGGGAAATG
TGGGGAAAAGGAAGAGAGATCAGATTGTTACTGTGTCTGTGTAGAAAGAAGTAGACATAGGAGACTCCATTTCCTTCTGTAC
**SEQ ID 351 - Third frame nucleotide sequence from HERV-K** 
GTGTACCCAACAGCTCCGAAGAGACAGCGACCATCAAGAACGGGCCATGATGACGATGGCGGTTTTGTTGAAAAGAAAAGGGGGAAATG
TGGGGAAAAGAAAGAGAGATCAGATTGTTACTGTGTCTGTGTAGAAAGAAGTAGACATAGGAGACACCATTTTGTTCTGTAC
**SEQ ID 352 - Third frame nucleotide sequence from HERV-K** 
GTGTACCCAACAGCTCCGAAGAGACAGCGACCATTGAGAATGGGCCATGATGACGATGGCGGTTTTGTCGAAAAGAAAATGGGGAAATG
TGGGGAAAAGAAAGAGAGATCAGATTGTTACTGTGTCTGTGTAGAAAGAAGTAGACATAGGAGACTCCATTTGTTCTGTAC
**SEQ ID 353 - Third frame nucleotide sequence from HERV-K** 
GTGTACCCAACAGCTCCGAAGAGACAGCGACCATGGAGAATGGGCCATGATGACGATGGCGGTTTTGTTGAAAAGAAAAGGGGGAAATG
TGGGGAAAAGAAAGAGAGATCAGATTGTCACTGTGTCTGTGTAGAAAGAAGTAGACATAGGAGACTCCATTTGTTCTGT
**SEQ ID 354 - Third frame nucleotide sequence from HERV-K** 
ACCCAACAGCTCCGAAGAGACAGCGACCATGGAGAATGGGCCATGATGACGATGGCGGTTTTGTTGAAAAGAAAAGGGGAAATGTGGG
GAAAAGAAAGAGAGATCAGATTGTCACTGTGTCTGTGTAGAAAGAAGTAGACATAGGAGACTCCATTTTGTTCTGTACTAAG
**SEQ ID 355 - Third frame nucleotide sequence from HERV-K** 
TACCCAACAGCTCTGAAGAGACAGCGACCATCGAGAACGGGCCATGATGACTATGGCAGTTTTGTCAAAAAGAAAAGGGGGAAATGTGG
GGAAAAGAAAGAGAGATCAGATTGTTACTGTGTCTGTGTAGAAAGAAGTAGACATAGCAGACTCCATTTTGTTCTGTA
**SEQ ID 356 - Third frame nucleotide sequence from HERV-K** 
GCCCCCACCCGGCAGCCACCTTGTCTCAGAGGGGTACCCAACAGCTCACTGAGAACGGGCCATGATGACGATGGCGGTTTTGTCGAACA
GAAAAGGGGGAAATGTCGGGAAAAGAAAGAGAGATCAGATTGTTACTGTGTCTGTGTAGAAAGAAGTAGACATAGGAGACTCCATTTTG
TTCTGTAC
**SEQ ID 357 - Third frame nucleotide sequence from HERV-K** 
GTGTACCCAACAGCTCCGAAGAGACAGCGACCTTCGAGAACGGGCCATGATGACGATGGCGGTTTTGTCAAAAAGAAAAGGGGAAATG
TGGGGAAAAGAAGGAGAGATCAGATTGTTACTGTGTCTGTGTAGAAAGAAGTATACATAGGAGACTCCATTTTGTTCTGTAC
**SEQ ID 358 - Third frame nucleotide sequence from HERV-K** 
GTGTACCCAACAGCTCCGAAGAGACAGCGACCATCGAGAACGGGCCATGATGACGATGGCGGTTTTGTCGAAAAGAAAAGGGGAAATG
TGGGGAAAAGCAAGAGAGATCAGATTGTTACTGTGTCTGTGTAGAAAGAAGTAGACATGGAGACTCCATTTTGTTCTGTAC
**SEQ ID 359 - Third frame nucleotide sequence from HERV-K** 
GTGTACCCAACAGCTCCGAAGAGACAGCAACCATCGAGAATGGGCCATGATGACAATGGCGGTTTTGTTGAAAAGAAAAGGGGAAGTG
TGGGGAAAAGAAAGAGAGATCAGATTATTACTGTGTCTGTGTAGAAAGAAGTAGACATAGGAGACTCCATTTTGTTCTGTAC
**SEQ ID 360 - Third frame nucleotide sequence from HERV-K** 
GTGTACCCAACAGCTCCGAAGAGACAGCGACCATCCAGAACGGGCCATGATGACGATGGTGGTTTTGTCGAAAAGAAAAGGGGAAATG
TAGGGAAAAGAAAGAGAGATCAGATTGCTACTGTGTCTGTGTAGAAAGAAGTAGACATAGGAGACTCCATTTTGTTCTGTAC
**SEQ ID 361 - Third frame nucleotide sequence from HERV-K** 
CCATCTGGGAGGTGTGCCCAACAGCTCATTGAGAAGGGCCATGATGACAATGGCGGTTTGGTTGAATGGAGAAGGGGAAGTGTGGGGA
AAAGAGGGAGAGATCGGATTGTTGTTGTGTCTGTGTAGAGGGAGGCAGACGTGGGAGACTCCATTTTGTTCTGTAC
**SEQ ID 362 - Third frame nucleotide sequence from HERV-K** 
TACCCAACAGCTCCGAAGAGACAGCGACCATCGAGAAAGAGCCATGATGACGATGGCGGTTTTGTTGAAAAGAAAAGGGGAAATATGG
GGAAAAGAAAGAGAGATCAGATTGTTACTGTGTCTGTGTAGAAAGAAGTAGACACAGGAGACTCCATTTTGTTCTGTAC
**SEQ ID 363 - Third frame nucleotide sequence from HERV-K** 
TGTACCCAACAGCTCCAAAGAGACAGCGACCATCGAGAACGGGCCATGATGACGATGGCGGTTTTGTCCAAAAGAAAAGGGGAAATGG
GAAAAGAGAGATCAGA
**SEQ ID 364 - Third frame nucleotide sequence from HERV-K** 
CTGCAGGTGTACCCAGCAGCTCCGAAGAGACAGCGACCATTGAGAATGGGTGATGACGACGATGGTGGTTTTGTCAAAAAGAAAAGGGG
GAAATGTGGGGAAAAGAAAGAGAGATCAGACTGTTACTGTGTCTATGTAGAAAAGGAAGACATAAGAAACTCCATTTTGATCTGTATTA
AGAAAAATTGTTCTGCTTTGCGATGC
**SEQ ID 365 - Third frame nucleotide sequence from HERV-K** 
CGAAGAGAGCGACCATCGAGAACGAGCCATGATGACAACGGTGGTTTGTCGAAAAGAAGGGGAAATGTGGGAAAAGAAAGAGATAT
CAGACTGTTACTGTGTCTATGT
**SEQ ID 366 - Third frame nucleotide sequence from HERV-K** 
GTGTACCCAACAGCTCCGAAGAGACAGCGACCATCGAGAACGGGCCATGATGACGATGGCAGTTTTGTCAAAAACAAAAGGGGGAAATG
TGGGGAAAAGAAAGAGAGATCAGATTGTTGTTGTGTCTGTGTAGAAAGAAGTAGACATAGGAGACTCCATTTTGTTCTGTAC
**SEQ ID 367 - Third frame nucleotide sequence from HERV-K** 
GTGTACCCAGCAGCTCCGAAGAGACAGCGACCACTGAGAACAGGCCATGACGACGATGGCGGTTTTGTCGAAAAGAAAAGGGGAAATG
TGGGGAAAAGAAACAGAAATCAGATTGTTACTGTGTCTGTGTAGAAAGAGATAGACATAGGAGACTCCATTTGTTCTGTAC
**SEQ ID 368 - Third frame nucleotide sequence from HERV-K** 
GTGTACCCAACAGCTCCAAAGAGACAGCGACCATCGAGAACGGGCCATGATGACAATGGCAGTTTTGTTGAAAAGAAAAGGGGAAATG
TGGGGAAAAGAAAGAGAGATCAGATTGCTACTGTGTCTGTGTAGAAAGAAGTAGACATAGGAGACTCCATTTTGTTCTGTAC
**SEQ ID 369 - Third frame nucleotide sequence from HERV-K** 
GTGTACCCAACAGCTCCGAAGAGACAGCAACCATCAAGAACGGGCCATGATGACGATGGCAGTTTTGTCAAAAAGAAAAGGGGCAAATG
TGGGGAAAAGAGAGATCAGATGTTACTGTGTCTGTGTAGAAAG
**SEQ ID 370 - Third frame nucleotide sequence from HERV-K** 
GTGTACCCAACAGCTCCGAAAAGACAGCGACCATTGAGAACAGGCCATGATGACGATGGCGGTTTTGTCGAAAAGAAAAGGGGGAAATG
TGGGGAAAAGAAAGAGAGATCAGATTGTTACTGTGTCTGTGTAGAAAGAAGTAGACATAAGAGACTCCATTTTGTTCTGTAT
**SEQ ID 371 - Third frame nucleotide sequence from HERV-K** 
GTGTATGCAGCAGCTCTGGAGAGACAGCGACCAGCGAGAAACGGCCATGATGATGATGGCGGTTTTGTCAAAAAGAAAAGGGGGATATA
TAGGGAAAAGAAAGAGAGATCAGACTGTTATTGTGTCTATGTAGAAAGG
**SEQ ID 372 - Third frame nucleotide sequence from HERV-K** 
CCGCCTGAGCAAAGGCCCAGGGAAATGAATGGGTGTCATTCTGGTCCTGACCTGAGGCACAGCCAGGAAGGTCCCTGTGGGAAAAGAA
AGAGATATCAGACTGTTACTGTGTCTATGTAGAAAGAAGTAGACGTAAGAGGCTCCATTTGTTCTGTAC
**SEQ ID 373 - Third frame nucleotide sequence from HERV-K** 
GTGTACCCAATAGCTCCGAAGAGACAGCGAACATCAAGAACGGGCCATGATGACAATGGCGGTTTTGTCGAAAAGAAAAGGGAAATGTG
GGGAAAAGAAAGAGAGATCAGATTGTTACTGTGTCTGTGT
**SEQ ID 374 - Third frame nucleotide sequence from HERV-K** 
GTGTACCCAACAGCTCCAAAGAGACAGCAACCATCGAGAACGGGCCATGATGACGATGGCGGATTTGTTCAAAAGAAAAGGGGAAATG
TGGGGAAAAGAAAGAGCGATCAGATTGTTACTGTGTCTGTGTAGAAAGAAGTAGACATAGGAGACTCCATTTTGTTCTGCAC
**SEQ ID 375 - Third frame nucleotide sequence from HERV-K** 
GTGTACCCAACAGCTCCGAAGAGACAGAGACCATGGAGAACGGGCCATGATGACGATGGCGGTTTTGTCGAAAAGAAAAGGGGAAATG
TGGGGAAAAGAAAGAGAGATCAGATTGTTACTGTGTCTGTGTAGAAAGAAGTAGACATAGGAGACTCCATTTTGTTCTGTAC
**SEQ ID 376 - Third frame nucleotide sequence from HERV-K** 
CTTAAGAGTCATCACCAGGACTTTCTTATAAGCTAATTAACAAATTTGTACATGGTTAACAATTGTTTACATTAAATTCTATTGGTAAA
GTAACTGATGTGATTTTGTTTTCTGAATTGACTCTGACTGATAGAGGGAAATAGACAACAAAAAGATAATACTTATTTACA
**SEQ ID 377 - Third frame nucleotide sequence from HERV-K** 
GTGTACCCAACAGCTCCGAAGAGACAGCGACCATCAAGAACAGGCCAAGATGACGATGGCAGTTTTGTCGAAAAGAGAAGGGGAAATG
TGGGGAAAAGCAAGAGAGATCAGATTGTTACTGTGTCTGTGTAGAAAGAAGTAGACATAGGAGACTCCATTTTCTTATGTAC
**SEQ ID 378 - Third frame nucleotide sequence from HERV-K** 
GTGTACCCAACGGCTCCAAAGAGACAGCGACCATCGAGAACGGGCCATGAAGACGATGGCGGTTTTGTCAAAAAGAAAAGGGGAAATG
TGGGGAAAAGAAAGAGAGATCAGATTGTTACTGTGTCTGTGTAGAAAGAAATAGACATAGGAGACTCCATTTTGTTCTGTAC
**SEQ ID 379 - Third frame nucleotide sequence from HERV-K** 
CCGCCCGAGCAAAGGCCCAGGGAAATGAATGGGTGTCATTCTGGTCCTGACCCGAGGCACAGCCAGGAAGGTCCCTGTGGGAAAAGAA
AGAGATATCAGACTGTTACTGTGTCTATGTAGAAAGAAGTAGACGTAAGAGGCTCCATTTTGTTGTG
**SEQ ID 380 - Third frame nucleotide sequence from HERV-K** 
GTTTTCACCACAGCCGAACAGGGCAGGACCCCAGCACCCGGACCCAGCGGACTTTGCCAAGGGATGGACCTGGCTGGGCCACGCGG
CTGTTTGTGTAGGGAAAAGAAAGAGAGATCACACTGTTACTGTGTCTATGTAGAAAAGGAAGACATAAACTCCATTTTGAGCTGTACTA
AGAAAAATTATTTTGCC
**SEQ ID 381 - Third frame nucleotide sequence from HERV-K** 
GTGTATCCAGCAGCTCCAAAGAGACAGCAACCAGCAAGAATGGGCCATAGTGACGATGGTGGTTTTGTCAAAAAGAAAAGGGGGGGATA
TGTAAGGAAAAGAGAGATCAGACTTTCACTGTGTCTATGTAGAAAAGGAAGACATAAGAAACTCCATTTTGATCTGTAC
**SEQ ID 382 - Third frame nucleotide sequence from HERV-K** 
CCACCGGAGCAAAGGCCCAGGGAAATGAATGGGTGTCATTCTGGTCCTGATCCGAGGCACAGCCAGGAAGGTCCCTGTGGGAAAAGAA
AGAGATATCAGACTGTTACTGTGTCTATGTAGAAAGAAGTAGACGTAAGAGGCTCCATTTTGTTCTG
**SEQ ID 383 - Third frame nucleotide sequence from HERV-K** 
GTGTATCCAACAGCTGTGAAGAGACAGCGACCATCGAGAACGGGCCATGATGACGATGGCGGTTTTGTCAAAAAGAAAAGGGGAAATG
TGGGGAAAAGAAAGAGAGATCAGATTGTTACTGTGTCTGTGTAGAAAGAAGTAGACATAGGAGACTCCATTTTCTG
**SEQ ID 384 - Third frame nucleotide sequence from HERV-K** 
GTGTACCCAACAGCTCTGAAGAGACAGCGACCATCAAGAACGGGCCATGATGATGATGGCAGTTTTGTCGAAAAGAAAAGGGGGAAATG
TGGGGAAAAGAGAGATCAG
**SEQ ID 385 - Third frame nucleotide sequence from HERV-K** 
CCAACAGCTCTGAAGAGACAGCGACCATCGAGAACGGGCCATGATGACGATGGCAGTTTTGTAGAAAAGAAAAGGGGAAATGTGGGA
AAAGAAAGAGAGAACAGATTGTTACTGTGTCTGTGTAGAAAGAAGTAGACATAGGAGACTCCATTTGTTCTGTAC
**SEQ ID 386 - Third frame nucleotide sequence from HERV-K** 
GTGTATCCAGCAGCTCCAGAGAGACAGCGACCAGCGAGAAGGGGCCATAATGATGGTGGCGGTTTTGTCAAAAAGAAAAGGGGGATATG
CAGGGAAAAGAAAGAGAGATCAGACTGTTACTGTGTCTACATAGAAAGG
**SEQ ID 387 - Third frame nucleotide sequence from HERV-K** 
GTGTACCCAACAGCTCCGAAGAGACAGCGACCATTGAGAACGGGCCATGATGACGATGGCAGTTTTGTCGAAAAGAAAAGGAGGAAATG
TGGGGAAAAGAGAGAACAG
**SEQ ID 388 - Third frame nucleotide sequence from HERV-K** 
CTGTACCCAACAGCTCCGAAGAGACAGCGACCATCGAGAACAGGCCATGATGACGATAGTGGTTTTGTCGAAAAGAAAAGGGGAAATG
TGGGGAAAAGCAAGAGAGATCAGATTGTTACTGTGTCTGTGTAGAAAGAAGTAGACATAGGAGACTCCATTTTGTTATGTAC
**SEQ ID 389 - Third frame nucleotide sequence from HERV-K** 
GACAGCGACCGACCAGAGAGAAGGGGCCATGATGATGGTGGTGGTTTTGTCAAAACGAAAAGGGGATATGTAGGGAAAAGAAAGAGAG
ATCAGACTGTTACTGTGTCTACATAGAAAGGGAAGACATAAGAGACTCCATTTTGAAAAAGACCTGTACTTTAAACAGTTGCTTTGACA
GAGACAGTTGCTTGAGTGCATTCATGTGTCTTCTTCCACAG
**SEQ ID 390 - Third frame nucleotide sequence from HERV-K** 
AGCCCCTCTGCCCAGCGGCCACCCCGTCTGGGAGGTGTACCCAATAGCTCATTGAGAACGGGCCATGATGCCGATGGCGGTTTTGTTGA
ATGGAAAAGGGGGAAATGTGGGGAAAAGATAGAGAGATCAGATTGTTACTGTGTCTGTATAGAAAGAAGTAGACATAGGAGACTCCATT
TTGTTCTGTAC
**SEQ ID 391 - Third frame nucleotide sequence from HERV-K** 
GTGTACCCAACATCTCCAAAGAGACAGCGACCATCGAGAACGGGCCATGATGACGATGGCGGTTTTGTCGAAAAGAAAAGGGAAATGT
GGGGAAAAGAAAG
**SEQ ID 392 - Third frame nucleotide sequence from HERV-K** 
GTGTACCCAACAGCTCCGAAGAGACAACGACCATTGAGAATGGGCCATGGTGACGATGGCGGTTTTGTCGAAAAGAAAAGGGGAAATG
TAGGGAAAAGAAAGAGAGATCAGATTGTTACTGTGTCTGTGTAGAAAGAAGTAGACATAGGAGACTCCATTTTGTTCTGTAC
**SEQ ID 393 - Third frame nucleotide sequence from HERV-K** 
CTACAGGTGTATCCAGCAGCTCAAGAGAGACATCGACCAGCGAGAAGGGGCCATGATGATGGTGGTGGTTTTGTGAAAACGAAAAGGGG
GATATATAGGGAAAAGAAAGAGAGATCAGACTGTTACTGTGTCTACACAGAAAGGAAGACATAAGAGACTCCATTTTGAAAAAGACCT
GTACTTTAAACAATTGCTTTGCTGAGATGTTGTTAATCTGTAGCTTTGCCCCGGCCACCTTGCCC
**SEQ ID 394 - Third frame nucleotide sequence from HERV-K** 
GTGTATCCAGCAGCTACGGAGAAACAGCGACCAGCGAGAACGGGCCATGATGACGATGGCGGTGTTGTCAAAAAGAAAAGGGGAAATG
TAGAGAAAAGAAAGAGGGATCAGACTGTCACTGTGTCTATGCAGAAAGG
**SEQ ID 395 - Third frame nucleotide sequence from HERV-K** 
TACCCAACAGCTCCGAAGAGACAGCAACCTTGGAGAACGGCCTTGATGACCTTGGCGGTTTTTCGAAAAGAAAAGGGGAATTTTGG
GGAAAAGAAAGGGGGATCAGATTTTTACTCCGTCTGTGTGGAAAGAAGTAGACATAGGGGACCCCATTTTGTTCTGTAC
**SEQ ID 396 - Third frame nucleotide sequence from HERV-K** 
GTGTACCCAACAGCTCCGAAGAGACAGCAACCATGGAGAACGGGCCATGATGACCATGGCGGTTTTGTCGAAAAGAAAAGGGGAAATG
TGGGGAAAAGAAAGAGAGATCAGATTGTTACTACGTCTGTGTAGAAAGAAGTAGACATAGGAGACTCCATTTTGTTCTGTAC
**SEQ ID 397 - Third frame nucleotide sequence from HERV-K** 
GTGTACCCAACAGCTCCGAAGAGACAGCGACCATCGAGAATGGGCCACGATGACGATGGCGGTTTTGTTGAAAAGAAAAGGGGAAATG
TGGGGAAAAGAAAGAGAGATCAGATTGTTACTGTGTCTGTGTAGAAAGTAGACATAGGAGACTCCATTTTGTTCTGTAC
**SEQ ID 398 - Third frame nucleotide sequence from HERV-K** 
GTGTACCCAACAGCTCCGAAGAGACAGCGACCATCGAGAACGGGCCATGATGATGATGGCGGTTTTGTTGAAAAGAGAAGGGGAAATG
TGGGGAAAAGAAAGAGATCAGATTGTTACTGTGTCTGTGTAGAAAG
**SEQ ID 399 - Third frame nucleotide sequence from HERV-K** 
GTGTACCCAACTGCTCCAAAAAGACAGCAACCATCGAGAACGGGCCATGATGACGATGGCAGTTTTGTCGAAAAGAAAAGGGGAAATG
TGGGGAAAAGAAAGAAAGATCAGATTGTTACTGTGTCTGTGTAGAAAGAAGTAGACATAGGAGACTCCATTTTGTTCTGTAC
**SEQ ID 400 - Third frame nucleotide sequence from HERV-K** 
GTATATCCAGCAGCTCCGGAGAGACAGCGACCAGCGAGAATGGGCCATGATGACGATGGCGGTTTTGTCAAAAAGAAAAGGGGGAAATG
CAGGGAAAAGAAAGAGAGATCAGACTGTCACAGTGTCTATGTAGAAAAG
**SEQ ID 401 - Third frame nucleotide sequence from HERV-K** 
GTGTACCCAACAGCTCCGAAGAGACAGCGATCATCGAGAACGGGCCGTGATAACGATGGCGGTTTTGTCAAAAAGAAAAGGGGAAATG
TGGGGAAAAGAAAGAGAGATCAGATTGTTACTGTGTCTGTGTAGGAAGAAGTAGACATAGGAGACTCCATTTTGTTCTGTAC
**SEQ ID 402 - Third frame nucleotide sequence from HERV-K** 
GTGTATCCAGCAGCTCCAGAGAGACAGCAACCAGCGAGAAGGGGTCATGATGATGGTGGTGGTTTTGTCAAAAAGAAAAGGGGGATATG
CAGGGAAAAGAAAGAGAGATCAGACAGTTACTGTGTCTATATAGAAAGG
**SEQ ID 403 - Third frame nucleotide sequence from HERV-K** 
GTGTATCCAGCAGCTCCGGAGAGACAGCGACCAGTGAGAAGGGGCCATGATGACGATGGCGGTTTTGTTAAAAAGAAAAGGGGGAAATG
TAGGGAAAAGAGAGAGATCAGACTGTCACTGTGTCTATGTAGAAAGGGAAGACATAAGAGACTCCACTTT
**SEQ ID 404 - Third frame nucleotide sequence from HERV-K** 
GTGTACCCAACAGCTCCGAAGAGACAGCGACCATCGAGAACGGGCCATGATGACGATGGCGGTTTTGTTGAAAAGAAAAGGGGAAATG
TGGGGAAAAGAAAAAGAGATCAGATTGTTATTGTGTCTGTGTAGAAAGAAGTAGATGTAGGAGACTCCGTTTTGTTCTGTAC
**SEQ ID 405 - Third frame nucleotide sequence from HERV-K** 
TCGAGAACGGGCCATGATGACGATGGCGGTTTGTTGAAAAGAAAAGGGGAAATGTGGGAAAAGAAAAAGAGATCAGATTGTTATTG
TGTCTGTGTAGAAAGAAGTAGATGTAGGAGACTCCGTTTTGTTCTGTACTAAGAAAAATTCTTCTGCCTTGGGATGCTGTT
**SEQ ID 406 - Third frame nucleotide sequence from HERV-K** 
TTGTATCCAGCAGCTCCAGAGAGACAGCGACCAGCAAGAAGGGGCCATGATGATGGTGGTGGTTTTTCAAAACGAAAAGGGGGATATG
TAGGGAAAAGAAAGAGAGATCAGACTCTTACAGACTCTTACTGTGTCTACATAGAAAGGAAGACATAAGAGACTCCAT
**SEQ 407 - Third frame nucleotide sequence from HERV-K** 
GTGTACCCAACAGCTCCGAAGAGAAAGCGACCATCGAGAATGGGCCATGATGACAATGGCGGTTTTGTCGAAAAGAAAAGGGGAATGT
GGGGAAAAGACAG
**SEQ ID 408 - Third frame nucleotide sequence from HERV-K** 
GTGTACCCAACAGCTCCGAAGAGACAGCAACCATCGAGAACGGGCCATGATGACGATGGCGGTTTTGTCGAAAAGAAAAGAGGGAAGCG
TGGGGAAAAGAAAGAGAGATCAGATTGTTACTGTGTCTGTGTAGAAAGAAGTAGACATAGGAGACTCCCTTTTGTTCTGTAC
**SEQ ID 409 - Third frame nucleotide sequence from HERV-K** 
GTGTACCCAACAGCTCCGAAGAGACAGCGACCATCGAGAACGGGCCATGATGACGATGGCGGTTTTGTCGAAAAGAAAAGGGGAAATG
TGGGGAAAAGCAAGAGAGATCAAATTGTTACTGTGTCTGTGTAGAAAGAAGTAGACATAGGAGACTCCATTTTGTTATG
**SEQ ID 410 - Third frame nucleotide sequence from HERV-K** 
GTGTATCCAGCAGCTTCGGAGACACAGCGACCGGCGAGAACGGACATGATGATGATGGCGGTTTTGTCAAAAAGAAAAGGGGGATATG
TAGGGAAAAGAAAGTGAGATCAGACTGTTACTGTATCTATGTAGAAAGG
**SEQ ID 411 - Third frame nucleotide sequence from HERV-K** 
GTGTACCCAACAGCTCCGAAGAGACAGCGACCAACGAGAACAGGCCATGATGACGATGGCGGTTTTGTCAAAAAGAAAAGGGGAAATG
TGGGGAAAAGAAAGAGAGATCAGGTTGTTACTGTGCCTGTGTAGAAAGAAGTAGACATAGGAGACTCCATTTTGTTCTGTAC
**SEQ ID 412 - Third frame nucleotide sequence from HER-K** 
AAATGTGGGGAAAAGCAAGAGCGATCAGATTGTTGCTGTGTCTGTGTAGAAAGAAGTAGACATAGGAGACTCCATTTGTTATGTACTA
AGAAAAATTCTTCTGCCTTGAGATTCTGTGACCTTACCCCCAACCCCATGCTCTCTGAAACATGTGCTGTGTCAAC
**SEQ ID 413 - Third frame nucleotide sequence from HERV-K** 
GTGTACCCAACAGCTCCGAAGAGACAGCGACCATCGAGAACGGGCCATGATGACGATGGCAGTTTTGTCATCAAGAAAAGGGGGAAACG
TGGGGAAAAGAGAGATCAG
**SEQ ID 414 - Third frame nucleotide sequence from HERV-K** 
GTGTACCCAACAGCTCCGAAGAGACAGCGATCATCGAGAATGGGCCATGATGACGATGGCAGTTTTGTCGAAAAGAAAAGGGGAAATG
TGGGGAAAAGAAAGAGAGATCAGATTGTTACTGTGTCTATGTAGAAAGAAGTAGACATAGGAGACTCCATTTTGTTCTGTAC
**SEQ ID 415 - Third frame nucleotide sequence from HERV-K** 
GTGTACCCAACAGCTCCAAAGAGACAGCGACCATTGAGAATGGGCCATGATGACGATGGCGGTTTTGTCGAAAAGAAAAGAGGGAAATG
TGGGGAAAAGAAAGAGAGATCAGATTGTTACTGTGTCTGTGTAGAAAGAAGTAGACATAGGAGACTCCATTTTGTTCTGTAC
**SEQ ID 416 - Third frame nucleotide sequence from HERV-K** 
GTGTACCCAACAGCTCCGAAGAGACAGCGACCATCGAGAATGGCCCATGATGACGATGGCGGTTTTGTCGAAAACAAAAGCGGGAAATG
TGGGGAAAAGAAAGAGAGATCAGATTGTTACCGTGTCTGTGTAGAAAGAAGTAGACATAGGAGACTCCATTTTGTTCTGTAC
**SEQ ID 417 - Third frame nucleotide sequence from HERV-K** 
GTGTACCCAACAGCTCTGAAGAGACAGCAACCATCGAGAACGGGCCATGATGACGATGGCGGTTTTGTCGAAAAGAAAAGGGGAAATG
TGGGGAAAAGAAAGAGAGATCAGATTGTTACTGTGTCTGTGTAGAAAGAAGTAGACACAGGAGACTCCATTTTGTTCTGTAC
**SEQ ID 418 - Third frame nucleotide sequence from HERV-K** 
GTGTACCCCACAGCTCCGAAGAGGCAGCGACCATCGAGAACGGGCCATGATGACAATGGCGGTTTTGTCGAAAAGAAAAGGGGAAATG
TGGGGAAAAGAAAGATCAG
**SEQ ID 419 - Third frame nucleotide sequence from HERV-K** 
GTGTACCCAACAGCTCCGAAGAGACAGCGACCATCGAGAACGGGCCATGATGATGATGGTGGTTTTGTCGAAAAGAAAAGGGGAAATG
TGGGGAAAAGAAAGAGAGATCAGATTGTTACCGTGTCTGTGTAGAAAGAAGTAGACATAGGAGACTCCATTTTGTTCTGTAC
**SEQ ID 420 - Third frame nucleotide sequence from HERV-K** 
CGAAGAGACAGACCATGGAGAACGGGCCATGATGACGATGGCGGTTTTGTCGAAAAGACAAGGGGGAAATGTGGGGAAAAGAAAGAGAG
ATCAGATTGTTACTGTGTCTGTGTAGAAAGAAGTAGACATAGGAGACACCATTTTGTTCTGTAC
**SEQ ID 421 - Third frame nucleotide sequence from HERV-K** 
GTGTACCTAGCAGCTCCAAAGAGACAGCGACCATCGAGGACAAGCCATGATGACAATGGTGGTTTTGTCAAAAAGAAAAGGGGGAAATG
TGGGGAAGAGAAAGAGAGATCAGACTGTTACTGTGTCTATGTAGAAAGAAGTAGACATAAGAGACTCCATTTTGTTCTGTAC
**SEQ ID 422 - Third frame nucleotide sequence from HERV-K** 
GTGTACCCAGCAGCTCCGAAGAGACAGCGACCATCAAGAACGGGCCATGATGACGATGGCAGTTTTGTCAAAAACAAAAGGGAGAATGT
GGGGAAAAGAAAGAGAGATCAGATTGTTACTGTGTCTATGCAGAAAAGGAAG
**SEQ ID 423 - Third frame nucleotide sequence from HERV-K** 
GAGGTGTACCCAACAGCTCCGAAGAGACAGCGACCATCGAGAACGGGCCATGATGACAATGGCAGTTTTGTCAAAAAGAAAAGGGGGAA
ATGTGGGGAAAAGAAAGAGAGATCAGATTGTTACTGTGTCTGTGTAGAAAGAAGGAGACATAGGAGACTCCATTTTGTTCTGTACCAAG
AAATGTTCTTCTGCCTTGGGATGCTGTTAATCTATAACCTTACCCCTAACCCCCTGCTCTCTGAAACATGTGCTGTG
**SEQ ID 424 - Third frame nucleotide sequence from HERV-K** 
GTGTACCCAACAGCTCCAAAGAGACAGCGACCATCGAGAACAGGCCATGATGAACGGGCCATGATGACGATGGCGGTTTTGTTGAAAAG
AAAAGGGGGAAATGCGGGGAAAAGAGAGATCAGA
**SEQ ID 425 - Third frame nucleotide sequence from HERV-K** 
GTGTACCCAACAGCTCCGAAGAGACAGCGACCATCGAGAATGGGCCATGATGACAATGGCAGTTTTGTGGAAAAGAAAAGGGGGAAATG
TGGGGAAAAGAAAGAGAGATCAGATTGTTACTATGTCTGTGTAGAAAGAAGTAGACACAGGAGACTCCATTTTGTTCTGTAC
**SEQ ID 426 - Third frame nucleotide sequence from HERV-K**
**SEQ ID 427 - Third frame nucleotide sequence from HERV-K** 
GTGTACGCAACAGCTCTGAAGAGACAGCGACCATCGAGAACGGGCCATGATGACGATGGCGGTTTTGTCGAAAAGAAAAGGGGAAATG
TGGGGAAAAGAAAGAGAGATCAGATTGTTACTGTGTCTGCATAGAAAGAAGTAGACATAGGAGACACCATTTTGTTCTATAC
**SEQ ID 428 - Third frame nucleotide sequence from HERV-K** 
GTGTACCCAACATCTCCAAAGAGACAGAGACCATCGAGAACAGGCCATGATGACGATGGTGGTTTTGTCAAAAAGAAAAGGGGAAATG
TGGGGAAAAGAAAGAGAGATCAGATTGTTACTGTGTCTGTGTAGAAAGAAGTAGACATAGGAGACTCCATTTTGTTCTGTAC
**SEQ ID 429 - Third frame nucleotide sequence from HERV-K** 
GTGTACCCAACAGCTCCAAAGAGACAGCGACCATCGAGAACGGGCCATGATGATGATGGCGGTTTTGTCGAAAAGAAAAGGGGAAATG
TGGGGAAAACAAAGAGAGATCAGATTGTTACTGTGTCTGTGTAGAAAGAAGTAGACATAAGAGACTCCATTTTGTTCTGTAC
**SEQ ID 430 - Third frame nucleotide sequence from HERV-K** 
GTGTACCCAACAGCTCCAAAGAGACAGCAATCATCCAGAACGGGCCATGATGACGATGGTGGTTTTGTCGAAAAGAAAAGGAGAAATG
CGGGGAAAAGAAAGAGAGATCAGATTGTTACCGTGTCTGTGTAGAAAGAAGTAGACATAGGAGACTCCATTTTGTTCTGTAC
**SEQ ID 431 - Third frame nucleotide sequence from HERV-K** 
GTGTATCCAACAGCTCTGAAGAGACAGCGACCATGGAGAACGGGCCATGATGACGATGGTGGTTTTGTCGAAAAGAAAAGGGGAAATG
TGGGGAAAAGAAAGAGAGATCAGATTGTTACTGTGTCTGTGTAGAAAGAAGTAGACATAGGAGACTCCATTTTGTTCTGTAC
**SEQ ID 432 - Third frame nucleotide sequence from HERV-K** 
GTGTACCCAACAGCTCCGAAGAGACAGCGGCCATCGAGAGCGGGCCATGATGACGATCGCGGTTTTGTTGAAAAGAAAAGGGGAAATG
TGGGGAAAAGAAAGAGAGATCAGATTGTTACTGTGTCTGTGTAGAAAGAAGTAGACATAGGAGACTCCATTTTGTTCTGTAC
**SEQ ID 433 - Third frame nucleotide sequence from HERV-K** 
TTGTACCCAACAGCTCCAAAGAGACAGCGACCATTGAGAATGGGCCATGATGCCGATGGCGGTTTTGTTGAAAAGAAAAGGGGAAATG
TGGGGAAAAGAAAGAGATCAGATTGTTACTGTGTCTGTGC
**SEQ ID 434 - Third frame nucleotide sequence from HERV-K** 
GTGTACCCAACAGCTCCAAAGAGACAGCGACCATTGAGAACGGGCCATGATGACGATAGTGGTTTTGTCGAAAAGAAAAGGGGAAATG
TGGGGAAAAGAAAGAGAGATCAGATTGTTACTGTGTCTGTGTAGAAAGAAGTAAACATAGGAGACTCCATTTTGTTCTGTAC
**SEQ ID 435 - Third frame nucleotide sequence from HERV-K** 
GTGTACCCAACAGCTCCGAAGAGACAGCGACCATCGAGAGCGGGCCATGATGACGATGGCGGTTTTGTCGAAAAGAAAAGGGGAAATG
TGGGGAAAAGGAAGAGAGGTCAGATTTGTACTGTGTCTGTGTAGAAAGAAGTAGACATAGGAGACTCCATTTTGTTCTGTAC
**SEQ ID 436 - Third frame nucleotide sequence from HERV-K** 
GAGGTGTACCCAACAGCTCCGAAGAGACAGCGACCATCGAGAACGGGCCATGATGACGATGGCAGTTTTGTCGAAAAGAAAAGGGGAA
ATGTGGGAAAAGAAAGAGAGATCAGATTGTTCCTGTGTCTGTGTAGAAAGAAGTAGACATAGGAGACTCCATTTTGTTCTG
**SEQ ID 437 - Third frame nucleotide sequence from HERV-K** 
GTGTACCCAACAGCTTGGAAGAGACAGCGACCATCGAGAATGGGCCATGATGACGATGGCGGTTTTGTCGAAAAGAAAAGGGGAAATG
TGGGGAAAACAGAGATCAG
**SEQ ID 438 - Third frame nucleotide sequence from HERV-K** 
GTATACCCAACTGCTCCGAAGAGACAGCAACCATCGAGAACGGGCCATGATGACGATGGCAGTTTTGTCAAAAAGAAAAGGGGAAATG
TGGGGAAAAGAAAGAGAGATCAGATTGTTGCTGTGTCTGTGTAGAAAGAAGTAGACATAGGAGACTCCATTTTGTTCTGTAC
**SEQ ID 439 - Third frame nucleotide sequence from HERV-K** 
GTGTACCCAACAGCTCCGAAGAGACAGCGACCATGGAGAACGGGCCATGATGACGATGGTGGTTTTGTCAAAAAGAAAAGGGGAAATG
TGGGGAAAAGAAAAAGAGATCAGATTGTTACTGTGTCTGTGTAGAAAGAAGCAGACATGGGAGACTCCGTTTTGTTCTGTAC
**SEQ ID 440 - Third frame nucleotide sequence from HERV-K** 
CCAGCCTGGCCAACATGGAGAAATCCCGTCTCTACTAAAAATACAAAATTAGCCAGGCATGGTGCTGCATGCCTGCAATCCTGTAGGGA
AAAGAAAGAGAGATCAGACTGTTACTGTGTCTGTGTAGAAAGGGAAGACATAAGAAATTCCATTTTGACCTGTACCTTGAACAATTGGT
TGGCTGAGATGCTGTTAATTTGTGACTTTGCCCCAAATTTGAGCTCACAA
**SEQ ID 441 - Third frame nucleotide sequence from HERV-K** 
GTGTACCCAACAGCTCCGAAGAGACAGCGACCATCGAGAACGGGCCATGATGACGATGGCGCTTTTGTCGAAAAGAAAAGGGGAAATG
TGGGGAAAAGAAAGAGAGATCAGATTGTTACTGTGTC
**SEQ ID 442 - Third frame nucleotide sequence from HERV-K** 
GTGTACCCAACAGCTCCGAAGAGACAGCAACCATTGAGAACAGGCCATAATGACGATGGCGGTTTTGTTGAAAAGAAAAGGGGAAATA
TGGGGAAAAGAAAGAGAGATCAGATTGTTACTGTGTCTGTGTAGAAAGAAGTAGACATAGGAGACTCCATTTTGTTCTGTAC
**SEQ ID 443 - Third frame nucleotide sequence from HERV-K** 
GTGTACCCAATAGCTCTGAAGAGACAGCGACCATCGAGAACGGGCCATGACGACGATGGCGGTTTTGTCAAAAAGAAAAGGGGAAATG
TGGGGAAAAGAAAGAGAGATCAGATTGTTACTGTGTCTATGTAGAAAGAAGTAGACATAGGAGACTCCATTTTGTTCTGTAC
**SEQ 444 - Third frame nucleotide sequence from HERV-K** 
CTACAGGTGTATCCAGCAGCTCCGGAGAGACAGCGGCTAGCGAGAACGGACCATGATGATGATGGCGGTTTTGTCAAAAAGAAAAGGGG
GATATGTAGGGAAAAGAGAGAGAGATCAGACTGTTACTGTGTCTATGTAGAAAGGGAAGACATAAGAGACTCCATTTTGAAAAAGACCT
GTACTTTGAACAATTGCTTTGCTCAGATGTTGTTAATTTGTAGTTTTGCCCCAGCCACTTTGACCCAAC
**SEQ ID 445 - Third frame nucleotide sequence from HERV-K** 
GTGTACCCAACAGCTCGGAAGAGACAGCGACCATCGAGAACGGGCCATGATGATGATGGCGGTTTTGTCAAAAAGAAAAGGGGGAAATG
TGGGGAAAAGAAAGAGAGATCAGATTGTTACTGTGTCTGTGTAGAAAGAAGTAGACATAGGAGACTCCATTTTGTTCTG
**SEQ ID 446 - Third frame nucleotide sequence from HERV-K** 
GTGTACCCAACAGCTCCGAAGAGACAGCGACCATCGAGAACAGGCCATGATGACGATCGCGGTTTTGTCAAAAAGAAATGGGGGAAAAT
GTGGGGAAAAAAAAGAGAGATCAGATTGTTACTGTGTCTGTGTAGAAAG
**SEQ ID 447 - Third frame nucleotide sequence from HERV-K** 
GTGTACCCAACAGCTCCAAAGAGACAGCGACCATTGAGAAGGGGCCATGATGACGATGGTGGTTCTGTCAAAAAGAAAAGGGGGAAATG
TGGGGAAAAGAAAGAGAGATCAGATTGTTACTGTGTCTGTGTAGAAAGAAGTAGACATAAGAGACTCCATTTTGTTCTGTAC
**SEQ ID 448 - Third frame nucleotide sequence from HERV-K** 
GTGTATCCAGCAGCTCCAGAGAGACAGCGACCAGCGAGAAGGGGCCATGATGATGGTGGTGGTTTTGTCAAAACGAAAAGGGGGATATG
TAGGGAAAAGAAAGAGAGATCAGACTCTTACTGTGTCTACATAGAAAGG
**SEQ ID 449 - Third frame nucleotide sequence from HERV-K** 
GTGTATCCAGCAGCTCCAGAGAGACAGCGACCAGCGAGAAGGGGCCATGATGATGGTGGTGGTTTTGTCAAAATGAAAAGGGGGATATG
TAGGGAAAAGAAAGAGAGATCAGACTGTTACTGTGTCTACATAGAAAGGGAAGCCATAAGA
**SEQ ID 450 - Third frame nucleotide sequence from HER-K** 
CTACAGGTGTATCCAGCAGCTCCGGAGAGACAGCGACCAGGGAGAAGGGGCCATGATGACCATGGCGGTTTTGTCAAAAAGAAAAGCGG
GAAATGTAGGGAAAAGAGACAGATCAGACTGTCACTGTGTCTATGTAGAAAGGGAAGACATAAGAGACTCCATTTGAAAAAGACCTGT
ACTCTAACAATTGCTTTGCTGAGATGTTGTTCATTTGTAGCTTTGCCCCAGCCACTTTGCCCCAGTCAC
**SEQ ID 451 - Third frame nucleotide sequence from HERV-K** 
CTACAGGTGTATCCAGCAGCTCCAGAGAGACAGCGACCAGCGAGAAGGGGCCATGATGATGGTGGTGGTTTTGTCAAAACGAAAAGGGG
GATATGTAGGGAAAAGAAAGAGAGATCAGACTGTTACTGTGTCTACATAGAAAGGGAAGCCATAAGAGACTCCATTTTGAAAAAGACCT
GTACTTTAAACAATTGCTTGCTGAGATGTTGTTTATCTGTAGCTTTGCCCCAGCCACTTTGCCCCAAC
**SEQ ID 452 - Third frame nucleotide sequence from HERV-K** 
CTACAGGTGTATCCAGCAGCTCCGGAGAGACAGCGACCAGCGAGAAGGACCATGATGACCATGGCGGTTTTGTCAAAAAGAAAAGCGG
GAAATGTAGGGAAAAGAGAGAGATCAGACTGTCACTGTGTCTATGTAGAAAGGAAGACATAAGAGACTCCATTTGAAAAAGACCTGT
ACTCTAACAATTGCTTTGCTGAGATGTTGTTCATTTGTAGCTTTGCCCCAGCCACTTTGCCCCAGTCAC
**SEQ ID 453 - Third frame nucleotide sequence from HERV-K** 
CTACAGGTGTATCCAGCAGCTCCGGAGAGACAGCGACCAGCGAGAAGGGACCATGATGACCATGGCGGTTTTGTCAAAAAGAAAAGCGG
GAAATGTAGGGAAAAGAGAGAGATCAGACTGTCACTGTGTCTATGTAGAAAGGGAAGACATAAGAGACTCCATTTGAAAAAGACCTGT
ACTCTAACAATTGCTTTGCTGAGATGTTGTTCATTTGTAGCTTTGCCCCAGCCACTTTGCCCCAGTCAC
**SEQ ID 454 - Third frame nucleotide sequence from HERV-K** 
CTACAGGTGTATCCAGCAGCTCCAGAGAGACAGCAACCAGCGAAAACAGGCCATAATGACTATGGCGGTTTGTCAAAAAGAAAAGGGG
GATATGTACGGCAAAGAAAGAGAGATCAGACTGTTACTGTGTCTATGTAGAAAGGGAAGACATAAGAAATTCCATTTTGACCTGTACCT
TGAACAATTGCTTTGCTGAGATGTTGTTAATTTGTAACTTTGCCCCAGCCACTTTGCCCCAA
**SEQ ID 455 - Third frame nucleotide sequence from HERV-K** 
CTGCACCCGCTGTCACCGTCACAGCTGGCCCCACCTCAGCCGGACACCCTGCCTGGGCCACTCCAAGTGACTGTCACAACCCGAGAGC
CTATGGCCAAGATGAGCTCCACCAAGTAAAAATGGTGGAGTGTGGGGAAAAGCAAGAGAGATCAGAGTGTCACTGTATCTGTGTAGAAA
GAAGTAGACATGGAAGACTCCATTTTGTTATGTAC
**SEQ ID 456 - Third frame nucleotide sequence from HERV-K** 
CCTCTGTGTCCCAGGCTAAAGCAGTCTTCCCGCCTCAGCCTCTCGAGTAGCAGAGACTGCTGCGGGGAAAAGCAAGAGAGATCAGATTG
TTACTGTGTCTGTATAGAAAGAAGTAGACATAGGAGACTCCATTTGTTCTGTAC
**SEQ ID 457 - Third frame nucleotide sequence from HERV-K** 
CTGTACCCAACAGCTCCGAAGAGACAGCGACCATCGAGAATGGGCCATGATGATGATGGTGGTTTTGTCAAAAAGAAAAGGGGGAAATG
TGGGGGAAAGAGA
**SEQ ID 458 - Third frame nucleotide sequence from HERV-K** 
GTGTACCCAACAGCTCCAAAGAGACAGCGACCATCGAGAAGGGGCCATGATGACGATGGCGGTTTTGTCGAAAAGAAAAGGGGGAAATG
TGAGGAAAAGAAAGAGAGATCAGATTGTTACTGTGTCTGTGTAGAAAGAAGTAGACATAGGAGACTCCATTTCATTCTGTAC
**SEQ ID 459 - Third frame nucleotide sequence from HERV-K** 
GTGTACCCAACAGCTCTGAAGAGACAGCGACCATCAAGAACGGGCCATGATGACGATGGCGGTTTTGTCGAAAAGAAAAGGGGAAATG
TGGGGAAAAGAAAGAGAGATCAGATTGTTACTGTGTCTGTGTAGAAAGAAGTAGACATAGGAGACTCCATTTTGTTCTGTAC
**SEQ ID 460 - Third frame nucleotide sequence from HERV-K** 
TGGCCAGCCGCCCCGTCTGGAGGTGTACCCAACAGCTGAGAACGGGCCATGATGACAATGGCGGTTTTGTGGAGTGGAAAGGGGGAA
AGGTGGGAAAAGATTGAGAAATCGGATGGTTGCCGTGTCTGTGTAGAAAGAGGTAGACATGGGAGATTTTTCATTTTGTTC
**SEQ ID 461 - Third frame nucleotide sequence from HERV-K**
**SEQ ID 462 - Third frame nucleotide sequence from HERV-K** 
GTGTACCCAACAGCTCCGAAGAGACAGCGACCATCGAGAATGGGCCATGATGACGATGGCGGTTTTGTAGAAAAAAAAGGGGGAAATGT
GGAGAAAAGAAAGAGAGAACAGATTGTTACTGTGTCTGTG
**SEQ ID 463 - Third frame nucleotide sequence from HERV-K** 
GTGTACCCAACAGCTCCGAAGAGACAGCGACCATCGAGAATGGGCCATGATGACGATGGCAGTTTTGTCGAAAAGAAAAGGGGAAATG
TGGGGAAAAGAAAGAGAGATCAGATTGTTACTGTGTCTGTGTAGAAAGAAGTAGACATAGGAGACTCCATTTTGTTCTGTAC
**SEQ ID 464 - Third frame nucleotide sequence from HERV-K** 
GTGTACCCAACAGCTCCGAAGAGACAGCGACCATCGAGAACGGGCCATGATGACGATGGCAGTTTTGTCGAAAAGCAAAGGGGGAAATG
TGGGGAAAAGAAAGAGAGATCAGATTGTTACTGTGTCTGTGTAGAAAGAAGTAGACATAGGAGACTCCATTTTGTTCTGTAC
**SEQ ID 465 - Third frame nucleotide sequence from HERV-K** 
GTGTACCCAGCAGCTCCGAAGAGACAGCGACCATCGAGAACGGGCCATGATGACGATGGCGGTTTTGTCGAAAAGAAAAGGGGAAATG
TGGGGAAAAGAAAGAGAGATCAGATTGTTACTGTGTCTGTGTAGAAAGAAGTAGACATAGGAGACTCCATTTTGTTCTG
**SEQ ID 466 - Third frame nucleotide sequence from HERV-K** 
GTGTACCCAACAGCTCCGAAGAGACAGCGACCATCGAGAAGGGGCCATGATGACGATGGCGGTTTTGTCAAAAAGAAAAGGGGAAATG
TGGGGAAAAGAAAGAGAGATCAGATTGTTACTGTGTCTGTGTAGAAAGAAGTAGACATAGGAGACTCCATTTTGTTCTGTAC
**SEQ ID 467 - Third frame nucleotide sequence from HERV-K** 
GTGTACCCAACAGCTCCGAAGAGACAGCGACCATCGAGAATGGGCCATGATGACGATGGCGGTTTTGTCGAAAAGAAAAGGGGAAATG
TGGGGAAAAGAAAGAGAGAACAGATTGTTACTGTGTCTATATAGAAAGAAGTAGACATAGGAGACTCCATTTTGTTCTGTAC
**SEQ ID 468 - Third frame nucleotide sequence from HERV-K** 
GTGTACCCAACAGCTCCGAAGAGACAGCGACCATCCAGAACGGGCCATGATGACGATGGCGGTTTTGTTGAAAAGAAAAGGGGAAATG
TAGGGAAAAGAAAGAGCGATCAGATTGTTACTGTGTCTGTGTAGAAAGAAGGAGACATAGGAGACTCCATTTTGTTCTGTAC
**SEQ ID 469 - Third frame nucleotide sequence from HERV-K** 
GTGTACCCAACAGCTCTGAAGAGACAGCGACCATTGAGAACGGGCCATGATGACAATGGCAGTTTTGTCGAAAAGAAGAGGGGAAATG
TGGGGAAAAGAAAGAGAGATCAGATTGTTACTGTGTCTGTGTAGAAAGAAGTAGACATAGGAGACTCCATTTTGTTCTGTAC
**SEQ ID 470 - Third frame nucleotide sequence from HERV-K** 
GTGTACCCAACAGCTCCGAAGAGACAGCGACCATCGAGAACGGGCCATGATGACGACGGCAGTTTTGTCGAAAAGAAAAGGGAAAATG
TGGGGAAAAGAAAGAGACATCAGATTGTTACTGTGTCTGTGTAGAAAGAAGTAGACATAGGAGACTCCATTTTGTTCTGTAC
**SEQ ID 471 - Third frame nucleotide sequence from HERV-K**
**SEQ ID 472 - Third frame nucleotide sequence from HERV-K** 
GTGTACCCAACAGTTCCGAAGAGACAGCGACCATCGAGAAAGGGCCATGAAGACGATGGCTGTTTTGTCAAAAAGAAAAGGGGAAATT
TGGGGAAAAGAAAGAGAGATCAGATTGTTACTGTGTCTGTGTAGAAAGAAGTAGACATAGGAGACTCCATTTTGTTCTGTAC
**SEQ ID 473 - Third frame nucleotide sequence from HERV-K** 
ATGTACCCAACACCTCTGAAGAGACAGCGACCATGGAGAACGGGCCATGATGACAATGGCGGTTTTGTCGAAAAGAAAAGGGGAAATG
TGGGGAAAAGAAAGAGAGATCAGATTGTTACTGTGTCTGTGTAGAAAGAAGTAGACATAGGAGACTCCATTTTGTTCTGTAC
**SEQ ID 474 - Third frame nucleotide sequence from HERV-K** 
GTGTACTCAACAGCTCCGAAGAGACAGCGACCAGGGAGAATGGGCCATGATGACGTGGCGGTTTTGTCGAAAAGAAAAGGGGGAAATGT
GGGGAAAAGAAAGAGAAATCAGATTGTTACTGTGTCTGTG
**SEQ ID 475 - Third frame nucleotide sequence from HERV-K** 
GTGTACCCAACAGCTCCGAAGAGACAGAGACCATCAAGAACGGGCCATGATGACGATGGCGGTTTTGCCGAAAAGAAAAGGGGAAATG
TGGGGAAAAGAAAGAGAGATCAGATTTTACTGTGTCTGTGCAGAAAGAAGTAGACATAGGAGACACCACTTTGTTCTGTAC
**SEQ ID 476 - Third frame nucleotide sequence from HERV-K** 
CTACAGGTGTATCCAGCAGCTCCGGAGAGACAGCGACCAGCGAGAAGGGGCCATGATGACCATGGCGGTTTTGTCAAAAAGAAAAGCGG
GAAATGTAGGGAAAAGAGAGAGATCAGACTGTCACTGTGTCTATGTAGAAAGGAAGACATAAGAGACTCCATTTTGAAAAAGACCTGT
ACTCTAACTATTGCTTTGCTGAGATGTTGTTCATTTGTAGCTTTGCCCCAGCCACTTTGCCCCAGTCACTTTGCCCC
**SEQ ID 477 - Third frame nucleotide sequence from HERV-K** 
CTACAGGTGTATCCAGCAGCTCCGGAGAGACAGCAACCAGCGAGAACGGGCCATGATGACTATGGCAGTTTTGTCAAAAAGAAAAGGGA
CATATGTAGGGAAAAGAAAGAGAGATCAGACTGTTACTGTGTCTATGTAGAAAAGAAAGACATAAGAGACTCCATTTTGAAAAAGACCT
GTACTTTGAACAATTGCTTTGCTGAGATGTTGTTAATTTGTAGCTTTGCCCCAGCCACTTTGACCCAACCTGGAGCT
**SEQ ID 478 - SEQ ID 49, excluding its 77 5' nucleotides**
   TTATGTGTATGCATATCTAAAAGCACAGCACTTAATCCTTTACATTGTCTATGATGCAAAGACCTTTGTTCAC

## Claims

1. A method for diagnosing prostate cancer, the method comprising the step of detecting the presence or absence of a HML-2 expression product in a patient sample, wherein the expression product is produced by a splicing event in which the 5' region of a HML-2 env coding region and the start codon of the env coding region are joined to a downstream coding region in the reading frame +2 relative to that of env.

2. The method of claim 1, wherein the patient sample is a prostate tissue sample or a blood sample.

3. The method of any preceding claim, wherein the expression product is a mRNA transcript or a polypeptide.

4. The method of any preceding claim, wherein the mRNA transcript comprises a sequence which has at least 50% sequence identity to one or more of SEQ IDs 19, 20, 21, 24, 25, 26, 38, 40, and/or 42.

5. The method of any preceding claim, wherein the mRNA transcript encodes a polypeptide having at least 50% sequence identity to one or more of SEQ IDs 7, 8, 9, 10, 11, 12, 28, 29, 30, 31, 34, 35, 36, 39, 41 43, 67, 68 and 69.

6. The method of any preceding claim, wherein the method comprises the step of extracting RNA from the patient sample.

7. The method of any preceding claim, wherein the expression product is detected by hybridization.

8. An isolated polynucleotide comprising:
(a) the nucleotide sequence of SEQ IDs 19, 20, 21, 24, 25, 26, 38, 40 and/or 42;
(b) a fragment of at least 60 nucleotides of (a), wherein:
(i) the fragment is RNA, and the 5' region and start codon of the env coding region are joined to a downstream coding region in the reading frame +2 relative to that of env;
(ii) the fragment comprises the splice junction;
(iii) the fragment is in the +2 reading frame relative to the start codon of PCAV env; or
(iv) the fragment comprises a region that utilises the +2 reading frame relative to the start codon of PCAV env; or
(c) a nucleotide sequence having at least 95% identity to (a); or (b) the complement of (a) or (b).

9. An isolated polypeptide encoded by a transcript produced by a splicing event in which the 5' region of a HERV-K env coding region and the start codon of a HERV-K env coding region are joined to a downstream coding region in the reading frame +2 relative to that of env in the HERV-K genome, comprising:
(a) an amino acid sequence selected from the group consisting of SEQ IDs 7, 8, 9, 10, 11, 12, 28, 29, 30, 31, 34, 35, 36, 39, 41, 43, 66, 67, 68 and 78 to 277;
(b) a fragment of at least 45 amino acids of:
(i) SEQ IDs 7, 8, 9, 10, 11, 12, 28, 29, 30, 31, 34, 35, 36, 66, 67, 68 and 78 to 277;
(ii) SEQ IDs 39, 41, 43, wherein the fragment comprises a region which is encoded by a transcript produced by a splicing event in which the 5' region and start codon of the HERV-K env coding region are joined to a downstream coding region in the reading frame +2 relative to that of env in the HERV-K genome; or
(iii) SEQ IDs 39, 41, 43, wherein the fragment comprises a region which is encoded by a transcript produced by a splicing event in which the 5' region and start codon of the HERV-K env coding region are joined to a downstream coding region in the reading frame +2 relative to that of env in the HERV-K genome, and wherein the fragment comprises an epitope; or
(c) a polypeptide sequence having at least 80% identity to (a).

10. The polypeptide of claim 9(b), wherein the fragment comprises a T cell or a B cell epitope of one or more of SEQ IDs 7, 8, 9, 10, 11, 12, 28, 29, 30, 31, 34, 35, 36, 39, 41, 43, 66, 67, 68 and 78 to 277.

11. An isolated polynucleotide which encodes the polypeptide of claim 9 or claim 10, wherein polypeptide(s) consisting or comprising of SEQ IDs: 11, 28, 29 or 31 are encoded by a polynucleotide selected from SEQ IDs: 19, 20 or 21.

12. An antibody that binds to the polypeptide of claim 9, claim 10 or claim 11.

13. The antibody of claim 12, wherein the antibody is a monoclonal antibody.

14. The polynucleotide, polypeptide or antibody of any one of claims 8 to 13, for use as a medicament.

15. The use of polynucleotide, polypeptide or antibody of any one of claims 8 to 13, in the manufacture of a medicament for preventing or treating prostate cancer, breast cancer, testicular cancer, multiple sclerosis and/or insulin-dependent diabetes mellitus.

16. An immunogenic composition comprising the polypeptide of claim 9 or claim 10 or claim 11, and a pharmaceutically acceptable carrier.

17. The composition of claim 16, further comprising an adjuvant.

## Patentansprüche

1. Verfahren zur Diagnose von Prostatakrebs, wobei das Verfahren den Schritt des Nachweises des Vorhandenseins oder des Fehlens eines HML-2-Expressionsprodukts in einer Patientenprobe umfasst, worin das Expressionsprodukt durch ein Spleiß-Ereignis produziert wird, bei dem die 5'-Region einer HML-2-env-Codierregion und das Startcodon der env-Kodierregion mit einer stromabwärts gelegenen Kodierregion im Leserahmen +2 relativ zu der von env verbunden werden.

2. Verfahren nach Anspruch 1, worin die Patientenprobe eine Prostatagewebeprobe oder eine Blutprobe ist.

3. Verfahren nach einem der vorhergehenden Ansprüche, worin das Expressionsprodukt ein mRNA-Transkript oder ein Polypeptid ist.

4. Verfahren nach einem der vorhergehenden Ansprüche, worin das mRNA-Transkript eine Sequenz umfasst, die eine Sequenzidentität von zumindest 50% mit ein oder mehr SEQ IDs 19, 20, 21, 24, 25, 26, 38, 40 und/oder 42 aufweist.

5. Verfahren nach einem der vorhergehenden Ansprüche, worin das mRNA-Transkript ein Polypeptid kodiert, das eine Sequenzidentität von zumindest 50% mit ein oder mehr SEQ IDs 7, 8, 9, 10, 11, 12, 28, 29, 30, 31, 34, 35, 36, 39, 41, 43, 67, 68 und 69 aufweist.

6. Verfahren nach einem der vorhergehenden Ansprüche, worin das Verfahren den Schritt der Extraktion von RNA aus der Patientenprobe umfasst.

7. Verfahren nach einem der vorhergehenden Ansprüche, worin das Expressionsprodukt durch Hybridisierung nachgewiesen wird.

8. Isoliertes Polynukleotid, umfassend:
(a) die Nukleotidsequenz von SEQ IDs 19, 20, 21, 24, 25, 26, 38, 40 und/oder 42;
(b) ein Fragment von zumindest 60 Nukleotiden von (a), worin:
(i) das Fragment RNA ist und die 5`-Region und das Startcodon der env-Kodierungsregion mit einer stromabwärts gelegenen Kodierungsregion im Leserahmen +2 relativ zu der von env verbunden sind;
(ii) wobei das Fragment eine Spleißverbindung umfasst;
(iii) wobei das Fragment im +2 Leserahmen relativ zu dem Startcodon von PCAV env liegt; oder
(iv) wobei das Fragment eine Region umfasst, die den +2 Leserahmen relativ zu dem Startcodon von PCAV env nutzt; oder
(c) eine Nukleotidsequenz mit einer zumindest 95%igen Identität mit (a); oder (b) das Gegenstück von (a) oder (b).

9. Isoliertes Polypeptid, kodiert durch ein Transkript, das durch ein Spleiß-Ereignis produziert wurde, wobei die 5`-Region einer HERV-K-env-Kodierregion und das Startcodon einer HERV-K-env-Kodierregion mit einer stromabwärts gelegenen Kodierregion im Leserahmen +2 relativ zu der von env im HERV-K-Genom verbunden sind, umfassend:
(a) eine Aminosäuresequenz, die aus der aus SEQ IDs 7, 8, 9, 10, 11, 12, 28, 29, 30, 31, 34, 35, 36, 39, 41, 43, 66, 67, 68 und 78 bis 277 bestehenden Gruppe ausgewählt ist;
(b) ein Fragment von zumindest 45 Aminosäuren von:
(i) SEQ IDs 7, 8, 9, 10, 11, 12, 28, 29, 30, 31, 34, 35, 36, 66, 67, 68 und 78 bis 277;
(ii) SEQ IDs 39, 41, 43, worin das Fragment eine Region umfasst, kodiert durch ein Transkript, das durch ein Spleiß-Ereignis produziert wurde, wobei die 5'-Region und das Startcodon der HERV-K-env-Kodierregion mit einer stromabwärts gelegenen Kodierregion im Leserahmen +2 relativ zu der von env im HERV-K-Genom verbunden sind; oder
(iii) SEQ IDs 39, 41, 43, worin das Fragment eine Region umfasst, kodiert durch ein Transkript, das durch ein Spleiß-Ereignis produziert wurde, wobei die 5'-Region und das Startcodon der HERV-K-env-Kodierregion mit einer stromabwärts gelegenen Kodierregion im Leserahmen +2 relativ zu der von env im HERV-K-Genom verbunden sind, und worin das Fragment ein Epitop umfasst; oder
(c) eine Polypeptidsequenz mit einer Identität von zumindest 80% mit (a).

10. Polypeptid nach Anspruch 9(b), worin das Fragment eine T-Zell- oder ein B-Zell-Epitop von ein oder mehr SEQ IDs 7, 8, 9, 10, 11, 12, 28, 29, 30, 31, 34, 35, 36, 39, 41, 43, 66, 67, 68 und 78 bis 277 umfasst.

11. Isoliertes Polynukleotid, welches das Polypeptid nach Anspruch 9 oder Anspruch 10 kodiert, worin das/die Polypeptid(e) aus SEQ IDs: 11, 28, 29 oder 31 bestehen oder diese umfassen, durch ein Polynukleotid, ausgewählt unter SEQ IDs: 19, 20 oder 21 kodiert sind.

12. Antikörper, der an das Polypeptid nach Anspruch 9, Anspruch 10 oder Anspruch 11 bindet.

13. Antikörper nach Anspruch 12, worin der Antikörper ein monoklonaler Antikörper ist.

14. Polynukleotid, Polypeptid oder Antikörper nach einem der Ansprüche 8 bis 13, zur Verwendung als Medikament.

15. Verwendung des Polynukleotids, Polypeptids oder Antikörpers nach einem der Ansprüche 8 bis 13, bei der Herstellung eines Medikaments zur Prävention oder Behandlung von Prostatakrebs, Brustkrebs, Hodenkrebs, multipler Sklerose und/oder insulinpflichtigem Diabetes mellitus.

16. Immunogene Zusammensetzung, umfassend das Polypeptid nach Anspruch 9 oder Anspruch 10 oder Anspruch 11 und einen pharmazeutisch annehmbaren Träger.

17. Zusammensetzung nach Anspruch 16, ferner umfassend ein Adjuvans.

## Revendications

1. Procédé pour diagnostiquer un cancer de la prostate, le procédé comprenant l'étape de détection de la présence ou de l'absence d'un produit d'expression de HML-2 dans un échantillon patient, dans lequel le produit d'expression est produit par un événement d'épissage dans lequel la région 5' d'une région codant pour env de HML-2 et le codon d'initiation de la région codant pour env sont réunis à une région codante aval dans le cadre de lecture en +2 par rapport à celui de env.

2. Procédé selon la revendication 1, dans lequel l'échantillon patient est un échantillon de tissu prostatique ou un échantillon sanguin.

3. Procédé selon l'une quelconque des revendications précédentes, dans lequel le produit d'expression est un transcrit d'ARNm ou un polypeptide.

4. Procédé selon l'une quelconque des revendications précédentes, dans lequel le transcrit d'ARNm comprend une séquence qui a une identité de séquence d'au moins 50 % avec une ou plusieurs des séquences SEQ ID NO:19, 20, 21, 24, 25, 26, 38, 40 et/ou 42.

5. Procédé selon l'une quelconque des revendications précédentes, dans lequel le transcrit d'ARNm code pour un polypeptide ayant une identité de séquences d'au moins 50 % avec une ou plusieurs des séquences SEQ ID NO:7, 8, 9, 10, 11, 12, 28, 29, 30, 31, 34, 35, 36, 39, 41, 43, 67, 68 et 69.

6. Procédé selon l'une quelconque des revendications précédentes, le procédé comprenant l'étape d'extraction de l'ARN à partir de l'échantillon patient.

7. Procédé selon l'une quelconque des revendications précédentes, dans lequel le produit d'expression est détecté par hybridation.

8. Polynucléotide isolé, comprenant :
(a) la séquence nucléotidique SEQ ID NO:19, 20, 21, 24, 25, 26, 38, 40 et/ou 42 ;
(b) un fragment d'au moins 60 nucléotides de (a), où :
(i) le fragment est un ARN, et la région 5' et le codon d'initiation de la région codant pour env sont réunis à une région codante aval, dans le cadre de lecture en +2 par rapport à celui de env ;
(ii) le fragment comprend la jonction par épissage ;
(iii) le fragment se trouve dans le cadre de lecture en +2 par rapport au codon d'initiation de env de PCAV ; ou
(iv) le fragment comprend une région qui utilise le cadre de lecture en +2 par rapport au codon d'initiation de env de PCAV ; ou
(c) une séquence nucléotidique ayant une identité d'au moins 95 % avec (a) ; ou (b) le complément de (a) ou de (b).

9. Polypeptide isolé codé par un transcrit produit par un événement d'épissage, dans lequel la région 5' de la région codant pour env de HERV-K et le codon d'initiation d'une région codant pour env de HERV-K sont réunis à une région codante aval dans le cadre de lecture en +2 par rapport à celui de env dans le génome de HERV-K, comprenant :
(a) une séquence d'acides aminés choisie dans le groupe consistant en SEQ ID NO:7, 8, 9, 10, 11, 12, 28, 29, 30, 31, 34, 35, 36, 39, 41, 43, 66, 67, 68 et 78 à 277 ;
(b) un fragment d'au moins 45 acides aminés
(i) de SEQ ID NO:7, 8, 9, 10, 11, 12, 28, 29, 30, 31, 34, 35, 36, 66, 67, 68 et 78 à 277 ;
(ii) de SEQ ID NO:39, 41, 43, le fragment comprenant une région qui est codée par un transcrit produit par un événement d'épissage dans lequel la région 5' et le codon d'initiation de la région codant pour env de HERV-K sont réunis à une région codante aval dans le cadre de lecture en +2 par rapport à celui de env dans le génome de HERV-K ; ou
(iii) de SEQ ID NO:39, 41, 43, le fragment comprenant une région qui est codée par un transcrit produit par un événement d'épissage dans lequel la région 5' et le codon d'initiation de la région codant pour env de HERV-K sont réunis à une région codante aval dans le cadre de lecture en +2 par rapport à celui de env dans le génome de HERV-K, et le fragment comprenant un épitope ; ou
(c) une séquence polypeptidique ayant une identité d' au moins 80 % avec (a) .

10. Polypeptide selon la revendication 9(b), dans lequel le fragment comprend un épitope de cellule T ou de cellule B d'une ou plusieurs des séquences SEQ ID NO:7, 8, 9, 10, 11, 12, 28, 29, 30, 31, 34, 35, 36, 39, 41, 43, 66, 67, 68 et 78 à 277.

11. Polynucléotide isolé qui code pour le polypeptide de la revendication 9 ou de la revendication 10, dans lequel le ou les polypeptides consistant en SEQ ID NO: 11, 28, 29 ou 31, ou la comprenant, sont codés par un polynucléotide choisi parmi SEQ ID NO:19, 20 ou 21.

12. Anticorps qui se lie au polypeptide de la revendication 9, de la revendication 10 ou de la revendication 11.

13. Anticorps selon la revendication 12, l'anticorps étant un anticorps monoclonal.

14. Polynucléotide, polypeptide ou anticorps selon l'une quelconque des revendications 8 à 13, pour utilisation en tant que médicament.

15. Utilisation du polynucléotide, du polypeptide ou de l'anticorps selon l'une quelconque des revendications 8 à 13 pour la fabrication d'un médicament destiné à prévention ou au traitement du cancer de la prostate, du cancer du sein, du cancer des testicules, de la sclérose en plaques et/ou du diabète sucré insulino-dépendant.

16. Composition immunogène comprenant le polypeptide de la revendication 9 ou de la revendication 10 ou de la revendication 11 et un support pharmaceutiquement acceptable.

17. Composition selon la revendication 16, qui comprend en outre un adjuvant.
